(19) Europäisches Patentamt / European Patent Office / Office européen des brevets

(11) **EP 2 591 364 B1**

(12) **EUROPÄISCHE PATENTSCHRIFT**

(45) Veröffentlichungstag und Bekanntmachung des Hinweises auf die Patenterteilung:
**05.11.2014 Patentblatt 2014/45**

(21) Anmeldenummer: **10843618.9**

(22) Anmeldetag: **28.12.2010**

(51) Int Cl.:
**G01N 33/68** *(2006.01)*

(86) Internationale Anmeldenummer:
**PCT/DE2010/001527**

(87) Internationale Veröffentlichungsnummer:
**WO 2012/003817 (12.01.2012 Gazette 2012/02)**

(54) **EIN NEUES VERFAHREN ZUM CHARAKTERISIEREN UND MULTIDIMENSIONALEN DARSTELLEN DES FALTUNGSVORGANGS DER PROTEINE**

NOVEL METHOD FOR CHARACTERIZING AND MULTI-DIMENSIONALLY REPRESENTING THE FOLDING PROCESS OF PROTEINS

NOUVEAU PROCÉDÉ DE CARACTÉRISATION ET DE REPRÉSENTATION MULTIDIMENSIONNELLE DU PROCESSUS DE REPLIEMENT DES PROTÉINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.07.2010 DE 102010026094**

(43) Veröffentlichungstag der Anmeldung:
**15.05.2013 Patentblatt 2013/20**

(73) Patentinhaber: **Han, Sigeng**
**60437 Frankfurt am Main (DE)**

(72) Erfinder: **Han, Sigeng**
**60437 Frankfurt am Main (DE)**

(56) Entgegenhaltungen:
**US-A1- 2008 270 094**

• BILSEL ET AL: "Molecular dimensions and their distributions in early folding intermediates", CURRENT OPINION IN STRUCTURAL BIOLOGY, ELSEVIER LTD, GB, Bd. 16, Nr. 1, 1. Februar 2006 (2006-02-01), Seiten 86-93, XP005289211, ISSN: 0959-440X, DOI: 10.1016/J.SBI.2006.01.007

• CHEN Y ET AL: "Protein folding: Then and now", ARCHIVES OF BIOCHEMISTRY AND BIOPHYSICS, ACADEMIC PRESS, US, Bd. 469, Nr. 1, 1. Januar 2008 (2008-01-01), Seiten 4-19, XP026268294, ISSN: 0003-9861, DOI: 10.1016/J.ABB.2007.05.014 [gefunden am 2008-01-01]

• ALEXEI V. BUEVICH ET AL: "Residue-Specific Real-Time NMR Diffusion Experiments Define the Association States of Proteins during Folding", JOURNAL OF THE AMERICAN CHEMICAL SOCIETY, Bd. 124, Nr. 24, 1. Juni 2002 (2002-06-01), Seiten 7156-7162, XP55007555, ISSN: 0002-7863, DOI: 10.1021/ja012699u

• BHARATH S. MAMATHAMBIKA ET AL: "Disulfide-Linked Protein Folding Pathways", ANNUAL REVIEW OF CELL AND DEVELOPMENTAL BIOLOGY, Bd. 24, Nr. 1, 1. November 2008 (2008-11-01), Seiten 211-235, XP55007556, ISSN: 1081-0706, DOI: 10.1146/annurev.cellbio.24.110707.175333

• PAMELA K. JENSEN ET AL: "Monitoring Protein Refolding Induced by Disulfide Formation Using Capillary Isoelectric Focusing-Electrospray Ionization Mass Spectrometry", ANALYTICAL CHEMISTRY, Bd. 70, Nr. 10, 1. Mai 1998 (1998-05-01), Seiten 2044-2049, XP55007557, ISSN: 0003-2700, DOI: 10.1021/ac9712963

• J THROCK WATSON: "Capture and identification of folding intermediates of cystinyl proteins by cyanylation and mass spectrometry", JOURNAL OF MOLECULAR GRAPHICS AND MODELLING, Bd. 19, Nr. 1, 1. Februar 2001 (2001-02-01), Seiten 119-128, XP55007566, ISSN: 1093-3263, DOI: 10.1016/S1093-3263(00)00127-3

- JIANG WU ET AL: "Trapping of intermediates during the refolding of recombinant human epidermal growth factor (hEGF) by cyanylation, and subsequent structural elucidation by mass spectrometry", PROTEIN SCIENCE, Bd. 7, Nr. 4, 1. April 1998 (1998-04-01), Seiten 1017-1028, XP55007569, ISSN: 0961-8368, DOI: 10.1002/pro.5560070419

- STEPHAN REHM ET AL: "The High Resolution NMR Structure of Parvulustat (Z-2685) from Streptomyces parvulus FH-1641: Comparison with Tendamistat from Streptomyces tendae 4158", CHEMBIOCHEM, Bd. 10, Nr. 1, 5. Januar 2009 (2009-01-05), Seiten 119-127, XP55007554, ISSN: 1439-4227, DOI: 10.1002/cbic.200800547

## Beschreibung

Die Erfindung betrifft ein neues Verfahren zum Charakterisieren und multidimensionalen Darstellen des Faltungsvorgangs der Proteine. Gegenstand der Erfindung ist ein mehrere Schritte umfassendes Verfahren kombiniert aus der kinetischen Anordnung der hydrodynamischen Größe des rückfaltenden und dabei modifizierten Proteins, der auf die bioinformatischen Erkennungsmuster beruhenden Zuordnung der aufgetrennten und proteolytisch fragmentierten Intermediate, der Klassifizierung der Faltungswegezugehörigkeit der dynamisch modifizierten Intermediate, der Charakterisierung der Faltungsabläufe und der multidimensionalen Visualisierung des charakterisierten Faltungsvorgangs.

Nach diesem neuen Verfahren werden die Intermediate der rückfaltenden und dabei modifizierten Proteine nicht mehr wie bei der konventionellen Methode nach ihrer einzelnen Disulfidbrückenbildung, sondern nach ihren 4 erfindungsgemäß definierten individuellen Charakteristiken, nämlich der hydrodynamischen Größe, dem Entstehungszeitpunkt, der Faltungswegezugehörigkeit und der Menge, identifiziert und digitalisiert. Dadurch können nicht nur die disulfidhaltigen Proteine sondern auch die disulfidfreien Proteine auf ihren Faltungsablauf ohne Beschränkung ihrer molekularen Art, ihres Typs und ihrer Größe untersucht, charakterisiert und multidimensional visuell dargestellt werden.

Zur Durchführung dieses Verfahrens können erfindungsgemäß vielfältige Produkte in Form von unterschiedlichen Assay-Kits, Geräten, Software und Automaten hergestellt und-eingesetzt werden.

## Das Gebiet der Erfindung

Das Gebiet der Erfindung umfasst die Forschung der Proteinfaltung und Proteopathy, das Proteinengineering, das Antikörperengineering, die molekulare Biologie, die Immunbiologie, die therapeutische Medizin, die Biotechnologie, die biotechnologische Erzeugung von Proteinpharmaka, Proteintaxonomie und die Nanotechnologie zur Entwicklung neuartiger funktioneller Proteinmaterialien.

## Technische Hintergründe

Proteine müssen korrekt gefaltet sein, um ihre biologische Funktion ausüben zu können. Die lineare Polypeptidkette muss nach der Synthese am Ribosom in die entsprechende Sekundär-, Tertiär- und Quartärstuktur überführt werden. Falsch gefaltete Proteine sind für eine Reihe von Krankheiten, die derzeit in der Diskussion stehen, verantwortlich. Dazu gehören zahlreiche Muskelkrankheiten, aber auch Alzheimer, die Creutzfeld-Jakob-Krankheit, Scrapie und BSE (Bovine spongiforme Enzephalopathie). Die Entwicklung eines effektiven Verfahrens zur Aufklärung des Mechanismus der Proteinfaltung, zur Erklärung der Ursache der Krankheiten und zur Entwicklung der neuartigen Proteintherapeutika ist bis heute eine große Herausforderung im Life-Science Bereich. Seit Christian Anfinsens (Anfinsen, 1972, Nobelpreisträger für Chemie) Experimenten zur Rückfaltung von Proteinen ist bereits bekannt, dass die Primärstruktur die gesamte Information für die Raumstruktur eines Proteins beinhaltet. Allerdings ist damit noch nicht zu erklären, wie sich die Proteine falten und ihre native Konformation finden. Mit der Lösung dieses "Problems der Proteinfaltung" haben sich die Wissenschaftler in den letzten Jahrzehnten weltweit intensiv beschäftigt. Viele Theorien und entsprechende Methoden und Techniken für das Untersuchen. Interpretieren und Charakterisieren der Proteinfaltung wurden in der letzten Zeit entwickelt.

1. Strukturvorhersage der Proteine

Die Strukturvorhersage des Proteins wurde zur Aufklärung des Faltungsmechanismus gebraucht Dafür gibt es bisher zwei bekannte einsetzbare Methoden. Die erste ist die auf dem Alignment der Aminosäuresequenz beruhende Methode, also die Homologiemodellierung (Guex, 1997; Schwede, 2003; Kopp, 2004) und das Threading (Hendlich, 1990; Sippl, 1996; Madej, 1995). Bei diesen wissensgestützten Methoden wird eine Aminosäuresequenz von unbekannter Struktur auf ihre Vereinbarkeit mit bekannten Proteinstrukturen hin untersucht. Wird eine deutliche Übereinstimmung festgelegt, lässt sich die bekannte Struktur als Ausgangsmodell heranziehen. Dies ist das bisher einzige praktische und effektive Verfahren, das zur Tertiärstrukturvorhersage für homologe Proteine benutzt wird. Die zweite ist die *ab initio*-Vorhersage (Karplus, 1990; Sippl, 1995; Shortle, 1997), bei der man die Faltung der Aminosäurekette ohne weiteren Bezug zu anderen bekannten Proteinstrukturen vorhersagen will. Mit Hilfe computergestützter Berechnungen wird versucht, die freie Enthalpie einer Struktur von gegebener Aminosäuresequenz zu minimieren oder den Faltungsprozess zu simulieren. Dazu ist ein weltweiter Strukturvorhersage-Wettbewerb CASP(the Critical Assessment of Techniques for Protein Structure Prediction) (www.predictioncenter.org), 1994 ins Leben gerufen worden, der alle zwei Jahre stattfindet. Bei diesem Wettbewerb werden Proteinsequenzen, deren 3D-Struktur kurz vor der Aufklärung stehen, veröffentlicht, die strukturellen Vorhersagen gesammelt und anschließend mit den experimentellen Strukturen verglichen.

2. Klassische Modelle für Proteinfaltung

Nach der klassischen Sichtweise der Proteinfaltung, die sich vorwiegend auf experimentelle Beobachtungen stützt, gibt es eine ganze Reihe von Modellen. Im einfachsten Fall gibt es ein Zwei-Zustandsmodell (Two-State Model). Die meisten Modelle gehen aber von einer Faltung in mehreren Schritten aus. All diesen Modellen ist gemeinsam, dass sie für einige Proteine korrekt sind, für andere hingegen nicht. Ganz generell gesagt sind in vielen Fällen tatsächlich Sekundärstruktur-Bildung und der hydrophobe Kollaps frühe Faltungsereignisse, aber auch das Mikrodomänen- und Puzzle- (jigsaw puzzle model) Faltungsmodell bzw. das molten globule-Modell (Ohgushi M & Wada A, 1983) sind bei der Faltung einiger Proteine realisiert worden.

Das Zwei-Zustandsmodell (Two-State-Model) ist das einfachste Modell für die Faltung eines kleinen Proteins, bei dem es nur zwei stabile Zustände gibt: gefaltet oder ungefaltet. Das sequenzielle Modell zeigt, dass die Faltung von ungefaltet nach gefaltet über die Zwischenstufen verläuft. Nach dem Vorschlag von Tsong *et al.* sollte eine Folge von Intermediaten den Weg definieren (Tsong *et al.,* 1972). Im framework-Modell (Kim und Baldwin, 1990) bilden sich erst unabhängig einzelne lokale Elemente der Sekundärstruktur, die später zueinander diffundieren und die Tertiärstruktur bilden. Demgegenüber geht das nucleation-Modell (Wetlaufer, 1973) von der Bildung eines Faltungs-Nukleus aus, von dem aus sich die Bildung nativer Strukturen fortpflanzt. Im Modell des hydrophoben Kollaps (Dill *et al.,* 1995) wird die Kontraktion der Polypeptidkette aufgrund der hydrophoben Wechselwirkungen der Seitenketten als erster Schritt postuliert, nachdem eine Umorientierung zur nativen Struktur auf lokaler Ebene erfolgt.

Nach dem Mikrodomänen-Faltungsmodell nehmen zuerst kleine strukturelle Subdomänen ihre native Konformation ein. Diese Domänen wachsen dann durch Ausbreitung oder Kollision mit anderen Subdomänen. Im Puzzle-Modell existiert wie bei allen anderen Modellen nur eine native Konformation, aber es gibt viele verschiedene Wege zu diesem Endzustand, ebenso wie ein Puzzle auf verschiedene Arten vollendet werden kann. Die Molten Globule Hypothese schlägt vor, dass ein Molten-Globule-Intermediat während der Faltung aller Proteine auftaucht. Dieses Faltungsintermediat hat alle Sekundärstrukturen des vollständig gefalteten Proteins, aber noch keine Tertiärstruktur-Bereiche.

In allen Modellen werden am Anfang der Faltungskaskade die Sekundärstruktur-Bereiche ($\alpha$-Helices und $\beta$-Faltblätter) in der erstaunlich kurzen Zeit im Bereich von einer Nanosekunde bis zu Sekunden gebildet. Der weitere Verlauf der Proteinfaltung, die der Diffusion, der Kollision und der Kontraktion der Sekundärstrukturen unterzogen ist und zur nativen Struktur der Proteine führt, hat eine Zeitskala von einigen Millisekunden bis mehreren Tagen.

3. Thermodynamische Modelle für Proteinfaltung

Aus den Experimenten, die C. Anfinsen und andere nach ihm durchgeführt haben, konnte gefolgert werden, dass die native Struktur von Proteinen einen thermodynamisch stabilen Zustand darstellt und damit dem globalen Minimum der ihnen zugänglichen Gibbs-Energie entspricht. C. Levinthal (Levinthal, 1968) beschrieb, dass es einen bestimmten Pfad (folding pathway) geben muss, dem ein Protein bei seiner Faltung folgt und an dem das Protein unter geeigneten Bedingungen unweigerlich eine vorgegebene Folge von Konformationen (Zwischenzuständen) durchläuft, bis es in den nativen Zustand mit dem minimalen Energiezustand gelangt. Insbesondere Modelle aus der statistischen Mechanik erlauben, dieses Pfadkonzept eines sequentiellen Faltungsprozesses durch ein Trichter-Konzept (Schultz, 2000) von parallelen Ereignissen zu ersetzen.

Das Trichter-Konzept der Proteinfaltung stellt die freie Energie (vertikale Achse) aller möglichen Proteinstrukturen als Funktion der konformationellen Freiheitsgrade dar (horizontale Achse). Verschiedene ungefaltete Stadien eines Proteins auf der Oberseite fallen in den Faltungstunnel hinein. Er besitzt viele verschiedene lokale Minima, in die das Protein hineinfallen kann. Einige dieser lokalen Minima repräsentieren Zwischenstufen (Intermediate) auf dem Weg zum energieärmsten nativen Zustand des Proteins. Ein Teil dieser Intermediate repräsentiert bereits relativ stabile kompakte Strukturen "Molten Globules", während andere lokale Minima als Falle wirken und Proteine in einem missgefalteten Zustand halten. Die meisten Proteinmoleküle werden nach diesem Trichter-Modell auf ihrem Weg zum gefalteten Zustand wohl nicht einfach einen glatten Trichter hinunterrutschen, sondern eher eine zerklüftete und schroffe Landschaft vorfinden, in der alle möglichen Unebenheiten, Gräben, Schwellen und Mulden zu überwinden sind, bevor sie im Tal landen.

4. Kinetik der Proteinfaltung

Die Proteinfaltung ist ein Prozess, der oft aus mehreren Phasen besteht. Zu den schnellen Phasen der Faltung gehören beispielsweise der hydrophobe Kollaps der Polypeptidkette, die Bildung von Wasserstoff-Brücken und die Ausbildung der sekundären Strukturelemente. Diese gehen im Bereich von Nano- bis Mikrosekunden vonstatten, während langsame Phasen im Bereich von Sekunden bis Stunden verlaufen. Beispiele für eine langsame Faltung sind RNase A (Ribonuklease A) oder Thioredoxin, während z.B. bei Lysozym oder Cytochrom c die schnelle Phase der Faltung dominiert. Es gibt allerdings nur sehr wenige Proteine, die überhaupt keine langsame Faltungsphase aufweisen.

Es ist bekannt, dass sich große Proteine in der Regel eher langsam falten und die Faltung der großen Proteine in der Zelle durch zwei unterschiedliche Klassen von zusätzlichen Proteinen unterstützt wird, die als Foldasen und Chaperone bezeichnet werden. Foldasen wie PPI (Peptidyl-Prolyl cis-trans-Isomerase im Periplasma), PDI und DsbA, B, C, D, G (Protein Disulfid-Isomerasen im Periplasma) haben eine klar definierte katalytische Aktivität, die die Bildung von kovalenten Verzweigungen der Polypeptidkette beschleunigt. Chaperone auf der anderen Seite verfüllen viele Funktionen, wobei die wichtigste Funktion vermutlich die ist, eine Umgebung für das naszierende Protein zu schaffen, in dem es sich falten kann, ohne dass es dem konkurrierenden Prozess der Selbstassoziation ausgeliefert ist. Das Bakterienchaperon GroEL (Hitzeschockprotein) beispielsweise hilft schätzungsweise der Hälfte aller mittelgroßen (30-60 kDa), neu synthetisierten Bakterienproteine bei der Faltung. Die Unterscheidung zwischen Foldasen und Chaperonen läßt sich nicht immer klar definieren, da einige Proteine z. B. die Disulfidisomerase (PDI), sowohl als Foldase als auch als Chaperon zumindest in *in vitro* Bedingungen wirksam ist.

5. Methoden und Techniken zur Untersuchung der Proteinfaltung

Die experimentellen Untersuchungen der Proteinfaltung kann man in zwei Arten erfassen. Die erste bezieht sich auf Experimente im Gleichgewichtszustand, in denen die Konformationen des Proteins als Funktion der Konzentration des Denaturierungsmittels oder der Temperatur dargestellt werden. Die andere bezieht sich auf kinetische Studien, bei denen die strukturellen Veränderungen des Proteins als Funktion der Zeit bei rapiden Bedingungsänderungen des Lösungsmittels dargestellt werden. Die jetzigen für die Untersuchungen der Proteinfaltung angewendeten Techniken, Methoden und wichtigen Chemikalien sind folgende:

• NMR (Kernresonanzspektroskopie), Röntgenkristallographie, Elektronenmikroskopie und AFM (Atomic Force Microscopy) zur Untersuchung der dynamischen Strukturänderung der ProteinIntermediate, die bei der Ent- und Rückfaltung entstehen und gegebenenfalls chromatographisch aufgetrennt werden können.

■ Spektroskopische Verfahren, wie z. B. Fluoreszenz-, UV (Ultraviolettstrahlung)-/VIS (sichtbare Spektrum)-Spektroskopie, ESR (Elektronenspinresonanz)-Spektroskopie, CD (Circulardichrois-mus)-Spektropolarimetrie und Fourier-Transform-Infrarot-Spektroskopie zur Untersuchung der Strukturveränderungen des Proteins bei der Ent- und Rückfaltung, DLS (Dynamische Lichtstreuung) und SLS (Statische Lichtstreuung) zur Messung des molekularen Radius der Proteine, ESI-MS (Elektronspraylonisation-Massenspektrometrie) und MALDI-MS (Matrix-unterstützte Laser-Desorption/ Ionisation-Massenspektrometrie) in unterschiedlichen Typen zur Massenbestimmung des Proteins und der enzymatisch gespaltenen Proteinfragmente.

■ Stopped-flow-, Quench-flow- und Gradienten-Techniken, Fluktuations- und Sprungmethoden in Kopplung mit spektroskopischen Methoden. Diese Methoden erreichen eine Zeitauflösung von wenigen Millisekunden. Bei einem stop flow-Experiment wird die Rückfaltung eines Proteins durch rasche Verdünnung der denaturierenden Lösung oder durch drastische Veränderung des pH-Wertes getriggert. Mit Hilfe der so genannten continuous flow-Techniken können Faltungsprozesse bis in den Bereich von 50-100 $\mu s$ untersucht werden.

■ Wasserstoff-Deuterium-Austausch in Kombination mit spektroskopischen Verfahren zur Untersuchung des kinetischen Faltungswegs des Proteins.

■ MD (Moleküldynamik) zur Computersimulation der molekularen Strukturänderungen der Faltung des Proteins.

■ 2D-Paperelektrophorese, native- und Natriumdodecylsulfat-Polyacrylamidgeielektrophorese (SDS-PAGE), Kapillarelektrophorese , (CE) und Flüssigchromatographie einschließlich der Reversed-Phase-Hochleistungsflüssigkeitschromatografie (RP-HPLC) zur Trennung, Untersuchung und Überprüfung der isolierten Intermediate, des Proteins und der enzymatisch gespaltenen Proteinfragmente.

■ Fluoreszenzmikroskopische Verfahren mit räumlicher und zeitlicher Höchstauflösung zur Untersuchung der Proteinfaltung und des Proteintransports innerhalb der Zelle, Protein-Protein-Wechselwirkungen sowie Strukturänderungen der Proteine bei der Faltung: Fluoreszenzdepolarisation, Kolokalisationsmessungen, Förster-Resonanzenergietransfer (FRET), zeitaufgelöste Resonanzenergietransfer (TRFREIT), fluorescence lifetime imaging (FLIM), fluorescence recovery after photobleaching (FRAP), sowie Fluoreszenz-Fluktuationsmethoden in verschiedenen Varianten, insbesondere fluorescence correlation spectroscopy (FCS).

■ Das Abfangen und die Identifizierung der disulfidhaltigen Intermediate. Dies geschieht, indem das disulfidhaltige Protein (oxidiertes Protein) durch Denaturierung und Reduktion mit bekannten Reduktions- und Denaturierungsmittel reduziert, entfaltet, dann vom Reduktions- und Denaturierungsmittel befreit, anschließend einer Modifikation der Thiolgruppen der Cysteinreste mit Abfangmitteln zum Beispiel Jodacetat bei der Reoxidation unterzogen und dann in Form von Zwischenprodukten abgefangen wird. Diese Zwischenprodukte als Intermediate werden dann chromatographisch aufgetrennt, proteolysiert, teilweise sequenziert und gegebenenfalls spektroskopisch je nach Position der Disulfidbildung in Proteinfragmente identifiziert (Creighton, 1978).

- Die Charakterisierung des Faltungsvorgangs durch die Zuordnung der Einzig-Disulfidbildungen in identifizierten Intermediaten und schematische Interpretation ihres Zusammenhangs (Creighton, 1988; Weissmann & Kim, 1991).

- Die direkte Beobachtung des Faltungsvorgangs eines einzigen Proteins mit Rasterkraftmikroskop (Cecconi, et al., 2005; Walther, et al., 2007).

- Die Chemikalien beinhalten bekannte Denaturierungsmittel, Reduktionsmittel, Reoxidationsmittel, Modifkationsreagenzien, Stabilisierungsmittel der Faltung, Foldasen, biochemische und chemische Chaperone und Inhibotoren der Faltung und Zellextrakte usw..

**Stand der Technik des Charakterisierens des Vorgangs der Proteinfaltung**

1. Verfahren zum Charakterisieren des Vorgangs der Proteinfaltung

Es gab zahlreiche Pionierarbeiten, um ein effektives Verfahren zum Charakterisieren des Faltungsvorgangs der Proteine zu entwickeln. Ein vielversprechendes Verfahren, das aus den oben beschriebenen Techniken und Methoden kombiniert ist und in Form von einem kommerziellen Produkt als standardisierbare Methode auf den Markt für die routinemäßige Laborpraxis der Charakterisierung des Vorgangs der Proteinfaltung angeboten wird, ist bisher allerdings noch nicht bekannt.

Bilsel et al., Current Opinion in Structural Biology, vol. 16 no. 1 Seiten 86 - 93, offenbart eine Menge von Techniken (Darstellung der hydrodynamischen Größe, Massenspektrometrie, FRET (involviert Seitenkettenreagenzien) usw.) zur Charakterisierung von Proteinefaltungsintermediaten und Überwachung des Faltungsvorgangs der Proteine.

Chen Y et al., Archives of Biochemistry and Biophysics, vol. 469 no. 1 Seiten 4 - 19, beschreibt eine Menge von Techniken zur Unterscheidung von Proteinefaltungsintermediaten.

Alexei V. Buevich et al., Journal of the American Chemical Society, vol. 124 no. 24 Seiten 7156 - 7162, beschreibt NMR-Techniken zur Unterscheidung von Proteinefaltungsintermediaten.

Bharath S. Mamahtambika et al., Annual Review of Cell and Developmental Biology, vol. 24 no. 1 Seiten 211 - 235, verwendet Thiolfängermoleküle zur Charakterisierung von Proteinefaltungsintermediaten.

Pamela K. Jensen et al., Analytical Chemistry, vol. 70 no. 10 Seiten 2044 - 2049, Jiang Wu et al., Protein Science, vol. 7 no. 4 Seiten 1017 - 1028 und J Throck Watson, Journal of Molecular Graphics and Modelling, vol. 19 no. 1 Seiten 119 - 128, offenbaren Überwachung der Faltungsvorgänge der Proteine mittels Massenspektrometrie.

Stephan Rehm et al., Chembiochem, vol. 10 no. 1 Seiten 119 - 127, offenbart die NMR-Strukturdaten von Parvulustat.

Das in den letzten Jahrzehnten weltweit dominierende konventionelle Verfahren zum Charakterisieren des Faltungsvorgangs der Proteine bezieht sich meistens auf die kleinen globularen Proteine und ist beschränkt auf die disulfidhaltigen, auch oxidativ genannten Proteine und betrifft selten die großen Proteine mit Multidomainen. Das Verfahren beruht auf der theoretischen Annahme, dass die Reihenfolge der Bildung der Disulfidbrücken den Faltungsprozess des Proteins indiziert. Dieses Verfahren lässt sich mit einer Kombination von zwei technischen Konzepten erfassen, nämlich der Identifizierung und Klassifizierung der einzig-disulfidhaltigen Intermediate des rückfaltenden Proteins und die anschließende Interpretation der Faltungswege durch Zuordnung der Reihenfolge der Disufidbildungen und der möglicherweise erfassten intermolekularen Umlagerungen in den identifizierten Intermediaten.

Dieses der Anordnung der Intermediate nach ihrer Disulfidbildung zugrunde liegende Verfahren wurde ursprünglich von Creighton TE (Creighton, 1978,1988) entwickelt. Das Protein wird in einem Ansatz mit Denaturierungsmittel GdmCL (Guanidiniumchlorid) und Reduktionsmittel DTE (1,4-Dithioerythrit) zugleich denaturiert und reduziert und durch die Gelfiltration vom Denaturierungs- und Reduktionsmittel befreit. Das reduzierte Protein wird dann der Reoxidation zur Disulfidbildung in einem Reoxidationspuffer mit GSH und GSSG (reduziertem und oxidiertem Glutathion) unterzogen, wobei das chemische Abfangmittel Jodacetat oder Jodacetamid zur Blockierung der noch freien bleibenden Thiolgruppen und zum Anhalten des Faltungsvorgangs in verschiedenen Zeitabständen zugegeben wird. Die dadurch abgefangenen Intermediate der Rückfaltung werden anschließend mit IEC (Ionenaustauschchromatographie) isoliert und weiterhin enzymatisch in die Fragmente gespalten und durch die zweidimensionale Papierelektrophorese separiert. Die Identifizierung der Intermediate erfolgt durch die Bestimmung der Position der Einzig-Disulfidbildung in den Fragmenten mittels Edman-Sequenzierung (Aminosäuresequenzierung). Die anschließende Charakterisierung der Faltungswege erfolgt durch die Zuordnung der Reihenfolge der Disulfidbildung in den Fragmenten.

Dieses konventionelle Verfahren wurde von Kim PS und seinem Kollegen (Weissmann & Kim, 1991) mit einer schnelleren und empfindlicheren Methode für die Ermittlung der Disulfidbindungen in den Intermediaten weiter verbessert. Das Ausgangsmaterial besteht aus den gereinigten Intermediaten, welche die reoxidierten Disulfidbindung und die vom Abfangmittel Jodacetat blockierten Thiolgruppen enthalten. Die Disulfidbindung dieser Intermediate wird zuerst mit Reduktionsmittel zu freien Thiolgruppen reduziert und dann mit einem fluoreszierenden Jodacetat-Derivat IAEDANS (5-(2-(2-Iodacetamido) ethylamino]-naphthalen-1-sulfonsäure) zur Erhöhung der Detektionsempfindlichkeit durch die kovalenten Bindungen markiert. Das Protein wird danach enzymatisch fragmentiert. Die markierten Cystein-Reste indizieren die ursprüngliche Disulfidbindung der Fragmente. Die Fragmente werden anschließend durch RP-HPLC (Reversed-Phase Hochleistungsflüssigkeitschromatografie) mit der gestiegenen Trennauflösung und Schnelligkeit separiert. Durch die Edman-Sequenzierung und die nötigen NMR-Untersuchung der getrennten Fragmente werden die Positionen der Disulfidbindungen in den Fragmenten festgestellt. Die Intermediate werden dadurch je nach Distribution ihrer Einzig-Disulfidbildung identifiziert. Die Faltungswege werden zum Schluss durch die Zuordnung der Reihenfolge der Disulfidbildung in den Intermediaten und Analyse ihrer intermolekularen Umlagerung schematisch interpretiert. Der Faltungsvorgang eines Proteins wird dadurch charakterisiert.

Dieses von Pionierarbeiten von Creighton und Kim geprägte Verfahren dominiert seit 20 Jahren weltweit die Praxis der Charakterisierung der Proteinfaltung. Die in den zahlreichen Veröffentlichungen dargestellten Faltungsstudien der Proteine werden auf dem Prinzip dieses Verfahrens und je nach Vorliebe der Technikzusammenstellung in verschiedenen modifizierten Formen durchgeführt. Die Untersuchungen erfordern größere Mengen des Proteins, manchmal auch einige 100mg und dauern meistens monatelang, bis der Faltungsvorgang eines kleinen Proteins interpretierend charakterisiert werden kann. Die Ergebnisse der Charakterisierung sind oft diversifizierend und können nur untereinander komplementierend interpretiert werden. Nach derzeitiger Angabe von UniProtKB/TrEMBL stehen heutzutage schon 11 Millionen Proteine mit charakterisierten Primärstrukturen und mehr als 66.000 Proteine mit aufgeklärten 3D-Strukturen (RCSB Protein Data Bank) für die nötigen Faltungsstudien zur Verfügung, aber bis heute konnte nur ein sehr kleiner Bruchteil dieser Proteine mit diesem Verfahren untersucht werden. Die Faltungswege der 3 kleinen Proteine, RNase A (Ribonuklease A), BPTI (bovine pancreatic trypsin inhibitor) und Hirudin, die als bekannteste Modellproteine nach diesem Verfahren am besten untersucht wurden, sind nur mit beschränkter Genauigkeit und teilweise mit fundamentaler Meinungsverschiedenheit (Disulfide Folding Pathway of BPTI, Science vol. 256, 3 April 1992) charakterisiert. Dies erweist, dass dieses Verfahren vom Stand der Technik für die Charakterisierung des Faltungsvorgangs nicht effektiv genug ist. Seine Durchführung ist kompliziert, arbeits- und zeitaufwendig, es benötigt teure Ausrüstungen und weist eine niedrige Gesamt-Effizienz auf. Seine Ergebnisse weichen ab, je nach der angenommenen Technikzusammenstellung des Benutzers. Es konnte deshalb nicht zu einem standardisierbaren kommerziellen Produkt entwickelt werden. Die Ursachen und die damit verbundenen Probleme liegen im ungünstigen Prinzip dieses Verfahrens und in den darauf zurückzuführenden technischen Beschränkungen.

2. Verfahrensprobleme des Stands der Technik

Das Problem des gegenwärtigen Verfahrens zur Charakterisierung des Faltungsvorgangs liegt darin, dass es sich ausschließlich auf die Suche nach Disulfidbildung der Proteininintermediate ausrichtet und sich die Trennung und Anordnung der Intermediate allgemein nur auf die grundlegende Methode der RP-HPLC (Reversed-Phase-Hochleistungsflüssigkeitschromatografie) und der CE (Kapillarelektro-phorese) konzentriert. Dies führt zwangsläufig zur prinzipiellen Beschränkungen des Verfahrens wie folgt:

- Erstens, ungefähr 30% der gesamten Proteine, die keine Disulfidbildung besitzen und nicht als oxidative Proteine zu bezeichnen sind, werden daher von diesem Verfahren ausgeschlossen, sie können also nicht damit untersucht werden.

- Zweitens, die Disulfidbildung als Indiz der Faltung eignet sich prinzipiell nur für die Untersuchung der kleinen Proteine, nicht aber der großen Proteine, denn die Separation und Identifizierung der einzig-disulfidhaltigen Intermediate werden mit der zunehmenden Proteingröße und der steigenden Zahl der Disulfidbindung immer schwieriger. Ein Protein mit 3 nativen Disulfidbindungen kann 15 mögliche einzig-disulfidhaltige Intermediate erzeugen, ein Protein mit 4 nativen Disulfidbindungen hat dann 28 und ein Protein mit 4 nativen Disulfidbindungen erzeugt 45. Diese explosionsartige Zunahme der Intermediate und ihre molekulare Größe stoßen auf die auftrennbaren Grenzen der HPLC-Methodik, die sich eigentlich nur für die Trennung der kleinen Proteine eignet. Deshalb versagt das Verfahren bei den großen Proteinen grundsätzlich.

- Drittens, die vielen wesentlichen Intermediate der disulfidhaltigen Proteine, deren Thiolgruppen sich bei der ersten Phase der Rückfaltung noch nicht zu Disulfidbrücken bilden, werden verfahrensmäßig nicht verfolgt und nicht als Intermediate betrachtet und daher künstlich von der Untersuchung ausgeschlossen. Die Faltungswege können daher nur unvollständig charakterisiert und repräsentiert werden.

- Viertens, die Konformationen der einzig-disulfidhaltigen Intermediate, die sich meistens schrittweise mit abnehmender molekularer Größe gruppieren, wie sie von der vorliegenden Erfindung als wichtiger Beweis der Entstehung der Faltungswege aufgewiesen wird, können nicht vom Verfahren des Stands der Technik differenziert, berücksichtig und erfasst werden. Dies führt zu einem großen Verlust der Informationen über die Details der Faltung, insbesondere der Faltung in der schnellen Phase.

- Fünftens, das zur Reoxidation gebrachte Protein, das nach der Reduktion anschließend von Denaturierungsmittel befreit ist und vor dem weiteren Gebrauch aufbewahrt wird, befindet sich bereits aufgrund der Folge der instinktiven, spontanen und schnellen Faltungsphase oft im Molten-Globule-Zustand, der eine vergleichbare Molekülgröße wie sein etwas kleineres natives Protein hat. Das heißt, die anschließende verfahrensgemäße Charakterisierung kann nur die letzte langsame Faltungsphase vom Molten-Globule-Zustand zum renaturierten Protein verfolgen, aber nicht den ganzen Faltungsprozess.

- Sechstens, die RP-HPLC-Methodik kann zwar die kleinen Intermediate schnell trennen, aber keine entsprechende Informationen über die Reihenfolge der zeitlichen Entstehung der verschiedenen abgetrennten Intermediate unmittelbar liefern. Dies erschwert und verlangsamt enorm den Prozess der Identifizierung der Intermediate und die Charakterisierung der Faltungswege.

- Siebtens, die untersuchten Proteine werden je nach Vorliebe der Technikzusammenstellung und der modifizierten Varianten des konventionellen Verfahrens untersucht und beziehen zwar alle bekannte neue Techniken und Methoden, zum Beispiel ERI-MS und MALDI-MS usw. mehr oder weniger ein, aber wegen der prinzipiellen Beschränkung des Verfahrens ist ihr Leistungsvermögen ineffektiv kombiniert und deshalb minimal ausgenützt.

- Achtens, die kostspielige Edman-Sequenzierung wird als unverzichtbares Mittel für die Identifizierung der Intermediate gebraucht.

- Neuntens, das Verfahren des Stands der Technik kann meistens wie oben gezeigt nur einen beschränkten Bruchteil des Faltungsvorgangs verfolgen, ist aber trotzdem nicht in der Lage, den Ablauf dieses Vorgangs detaillierend zu beschreiben, denn dies Verfahren kann nicht alle vorkommende Intermediate, sondern nur die sogenannten Signifikant-Intermediate zum Identifizierten und Charakterisieren hereinbringen.

- Zehntens, die Ergebnisse der Untersuchungen vom Vorgang der Proteinfaltung können nicht in einem Koordinatensystem genau repräsentiert, sondern nur schematisch interpretiert werden.

- Elftens, das Verfahren ist kostenaufwendig, zeitraubend und ist nicht in der Lage zu standardisieren, miniaturisieren und automatisieren.

## Aufgabe der Erfindung

Die der Erfindung zugrunde liegende Aufgabe besteht darin, ein neuartiges Verfahren zum effektiven und effizienten Charakterisieren des Faltungsvorgangs sowohl für die disulfidhaltigen als auch die disulfidfreien Proteine zur Verfügung zu stellen. Das neue Verfahren soll in der Lage sein, die Proteinfaltung ohne Beschränkung ihrer Arten, Typen und Größen zu untersuchen, alle dabei vorkommenden Faltungswege und entsprechenden Intramolekularumlagerungen zu erfassen, den Vorgang der Proteinmissfaltung zu identifizieren, die vollständige Charakterisierung des Vorgangs sowohl der langsamen als auch der schnellen Phase der Faltung zu erbringen und die Charakterisierungsergebnisse multidimensional und in vielfältigen Formen anschaulich zu visualisieren.

Das neue Verfahren kann in unterschiedlichen molekularen Umgebungen und Ausführungsformen unter verschiedenen physikochemischen und biochemischen Bedingungen für seine vielfältigen Anwendungen zur Aufklärung des Mechanismus der Faltung, der Missfaltung, der Aggregation, der Interaktion, der Selbstassemblierung, der Polymerisation, der Alterung, des Verschleißes und der nascenten Biosynthese der Proteine, zur Rationalisierung und Leistungserhöhung des Antikörperengineerings und des Proteinengineerings, zur Verbessrung der Aktivität und Funktionalität der Proteine, zur Optimierung der biotechnologischen Herstellung der Tagetproteine, zur Entwicklung von Nano-Proteinmaterialien und zur Anreicherung der Proteintaxonomie usw. eingesetzt werden.

Insbesondere wird das Verfahren sowohl für die Verfahrensentwicklung der biotechnologischen Herstellung der *in vitro* simulierten bekannten *in vivo* posttranslational modifizierten Proteine als auch für die Suche nach neuartigen durch den Einfluss auf Proteinfaltung und -abbau hervorgerufenen biologischen und chemischen Wirkstoffen und Proteintherapeutika gebraucht.

Das neue Verfahren soll weniger Proteinmaterial gebrauchen, leicht handhabbar, zeitsparend und standardisier-, automatisier- und miniaturisierbar sein. Die für die Ausführung des Verfahrens gebrauchten Mittel in Form von unterschiedlichen Assay-Kits, Geräten und Software, inklusive der spezifischen Konzeption und des Entwurfs für die Automatisierung des Verfahrens, sollen zur Verfügung gestellt werden. Ein in einem multidimensionalen Energielandschaftsystem übertragbares Multifaltungswege-Modell für die optimale Ausführung und Anwendung des Verfahrens soll etabliert werden.

**Lösung der Aufgabe**

Oben genannte Aufgabe wird gemäß den Merkmalen der Patentansprüche der vorliegenden Erfindung gelöst. Die Besonderheit der Lösung liegt darin, dass die Intermediate der rückfaltenden und dabei modifizierten Proteine nicht mehr wie bei der konventionellen Methode des Stands der Technik nur nach ihrer einzelnen Disulfidbrückenbildung, sondern nach ihren 4 individuellen und digitalisierbaren Charakteristiken, nämlich der hydrodynamischen Größe, dem Entstehungszeitpunkt, der Faltungswegezugehörigkeit und der Menge, identifiziert werden. Die darauf beruhende Charakterisierung weist eine hohe Gesamt-Effizienz auf. Der dadurch charakterisierte Vorgang der Proteinfaltung wird nicht mehr wie beim Stand der Technik schematisch interpretiert, sondern viel exakter und in seiner Vielfalt multidimensional visuell dargestellt. Die Konstruktion, Überprüfung, Optimierung und Rationalisierung der Verfahrensschritte werden hierbei von einem neuen etablierten Multifatungswege-Modell unterstützt. Das erfindungsgemäße Verfahren ist daher dem Stand der Technik für die Charakterisierung der Proteinfaltung eindeutig überlegen.

**Zusammenfassung der Erfindung**

Gegenstand der Erfindung ist ein mehrere Schritte umfassendes Verfahren kombiniert aus der kinetischen Anordnung der hydrodynamischen Größe des rückfaltenden und dabei modifizierten Proteins, der auf die bioinformatischen Erkennungsmuster beruhenden Zuordnung der aufgetrennten und proteolytisch fragmentierten Intermediate, der Klassifizierung der Faltungswegezugehörigkeit der modifizierten Intermediate, der Charakterisierung der Faltungsabläufe und der computerunterstützten Visualisierung des charakterisierten Faltungsvorgangs in einem Multikoordinatensystem, wie in den Ansprüchen definiert. Dieses Verfahren bezieht sich auf die konzipierten vielfältigen dynamischen Modifizierungen der rückfaltenden Proteine, die Modifizierung und Verbesserung der Schlüsseltechnik für die Auftrennung der Intermediate und die etablierten neuen Methoden für die Identifizierung der Faltungswegezugehörigkeit der Intermediate. Die Erfindung beruht vor allem auf der vom Erfinder erstellten Zusammensetzung der folgenden fundamentalen Konzeptionen und dem dabei neu etablierten Multifaltungswege-Modell:

1) Der Vorgang der Rückfaltung eines zuvor optimal vollständig entfalteten Proteins kann nach seiner sich allmählich verkleinernden hydrodynamischen Größe entsprechend seines abnehmenden Energiezustands indiziert werden, wobei die veränderten Strukturen des faltenden Proteins durch räumliche Störung der mit zeitlichen Abständen erfolgten chemischen oder enzymatischen Modifizierung regelmäßig angehalten und abgefangen, als Intermediate aufgetrennt und nach der Reihenfolge ihrer hydrodynamischen Größe dargestellt werden.

Die hydrodynamische Größe ist hierbei definiert als eine vorwiegend mit dem hydrodynamischen Radius eines Proteins beschriebenen Charakteristik, die weitergehend durch Informationen über den strukturellen Aufbau, die Verteilung der Ladungen, Polarität, Hydrophobi- und Hydrophilizität auf der Moleküloberfläche usw. angereichert werden kann. Der hydrodynamische Radius $R_h$ (oder Stokes'sche Radius) ist der Radius einer dem Protein hydrodynamisch äquivalenten Kugel und ist deshalb im Unterschied zu anderen aus der Polymeranalytik bekannten Größen nicht statistisch, sondern phänomenologisch definiert. Die erfindungsgemäß direkt mit dem hydrodynamischen Radius dargestellte hydrodynamische Größe beschreibt also die Wirkung der Proteine in Transportprozessen (Viskosität, Diffusion, Permeation) und hängt sehr stark von der Gestalt und Form der Proteine ab. Die Intermediate eines Proteins, die in bestimmten Zeitpunkten entstehen und über individuelle Konformationen verfügen, lassen sich daher mit ihren hydrodynamischen Größen, die den unterschiedlichen Energiezuständen entsprechen, leicht unterscheiden.

Die hydrodynamische Größe kann dabei beträchtlich von der realen Größe des Teilchens abweichen und ist meist kleiner als die effektive Größe des Teilchens. Aber dies beeinträchtigt in keiner Weise die Methodik, die hydrodynamische Größe als Maßstab für die zu untersuchenden Intermediate des Proteins zu indizieren, denn hier wird vor allem nach der Relativität der hydrodynamischen Größe und der dadurch ermittelten Reihenfolge der Größe der Intermediate gefragt, nicht nach der absoluten Größe.

Diese durch Modifizierung abgefangenen hydrodynamischen Größen können von den realen zum Modifizierungszeitpunkt verfügten hydrodynamischen Größen abweichen. Aber diese Abweichungen beschränken sich auf die strukturelle Schwankung zwischen den sequenziell benachbarten Intermediaten und können die Reihenfolge der

EP 2 591 364 B1

realen hydrodynamischen Größe der Intermediate nicht ändern und deshalb dem Prinzip der Methodik nicht schaden. Der Grund dafür liegt darin, dass die während der Rückfaltung in zeitlichen Abständen erfolgten Modifizierungen aufgrund der allmählichen strukturellen Verkleinerung und der dabei immer beschränkteren Zugänglichkeit der Reagenzien meistens an der Oberfläche der Intermediate, stattfinden. Dies kann nicht zur stark energieerhöhenden Entfaltung, sondern nur zur Störung der Rückfaltung und dem weitergehenden Abfangen der Intermediate führen. Die durch diese Störung verursachten strukturellen Schwankungen beschränken sich auf so niedrige Energiedifferenzen, dass sie nicht stark genug sind die Reihenfolgen der realen hydrodynamischen Größen zu verändern und die ihnen zugrunde liegenden Energiebarrieren zu überwinden.

Die hydrodynamische Größe kann spektroskopisch (Lichtstreuung-, Fluoreszenz-Korrelations-, Elektronenspinresonanz- und Kernspinresonanz-Spektroskopie sowie Fluoreszenzpolarisation usw.) bevorzugt mit DSL (Dynamische Lichtstreuung) bestimmt werden. Die hydrodynamische Größe wird hierbei auch mit Trägheitsradius (radius of gyration, RMS-radius root mean square radius) gleichwertig definiert und kann spektroskopisch vorzugsweise mit SLS (Statische Lichtstreuung) gemessen werden.

Die hydrodynamischen Größen der Intermediate sind im Prinzip proportional zu ihren Energiezuständen und können deshalb bei Bedarf mit ihren spektrometrisch ermittelten thermodynamischen Größen ausgetauscht werden.

2) Die bei der Rückfaltung entstehenden Intermediate des Proteins sind vielfältig und können nach ihrer Gemeinsamkeit der Charakteristiken in verschiedene Gruppen je nach den zugehörigen Faltungswegen angeordnet werden. Sie haben nicht nur unterschiedliche hydrodynamische Größen, sondern auch eine Vielfalt von strukturellen Gestaltungen, die über die Zugänglichkeit und Effizienz der gezielten Modifizierungen entscheidet und als Grundlage der Differenzierung und Identifizierung der Intermediate dient. Dies ermöglichst es, diejenigen Aminosäurereste, zum Beispiel Lysinreste, Cysteinreste, Tyrosinreste, Histidinreste, Argininreste, Tryptophanreste, Methioninreste, Glutamat- und Aspartatreste des faltenden Proteins in gezielten zeitlichen Abständen mit spezifischen Seitenkettenreagenzien bedingt durch ihre strukturelle Zugänglichkeit dynamisch und kovalent zu modifizieren.

Die dadurch modifizierten Intermediate mit den abgefangenen Strukturen sind in verschiedenen hydrodynamischen Größen und sowohl von der variierten Platzierung der Modfikation als auch von dem zeitlichen Rückgang der Modifikationseffizienz aufgrund der strukturellen Verkleinerung und der nachfolgenden Abnahme der Zugänglichkeit der Reagenzien geprägt. Die Intermediate mit gemeinsamer Prägung sind in einer bestimmten Gruppe anzuordnen, die zu einem bestimmten Faltungsweg gehört. Dadurch unterscheiden sich die abgefangenen Intermediate nicht nur von der hydrodynamischen Größe und dem Zeitpunkt der Entstehung, sondern auch von ihrer Wegezugehörigkeit der Faltung.

Diese in zeitlichen Abständen abgefangenen Intermediate können entweder mit der erfindungsgemäß modifizierten und verbesserten nativen Polyacrylamidgelelektrophorese aufgetrennt, differenziert, über ihre Menge, die als vierte Charakteristik, der Intermediate bezeichnet ist, quantifiziert, kinetisch verfolgt und mit ihrer hydrodynamischen Größe als Funktion des Zeitablaufs der Faltung zweidimensional dargestellt werden oder zuerst flüssigchromatograpbisch aufgetrennt, quantifiziert und dann je nach Bedarf und Wunsch mit den spektroskopischen oder/und thermodynamischen Verfahren nach ihren hydrodynamischen Größen differenziert und dann in zweidimensionaler Form, also ihrer hydrodynamischen Größe (ggf. mit quantifizierter Menge) als Funktion des Zeitablaufs der Faltung, wieder dargestellt werden.

Diese zweidimensionale Darstellung präsentiert ein Rückfältungsfingerabdruckprofil eines Proteins, der jeweils vor allem von 3 Charakteristiken der Intermediate, der hydrodynamischen Größen, den Zeitpunkten ihrer Entstehung und der quantifizierten Mengen eindeutig geprägt ist Die Faltungswegezugehörigkeiten aller bei der Rückfaltung abgefangenen und aufgetrennten Intermediate können zunächst mithilfe der folgenden Kriterien einordnet werden, die auch von der Proteinevolutionstheorie und der Proteinfaltungskinetik unterstützt werden:

- die zuerst mit der größten Menge erscheinenden Intermediate gehören meistens zum nativen dominanten Faltungsweg und auf diesem Faltungsweg entstehen die wenigsten Intermediate,

- das zu den nativen Faltungswegen gehörende Intermediat verfügt meistens über eine Konvergenz-Struktur und das zu den nicht nativen Faltungswegen oder zu dem Mißfaltungsweg gehörende Intermediat besitzt hingegen oft DLS-detektierbare Divergenz-Strukuren,

- die in gleicher quantifizierter Menge erscheinenden Intermediate gehören meistens zu demselben Faltungsweg und diese Menge entscheidet über die Breite des Faltungswegs oder den Durchmesser des Faltungskanals, also die Kompetenz eines Faltungswegs.

Die weitergehend genauere Einordnung der Faltungswegzugehörigkeit beginnt mit der proteolytischen und chemischen Fragmentierung der aufgetrennten Intermediate. Die anschließende Klassifizierung der Fragmente erfolgt durch den bioinformatikunterstützten Vergleich mit den theoretischen Fragment-Massenerkennungsmustern. Hierbei erfolgt die Klassifizierung der Fragmente nach der Distribution ihrer von den Typen und Ausmaßen der Modifizierung bestimmten Molekolgewichten und ihren untereinander möglicherweise erfolgten Bindungsmustern der Disulfidbildung und/oder der eingefügten Crosslinker, die zur Identifizierung der sich um die Faltungswege gruppierenden Intermediate und zur Schlussfolgerung der Wegezugehörigkeit der Faltung führen können. Hiermit werden Massenspektrometrien (z. B. ESI-TOF-MS, Elektrospray-Flugzeit-Massenspektrometrie und MALDI-TOF-MS, Matrix-unterstützte Laser-desorptionst-Ionisations-Flugzeit-Massenspektrometrie) zur Erfassung der Anzahl der Fragmente und deren Molekülgewichte eingesetzt.

Die dadurch zugeordneten Intermediate lassen sich je nach Gemeinsamkeit ihrer Charakteristik in den zugehörigen Gruppen qualifizieren. Die sich im Zeitablauf gruppierenden Intermediate indizieren jeweils einen Faltungsweg. Der Faltungsvorgang eines Proteins wird dann als vollständig charakterisiert, wenn alle existierenden Faltungswege identifiziert werden.

Hierbei wurde erfindungsgemäß gefunden:

- Ein optimal vollständig zum Zufallsknäuel (Random Coil) entfaltetes Protein enthält mindestens zu einem kleinen Bruchteil die strukturell leicht geformten ursprünlichen Populationen. Diese Populationen können jeweils als Mischung der anfänglichen Intermediate betrachtet werden, die über unterschiedliche initiale Sekundärstrukturen in ihren eigenständigen Mikrodomainen verfügen und sich hierarchisch und gruppierend mit sehr niedrigen Energiedifferenzen verteilen und daher auf die Ursprünge der Faltungswege oder die Quellen der Faltungskanäle hindeuten.

- Die Rückfaltung eines Proteins kann in 4 mit eigenständiger Zeitskala und Tempo nacheinander erfolgten Phasen eingeteilt werden, nämlich der in Nano- bis Millisekunden superschnell erfolgten Phase-I für die Entstehung der Populationen der Seed-Strukturen zum Start des Aufbaus der Faltungswege, der in Mikro- bis Sekunden schnell erfolgten Phase-II für den Aufbau der parallelen Faltungswege, der Sekunden bis Minuten dauernden Phase-III für das nachfolgende Durchlaufen der Faltungen entlang der aufgebauten Wege und der je nach strukturellem Aufbau des jeweiligen Proteins in unterschiedlichen Tempi erfolgten Phase-IV für die weitergehenden intramolekularen Rearrangements der Mikroregionen und die Evolution der Tertiärstruktur.

- Die in Phase-I erbrachten Populationen verschiedener Seed-Strukturen sind nicht zufällig entstanden, sondern von der Primärstruktur des Proteins definiert. Sie entscheiden weiterhin über die nachkommenden Faltungswege. Die in Phase-II parallel aufgebauten Faltungswege sind vielfältig und voneinander unabhängig. Jeder Faltungsweg besteht aus mehreren oder wenigen sich gruppierenden metastabilen Intermediaten, die die Trittsteine der jeweiligen Faltungswege markieren. Die Phase-II ist zu dem Zeitpunkt beendet, in dem das renaturierte Protein vorkommt und die höchste Anzahl der Intermediate erscheint. Die Rückfaltung in Phase-III läuft parallel entlang der in Phase-II aufgebauten eigenständigen Faltungswege und folgt zeitgleich mit der in Phase-II entstehenden Kinetik bis zum Zeitpunkt, in dem allen anfänglich nicht strukturierten Populationen im Pool des entfalteten Proteins und die meisten gewesenen Intermediate der Faltungswege nicht mehr vorhanden sind. In Phase-IV wird die native Struktur eines renatuierten Proteins vervollständigt.

- Jedes dabei entstehende Intermediat kann mit mindestens 4 eigenen individuellen Charakteristiken identifiziert und bezeichnet werden, nämlich der hydrodynamischen Größen, dem Zeitpunkt der Entstehung, der Zugehörigkeit zu den Faltungswegen und der zu quantifizierenden Menge.

- Die Faltungswege können je nach ihrer Faltungskompetenz allgemein in 3 Arten klassifiziert und jeweils als Primär-, Sekundär- und Missfaltungsweg bezeichnet werden. Diese Faltungswege schliessen womöglich weiterhin mit eigenen nachrangigen Faltungswegen parallel an. Die Faltung auf den Primärfaltungsweg donimiert den Faltungsvorgang. Die Faltung auf den Sekundärfaltungsweg liefert zum Schluß angemessene Intermediate mit flexibelen nativen untereinander ähnlichen Strukturen, die durch intramolekulare Umlagerung zum nativen Protein umwandeln können. Die Faltung auf den Missfaltungsweg erbringt die fehlerhaften Intermediate, die entweder sehr langsam durch intramolekulare Umlagerung zum nativen Protein umwandeln oder bis zum Ende der Faltung als missgefaltetes Protein übrigbleiben.

- Der Sekundär- und Missfaltungsweg können sich durch ihre Abzweigungen untereinander vereinen oder jeweils auf den Primärfaltungsweg in einer energiearmen Richtung zusammenfließen. Der nachrangige Faltungsweg kann auch durch die Abzweigung mit seinen Hauptweg fusionieren, Dabei entsteht ein bestimmtes Intermediat, das zu beiden Faltungswegen gehört.

- Die Verhältnisse der parallenen Faltungswege können durch Regulierung der Reaktionsbedingungen z. B. die Konzentration des Denaturierungs-, Reoxidations und Stabilisierungsmittels, des pH-Werts und der Ionenstärke, der Temperatur, des Einsatzes der Chaperone oder Foldasen usw. verändert werden. Die Rückfaltung eines Proteins kann dadurch z. B. zugunsten der Faltung auf dem Primärfaltungsweg beschleunigt werden, um die Produktivität eines durch Renaturierung gewonnenen Proteinwirkstoff zu erhöhen, oder dadurch kontrolliert verlangsamt werden, um die Proteinaggregation zu reduzieren oder die Charakterisierung des Faltungsvorgangs zu erleichtern. Aber die von der Primärstruktur des Proteins definierten Hauptfaltungswege und ihre nachrangigen Faltungswege können nicht dadurch geändert werden, das heißt, es entsteht dabei kein neuer Faltungsweg, bzw. keine gewesenen Faltungswege werden dadurch verschwinden. Hierbei soll der dadurch verstärkt präsentierte Faltungsweg nicht als neuer Faltungsweg identifiziert und die dadurch schwächer präsentierten Faltungswege nicht als ungewesenen Faltungswege ignoriert werden. Es kann auch das Intermediat geben, das zum Missfaltungsweg gehört und über eine hydrodynamische Größe und somit einen Energiezustand kleiner als sein natives Protein verfügt.

- Die strukturelle Heterogenität eines rückfaltenden Proteins nimmt zuerst bis zum Ende der Phase-1 superschnell und sprunghaft ab und reduziert sich dann durchschnittlich langsamer und stufenweise bis zum Ende der Phase-IV. Die kinetischen Meilensteine der starken Abnahme der strukturellen Heterogenität werden den Stufen der Tempolimite entsprechend durch die relativ stabilen Strukturen der Intermediate sichtbar gemacht und befinden sich jeweils am Ende der Phase-1 bis -3. Die Abnahme der strukturellen Heterogenität erscheint deshalb stufenweise und allgemein proportional zu der Abnahme der hydrodynamischen Größen eines rückfaltenden Proteins. Der Abnahmeprozess der strukturellen Heterogenität während der Rückfaltung eines Proteins ist von seiner Art, seinem Typ und somit von seinen Faltungswegen abhängig.

- Der Faltungsvorgang eines Proteins kann vorwiegend durch die gesamten Charakteristiken aller in den Phasen -1 und -2 der Faltung vorkommenden Intermediate unter Standardbedingungen charakterisiert und als Faltungsfingerabdruck eines Proteins bezeichnet werden.

- Auf Basis dieser gefundenen neuen Erkenntnisse wird ein den Energielandschaften entsprechendes trichterförmiges Multifaltungswege-Modell (Figur-11) etabliert. Das Modell ist übertragbar in einem multidimensionalen Koordinatensystem. Die 4 phasigen Faltungen werden in diesem Modell weiter mit 5 oft in Experimenten erscheinenden Funktionszonen jeweils in verschiedenen graphischen Formen dargestellt, wobei der Energiezustand und die hydrodynamische Größe als Funktion der Faltungszeit entsprechend eingezeichnet werden. Dies wird im Folgenden beschrieben und interpretiert:

- Die oberste Zone-A wirkt wie ein Pool des im Random Coil entfalteten Proteins, zum Teil mit leicht unterschiedlich strukturierten Populationen. Bedingt durch den instinktiven Antrieb und die räumliche Verfügbarkeit erfolgt die Rückfaltung der Phase-1 in Millisekunden superschnell. Die Rückfaltung in Zone-B bildet einige dominante strukturelle Populationen, die initial native Sekundärstrukturen besitzen und über ähnliche hydrodynamische Größen und Energiezustände verfügen. Das rückfaltende Protein erreicht damit sein erstes Tempolimit infolge der Erschöpfung der vorgezogenen Sekundärstrukturbildung.

- Der Aufbau der Multifaltungswege in Phase-II beginnt mit dem ersten vorkommenden Intermediat am Ende der Zone-B und ist beendet beim Erscheinen des renaturierten Proteins in der Zone-E. Die Populationen mit den initial nativen Sekundärstrukturen, auch als Seedpopulationen der jeweiligen Faltungswege genannt, überwinden zunächst die individuellen Hauptenergiebarrieren und falten dann mit eigenständigem Tempo über einige strukturell metastabile Stufen, die in Form von Intermediaten erscheinen und als markierte Trittsteine auf dem jeweiligen Faltungsweg bezeichnet werden, durch den zusammentreffenden Molten-Globule-Zustand bis zur renaturierten Struktur zurück.

- Die auf dem Primärfaltungsweg faltende Population einschließlich ihrer potenziellen Subpopulationen verfügt über gleichzeitig gebildete native Sekundärstrukturen, die die Rückfaltung dominieren werden. Die Rückfaltung auf diesem Weg mit sehr schnellem Tempo erbringt kaum Intermediat. Der Sekundänfaltungsweg stammt aus der Population mit teilweise gebildeten nativen Sekundärstrukturen und besteht deshalb aus mehr oder wenigen nativen Intermediaten. Der Missfaltungsweg entsteht aus den Populationen mit den fehlerhaft gebildeten nicht nativen Sekundärstrukturen und wird daher oft von mehreren nicht nativen Intermediaten begleitet. Die den Hauptfaltungsweg parallel begleitenden nachrangigen Faltungswege entstammen aus strukturellen Subpopulationen mit jeweils abweichender Sekundärstrukturenbildung und führen meist während der Faltung durch die intramolekulare Umlagerung zum Hauptfaltungsweg zurück.

- Die potentielle Komplexität der Multifaltungswege wird in diesem Modell symbolisch repräsentiert. Es wird gezeigt, dass jede Art der Seedpopulation, die jeweils für die Bildung der symbolisierten Primär-, Sekundär- und Missfaltungswege verantwortlich ist, zunächst jeweils die 3 in unterschiedlicher Stärke definierten Energiebarrieren überwinden soll, um den Zugang zu den Faltungswegen zu erreichen, und dann jeweils entlang der 3 alternativen Faltungswege mit individuellen Nebenenergiehindernissen rückfalteten, wobei sich die entsprechenden Intermediate entwickeln. Dadurch entstehen womöglich mindestens 27 Faltungswege, ohne die durch Abzweigung erbrachten Faltungswege mitzuzählen. Aber die Rückhaltung eines Proteins kann meist je nach eigener Natur nur sehr beschränkt auf bestimmten dazugehörigen Faltungswegen parallel erfolgen.

- Die Faltungswege treffen zusammen im Molten-Globule-Zustand in Zone-D. Dabei erfolgen die meisten intramolekularen Umlagerungen einschließlich der nativ orientierten Disulfidumlagerungen der Intermediate aus verschiedenen Faltungswegen.

- Das entlang der Multifaltungswege und über den Molten-Globule-Zustand rückfaltende und zunächst in Zone-E renaturierte Protein besitzt die variierenden und flexiblen Strukturregionen, die der Proteinevolution zugrunde liegen und für die Einbehaltung seiner Anpassungsfähigkeit und Funktionalität gegenüber den Substraten verantwortlich sind. Dieses renaturierten Protein lagert seine Struktur kontinuierlich vollständig nativ um während des weitergehenden Durchlaufs der Faltung in Phase-III und bis zum Ende der Phase- IV. Die Zone-E vertritt dabei nicht nur die renaturierte und native Struktur des Proteins, sondern auch die durch intramolekulare Umlagerung zurückgeführten Strukturvarianten, inklusive der missgefalteten Strukturen, deren hydrodynamische Größe und Energiezustand manchmal sogar niedriger als nativ sind.

- Das Modell zeigt 4 Tempolimite der Proteinfaltung. Das erste befindet sich am Ende der Phase-I und zugleich dem Eingang der Hauptenergiebarrieren der Faltungswege. Das zweite sind die stufenweise vorkommenden Nebenenergiebarrieren, erscheinend in Form von sequenziellen Intermediaten auf den Faltungswegen. Das dritte entsteht beim Molten-Globule-Zustand infolge der intramolekularen Umlagerungsbarrieren und Einsturzbarrieren zum renatuierten Protein. Das vierte geht auf die Rearrangementbarrieren zur Vervollständigung der nativen Struktur des Proteins zurück. Das erste und dritte Tempolimit haben eine starke Engpasswirkung. Sie entscheiden vorwiegend über die Faltungsgeschwindigkeit eines Proteins. Die unterschiedlichen Stärken der Tempolimite sind quantitativ bestimmbar durch die Analyse der Mengeverteilung der auf den jeweiligen tempolimitierten Stadien akkumulierten Intermediate.

- Das Modell besagt, dass der Faltungsvorgang eines Proteins durch die Identifizierung der parallel an den Tempolimitstadien und dazwischen entstehenden Intermediate charakterisiert werden kann. Ein aus den Faltungswegen und einbezogenen Intermediaten bestehendes Muster kann dadurch als Grundlage des experimentellen Faltungsfingerabdrucks erstellt werden,

- Das Modell zeigt, dass die Faltung auf Multifaltungswege prinzipiell nicht kooperativ wirkt. Das kooperative Verhalten wird nur dann geschehen, wenn die Faltung dominierend auf dem Primärfaltungsweg läuft.

- Das Modell gilt für alle Proteine. Jedes Protein, egal ob es auf dem Singlefaltungsweg oder Multifaltungsweg, superschnell oder langsam faltet, oder zuerst superschnell und dann langsam faltet, oder nur langsam faltet, kann in diesem Modell seine eigenen Faltungswege entsprechend wiedergefunden werden. Das Modell wird daher unter anderen vor allem für die Verbesserung der Konstruktion, Rationalisierung der Überprüfung und Orientierung der Optimierung der Verfahrensschritte und somit für die optimale Ausführung und Anwendung des erfindungsgemäßen Verfahrens gebraucht.

3) Der dadurch charakterisierte Faltungsvorgang kann multidimensional dargestellt werden, indem die Charakteristiken aller Intermediate zuerst digitalisiert und die hydrodynamische Größe aller Intermediate jeweils gegen ihren Zeitablauf der Entstehung, der Faltungswege und gegebenenfalls ihre quantifizierte Mengen in einem multidimensionalen Koordinatensystem angegeben wird. Die Darstellung des Faltungsvorgangs kann durch neue Kombinationen der mit diesen 4 Charakteristiken definierten Koordinaten und den Einsatz der Computergraphik in Vielfalt visualisiert werden. Hierbei lassen sich z. B. die Faltungswege (der Aufbau der Faltungskanäle), ihr kinetischer Ablauf, ihr prozentualer Beitrag zur Faltung, der Vorgang der Missfaltung, die Entstehung der intermolekularen Umlagerung, die Reihenfolge der Disulfidbildung, die Wirkungen der biologischen und chemischen Einflussfaktoren auf Faltung, der Vorgang des Beginns der Aggregation der Proteine, die *in vitro* Wirkungen eines Faltungsinhibitors oder -chaperons auf die Aktivität, Funktionalität und den Abbau eines Proteins sowie der Vorgang einer *in vitro* simulierten dynamischen co- und posttranslationalen Modifizierung usw. aus der veranschaulichten Graphikdarstellung eines charakterisierten Faltungsvorgangs leicht ermitteln. Die Beziehung zwischen räumlicher Struktur und Energielandschaft aller Intermediate eines rückfaltenden Proteins kann sowohl dem Trichter-Konzept (Schultz, 2000) entsprechend qualitativ und quantitativ dargestellt werden, als auch in anderen vielfältigen Formen der Energielandschaft multidimensional visualisiert werden.

**Detaillierte Beschreibung der Erfindung**

Das erfindungsgemäße. Verfahren wird zuerst allgemein in den folgenden Schritten vereinfacht erfasst und dann Schritt für Schritt detailliert.

1. Allgemeine Analytik des zu charakterisierenden Proteins

Auf Basis der Analyse der Merkmale der primären, sekundären und gegebenenfalls dreidimensionalen Struktur und der biologischen und der physikochemischen Eigenschaften des Proteins wird zunächst das theoretische Fragmenten-Massenerkennungsmuster hergestellt und entschieden, welche Vorgehensweise zuerst angewendet wird, die sich zum Beispiel auf die Modifikationsart, das seitenkettenspezifische Reagenz und die Methode der Auftrennung der Intermediate bezieht.

2. Auftrennung der optimal entfalteten Proteinprobe mit maximierten hydrodynamischen Größen

Das Protein wird denaturiert, reduziert und gegebenenfalls von Reduktionsmittel befreit. Die chromatographisch abgetrennte beste Proteinprobe verfügt über eine maximale hydrodynamische Größe, wobei die Disulfidbrücken des disulfidhaltigen Proteins zu freien Thiolgruppen komplett reduziert und vollständig entfaltet werden.

3. Dynamische Proteinmodifikationen

Das rückfaltende Protein wird dadurch in zeitlichen Abständen zum Beispiel unter oder ohne Einflussfaktoren mit Seitenkettenreagenzien je nach Zugänglichkeit der betreffenden Reste der Aminosäure in unterschiedlichem Ausmaß modifiziert und zu den vielfältigen und strukturell relativ stabilen Intermediaten jeweils mit eigenen individuellen Charakteristiken, nämlich der hydrodynamischen Größe, dem Zeitpunkt der Entstehung, der Zugehörigkeit der Faltungswege und dem prozentualen Beitrag zur Faltung gebracht und für die Auftrennung und Identifizierung bereitgestellt werden.

4. Auftrennung und Quantifizierung der Intermediate und zweidimensionale Darstellung ihrer hydrodynamischen Größen und Stoffmengen als Funktion ihrer Entstehungszeitpunkte

Die bei der Rückfaltung in zeitlichen Abständen durch Modifizierung abgefangenen Intermediate werden bevorzugt mittels der erfindungsgemäß verbesserten Gelelektrophorese aufgetrennt und durch Scanning der Gelbandenintensität quantifiziert, wobei die sich in verschiedenen Gelbanden befindenden. Intermediate mit ihren unterschiedlichen hydrodynamischen Größen als Funktion ihres Rückfältungszeitablaufs zweidimensional auf dem Gel dargestellt werden. Sie können auch zuerst flüssigmikrochromatographisch aufgetrennt und dann der spektroskopischen Differenzierung ihrer hydrodynamischen Größe und zugleich der Quantifizierung ihrer Menge unterzogen und anschließend dem 4 phasigen Multifaltungswege-Modell entsprechend in eine multidimensionale Darstellung gebracht werden, indem die Reihenfolge ihrer hydrodynamischen Größen und ihrer quantifizierten Mengen gegen den Zeitverlauf der Rückfaltung aufgetragen wird. Dadurch werden alle Intermediate nach ihren hydrodynamischen Größen, Mengen und Entstehungszeitpunkten tabellarisch angeordnet. Die hydrodynamischen Größen der Intermediate können hierbei auch gegebenenfalls durch ihre nach der Auftrennung gemessenen thermodynamischen Größen ausgetauscht werden.
Ein Rückfaltungsfingerabdruckprofil eines Proteins kann hierbei zunächst erstellt werden, indem die bis zum Ende der Aufbauphase der Faltungswege entstehenden Intermediate mit ihren unterschiedlichen hydrodynamischen Größen und quantifizierten Mengen als Funktion ihres Rückfaltungszeitablaufs entweder elektrophoretisch auf dem Gel oder nach der flüssigchromatographischen Auftrennung und Quantifizierung mit Hilfe der Computergraphik zweidimensional dargestellt werden.

5. Fragmentierung der Intermediate durch In-Gel-Verdau oder In-Lösung-Verdau

Mit Fragmentierung beginnt die weitere genauere Differenzierung der Wegezugehörigkeit aller Intermediate. Alle gelelektrophoretisch oder mikrochromatographisch nach der hydrodynamischen Größe aufgetrennten Intermediate werden enzymatisch vorzugsweise mit Trypsin in die Fragmente gespalten. Die Fragmentierungen können zusätzlich unter anderem mit den Endoproteasen Lys-C, Glu-C und Asp-N, wenn notwendig auch mit ausgewählten Exoproteasen oder chemisch erfolgen.

6. Massenspektrometrische Detektion der fragmentierten Intermediate

Die Molekulargewichte aller einzelnen Fragmente und die durch Disulfidbrücken und/oder Crosslinker gebundenen großen Fragmente der Intermediate werden massenspektrometrisch (ESI-TOF-MS, Elektrospray-Flugzeit-Massenspektrometrie und MALD-TOF-MS, Matrix-unterstützte Laser-desorptions/-Ionisations-Flugzeit-Massenspektrometrie) gemessen und in einer Datenbank erfasst. Hierbei können MALDI-TOF-MS/MS(Tandem-Massenspektrometrie) oder MALDI-TOF-MS-PSD (Post-Source-Decay), oder zusätzliche exoenzymatische und/oder chemische Hydrolyse für die exakte Differenzierung sich ähnelnder Fragmente eingesetzt werden.

7. Feststellung der Faltungswegezugehörigkeit der Intermediate

Durch Vergleich der massenspektrometrisch erfassten Anzahl und Masse der Fragmente mit den theoretischen Fragmenten-Massenerkennungmustem werden die Art, die Anzahl und der Ort der erfolgten Modifikation als eigene individuelle Merkmale für jedes Intermediat ermittelt Aufgrund dieser Merkmale lassen sich die Intermediate auf Gemeinsamkeiten zu einigen anderen Intermediaten in bestimmte Gruppen einordnen und durch diese Gruppenzugehörigkeit kann man auf einen bestimmten Faltungsweg schließen. Wird die Gruppenzugehörigkeit eines Intermediates festgelegt, ist seine Faltungswegezugehörigkeit zugleich festgestellt. Diese Feststellung wird dabei immer durch die vorstehend beschriebenen Kriterien, die von den Proteinevolutionstheorie und Proteinfaltungskinetik unterstützt werden, kompensiert und komplementiert.

8. Identifizierung und Klassifizierung der Intermediate

Dadurch kann jedes Intermediat mit seiner zuvor festgestellten hydrodynamischen Größe, dem Zeitpunkt seiner Entstehung, der quantifizierten Menge und der dabei zusätzlich ermittelten Zugehörigkeit der Faltungswege bezeichnet und mit diesen 4 wichtigen Charakteristiken identifiziert werden. Alle identifizierten Intermediate lassen sich je nach Gemeinsamkeit ihrer Merkmale in unterschiedliche Gruppen einordnen und weiterhin jeweils nach der Gruppenzugehörigkeit in verschiedene Faltungswege klassifizieren. Die in bestimmten Faltungswegen klassifizierten Intermediate können u. a. noch drei Kriterien erfüllen, nämlich Vorhandensein gleicher Merkmale der Modifikation, allmähliche Verkleinerung ihrer gruppenzugehörigen hydrodynamischen Größe und die genauen Zeitpunkte der Entstehung jedes um den Faltungsweg gruppierten Intermediates.

9. Charakterisierung des Faltungsvorgangs

Durch die parallele Darstellung aller in unterschiedlichen Faltungswegen eingeordneten Intermediate jeweils mit ihren identifizierten Charakteristiken in einem multidimensionalen Koordinatensystem können die den Wegen zugehörigen Faltungstempi, der dominante Weg der nativen Faltung, der Weg der schnellsten und langsamsten Faltung, der Weg zur Missfaltung, die Faltungskinetik in allen Wegeabläufen, die Reihenfolge der Disulfidbildung und/oder Crosslinkerbildung, die intermolekularen Umlagerungen und die Faltungswege-Abzweigung, - Erweiterung, -Kreuzung, -Überquerung und -Zusammentreffen sowie die dabei betroffenen Intermediate usw. direkt ermittelt werden. Der Faltungsvorgang eines Proteins wird dadurch charakterisiert.

10. Graphische und visuelle Darstellung des Faltungsablaufs

Der charakterisierte Faltungsvorgang kann mithilfe der Computergraphik in einem multidimensionalen Koordinatensystem in Vielfalt visualisiert und zur Animation gebracht werden, wobei die Faltungswege auch z.B. als Faltungskanäle für die Multikoordinatendarstellung und Faltungstunnel für die Trichterlandschaften usw. genannt werden können.
Die Ausführung des Verfahrens kann sowohl Schritt für Schritt manuell als auch teilweise oder/und vollständig automatisiert erfolgen, wobei die Mittel zur Ausführung in Form von unterschiedlichen Assay-Kits, Geräten und Software sowie den spezifischen konzipierten und entworfenen Automaten im Einsatz gebraucht werden.

1. Allgemeine Analytik des zu charakterisierenden Proteins

Als erster Schritt wird die Analyse der Merkmale der primären, sekundären und dreidimensionalen Struktur und der physicochemischen Eigenschaften des zu charakterisierenden Proteins vorgenommen, um die entsprechende Entscheidung zu treffen, mit welcher Vorgehensweise die Charakterisierung durchzuführen ist, wobei alle Schritte des Verfahrens mit ihren individuellen Handhabungen und die Auswahl der Modifikationsart, des seitenkettenspezifischen Reagenzes und der Methode der Auftrennung der Intermediate und der Differenzierung ihrer hydrodynamischen Größen inklusive der Untersuchungsbedingungen wie z.B. die Proteinkonzentration, die Lösungsmittel, die Ionenstärke, der pH-Wert und die Temperatur usw. festgelegt werden.

Die erfindungsgemäß speziell entwickelten Programme für die Klassifizierung des Proteins nach der Größe des Molekulargewichts, der Art von disulfidhaltig oder disulfidfrei, der Type von hydrophil oder hydrophob, der Eindomäne- oder Multidomänenproteine, für die Auswahl der seitenkettenspezifischen Modifikationsreagenzien, für die Herstellung und Auswertung der theoretischen Fragmente-Massenerkennungsmuster basierend auf der proteolytischen Spaltung der modifizierten Intermediate und für die Herausstellung, Differenzierung und Auswertung der theoretischen Fragmente-Massenerkennungsmuster der proteolytisch gespalteten Intermediate in Bezug auf alle Arten der erfindungsgemäßen Modifikationen können hierbei eingesetzt werden.

Bei der Analytik in Bezug auf die Anwendungen der Erfindung können die zusätzlichen Programme wie zum Beispiel für die Herausstellung und Auswertung der theoretischen Fragmente-Massenerkennungsmuster der *in vitro* posttranslational modifizierten und proteolytisch gespalteten Intermediate und für die Herausstellung und Auswertung der theoretischen Fragmente-Massenerkennungsmuster der bei *in vitro* simulierten Rückfaltung unter chemischen und/oder biologischen Einflussfaktoren modifiziert und proteolytisch gespalteten Intermediate benutzt werden.

2. Auftrennung der optimal entfalteten Proteinprobe mit maximierten hydrodynamischen Größen

Das optimal entfaltete Protein verfügt über eine maximierte hydrodynamische Größe. Die Abtrennung dieser Proteinprobe geht von der Denaturierung und Reduktion des Proteins aus und eignet sich sowohl für disulfidhaltige als auch für disulfidfreie Proteine. Das disulfidhaltige Protein wird in einem Ansatz mit Denaturierungsmittel und Reduktionsmittel zugleich denaturiert, reduziert und anschließend chromatographisch vom Reduktionsmittel befreit und dabei in verschiedenen Proteinproben mit den differenzierten hydrodynamischen Größen aufgetrennt. Die Abtrennung des Reduktionsmittels für bestimmte Proteine, deren intermolekulare Reoxidation leicht stattfindet, sollte schrittweise veranlasst werden. Durch die spektrometrische Untersuchung z.B mit DLS und die Bestimmung der reduzierten Thiol-Reste im Molekül mit bekanntem Ellmansreagenz wird festgelegt, ob das zu untersuchende Protein vollständig denaturiert und seine Disulfidbrücken zu freien Thiolgruppen komplett reduziert worden sind. Die beste aufgetrennte Proteinprobe mit sicher vollständig reduziertem und denaturiertem Protein verfügt über die maximale hydrodynamische Größe und wird als Ausgangsmaterial für die anschließende dynamische Modifizierung verwendet. Die Denaturierung und Reduzierung erfolgen in einer Pufferlösung mit bekanntem Denaturierungs- und Reduktionsmittel, z. B. mit Guanidiniumchlorid und DTE (1,4-Dithioerythrit). Die Konzentration beträgt jeweils bis zu 20mg/ml für Proteine, bis zu 8M für das Denaturierungsmittel und bis zu 0,4M für das Reduktionsmittel. Die Entfaltung des Proteins kann gegebenenfalls durch Erhöhung der Temperatur, Einsatz der Hilfssubstanzen und Einstellung des pH-Werts usw. maximiert werden. Die Chromatographie zur Entfernung des Reduktionsmittels erfolgt vorzugsweise mit dem Puffer aus hochkonzentriertem Denaturierungsmittel in niedrigem pH-Wert. Die Denaturierung oder/und Entfaltung der disulfidfreien Proteine erfolgt meistens ohne Reduktionsmittel. Die Überprüfung und Festlegung ihrer besten Proteinprobe mit der maximalen hydrodynamischen Größe erfolgen spektrometrisch vorzugsweise mit DLS nach der flüssigchromatographischen Auftrennung.

3. Dynamische Proteinmodifikationen

Das optimal vollständig denaturierte Protein verfügt über eine maximale hydrodynamische Größe. Durch schnelle Konzentrationsabnahme des Denaturierungsmittels bei der Verdünnung der Ansatzprobe oder durch Änderung der Temperatur und des pH-Werts beginnt das denaturierte und entfaltete Protein sich wieder zurückzufalten, wobei seine hydrodynamische Größe allmählich verkleinert wird. Bei der chemischen oder biologischen Modifizierung mit zeitlichen Abständen wird die Rückfaltung des Proteins durch die räumliche Störung der modifizierten Gruppen der Seitenketten angehalten. Das Protein wandelt sich dabei zu den vielfältigen und strukturell relativ stabilen Intermediaten um, wobei die Strukturen der Intermediate durch das eingesetzte Reoxidationsmittel für die reduzierten disulfidhaltigen Proteine oder Vernetzungsmittel für die disulfidfreien Proteine mit individuellen Merkmalen der neu gebundenen Disulfidbrücken oder CrossLinker-Bindungen zusätzlich geprägt werden. Es wurde erfindungsgemäß gefunden, dass die Vielfalt der Modifizierung des Proteins als Hauptgrundlage und wesentliche Voraussetzung für die vollständige Charakterisierung des Faltungsvorgangs dienen kann und dass die vom Reagenz und der Methode abhängige Reaktionsgeschwindigkeit der Modifikation über die Genauigkeit des charakterisierten Faltungsvorgangs insbesondere über die erste schnelle Phase des Faltungsvorgangs entscheiden kann. Hierbei gewährleistet die Vielzahl der Möglichkeiten zur Modifikation des rückfaltenden Proteins allen bei der Faltung entstehenden Intermediaten mit den für sich beanspruchten und geeigneten Reagenzien entsprechend modifiziert zu werden, nach ihren strukturellen Merkmalen differenziert und jeweils nach ihren individuellen Charakteristiken identifiziert zu werden. Die Modifikation kann je nach der Zielsetzung der Charakterisierung jeweils in verschiedenen Ausführungsformen erfolgen und je nach der Art, Type, und Größe der zu untersuchenden Proteine mit den passenden Vorgehensweisen und Methoden sowie auszuwählenden Chemikalien und Materialien wie im Folgenden zum Einsatz kombiniert werden.

Die Proteinmodifizierung erfolgt erfindungsgemäß gezielt an denjenigen Resten zum Beispiel von Cystein, Lysin, Tyrosin, Histidin, Arginin, Tryptophan, Methionin, Glutaminsäure, Asparagin und Asparaginsäure einschließlich des N- und C-Terminus des rückfaltenden Proteins und läßt sich erfindungsgemäß je nach ihrer Art, Type und Ausmaß sowie der Zielsetzung der Anwendungen in die folgenden Ausführungsformen einteilen:

- die dynamische Modifizierung der disulfidhaltigen Proteine,

- die dynamische Modifizierung der disulfidfreien Proteine,

- die dynamische Modifizierung der Multidomänenproteine,

- die simuliert dynamische *in vitro* co- und posttranslationale Modifikation,

- die dynamische Modifizierung bei simulierter *in vitro* Proteinfaltung

- die dynamische Modifizierung bei *in vitro* Proteinbiosynthese

Unter dynamischer Modifizierung der disulfidhaltigen Proteine ist erfindungsgemäß zu verstehen, dass die reduzierten und denaturierten disulfidhaltigen Proteine bei ihrer Reoxidation in zeitlichen Abständen portionsweise herausgenommen, vorzugsweise durch die Singlemodifizierung mit den Seitenkettenreagenz zur Blockierung der noch freien Thiolgruppen zusammengemischt und durch Anhalten ihrer faltenden Strukturen zu den vielfältigen Intermediaten mit unterschiedlicher struktureller Gestaltung und hydrodynamischen Größen gebracht werden. Hierbei werden verschiedene native und nicht-native Disulfidbildung, die zum Erkennungsmuster der charakteristischen Vernetzung der proteolytischen Fragmente führen können, generiert und die übrigen Thiolgruppen durch eingefügte Seitenkettenreagenzen blockiert. Diese dienen als eine Grundlage für die Klassifizierung der Gruppenzugehörigkeit der disulfidhaltigen Intermediate. Zur genauen Differenzierung der dem Faltungsweg zugehörigen Intermediate ist die Multimodifizierung erfindungsgemäß einzusetzen, wobei die Reste von Cystein, Histidin, Lysin, Methionin und Arginin sich jeweils mit den seitenkettenspezifischen Reagenzien getrennt zur Reaktion bringen lassen oder ausschließlich mit einem einzelnen Reagenz z. B. Jodacetamid unter Kontrolle der Reaktionsbedingungen, beispielsweise durch die schrittweise Veränderung des pH-Werts.

Unter dynamischer Modifizierung der disulfidfreien Proteine ist erfindungsgemäß zu verstehen, dass die denaturierten Proteine, die keine fähigen Thiolgruppen zur Disulfidbildung haben, bei ihrer Rückfaltung in zeitlichen Abständen portionsweise herausgenommen und anschließend je nach der Besonderheit der sequenziellen Verteilung und strukturellen Zugänglichkeit ihrer modifizierbaren Seitenketten sowie ihres enzymatischen Spaltungsmusters der Singlemodifizierung, der Multimodifizierung und/oder der internen Crosslinker-Modifizierung unterzogen werden. Die Singlemodifizierung richtet sich auf die einzelne Aminosäure, die in ihrer Primärstruktur genug vorhanden und gut verteilt und leicht mit ausgewähltem einzelnen Seitenkettenreagenz zu modifizieren ist. Die Multimodifizierung eignet sich für einige Aminosäuren, die im einzelnen nicht gut, aber zusammen optimal an der Sequenz verteilt und leicht mit wenigstens einem Seitenkettenreagenz modifiziert werden können. Diese ausgewählten Aminosäuren können jeweils mit dem einzelnen Seitenkettenreagenz im gleichen Ansatz durch Veränderung der Reaktionsbedingungen oder durch die Mischung der parallelen mit den multiplen spezifischen Seitenkettenreagenzien getrennt durchgeführten Reaktionen modifiziert werden. Die interne Crosslinker-Modifizierung sorgt für die Einfügung der Disulfidbrückenähnlichen Bindungen zur Vernetzung der proteolytischen Fragmente. Diese Modifizierungen verleihen den Intermediaten die individuellen Merkmale der Art, Zahl und Stelle der eingefügten Reagenziengruppen sowie der Erkennungsmuster der Vernetzung der proteolytischen Fragmente, die abhängig von der Mikroumgebung des rückfaltenden Proteins sind und deshalb als Grundlage für die Bestimmung der hydrodynamischen Größe und Klassifizierung der Gruppenzugehörigkeit der disulfidfreien Intermediate dienen.

Unter der dynamischen Modifizierung der Multidomänenproteine ist erfindungsgemäß zu verstehen, dass die voneinander unabhängigen und isolierten Proteindomänen der Multidomänenproteine oder die untereinander abhängigen und zusammengesetzte Multidomänenproteine nach der Denaturierung bei ihrer Rückfaltung in zeitlichen Abständen portionsweise herausgenommen und anschließend je nach ihrer sequenziellen, strukturellen und proteolytischen Merkmalen und Eigenschaften jeweils der Singlemodifizierung, Multimodifizierung und/oder internen Crosslinker-Modifizierung selektiv unterzogen werden. Hierbei können die nulllänge, homo- und heterobifunktionelle Reagenzien sowie Biotinylierungsreagenzien usw. zur Einfügung der disulfidbrückenähnlichen Vernetzung der proteolytischen Fragmente und insbesondere die Hilfssubstanzen zur optimalen Renaturierung der großen Multidomänenproteine eingesetzt werden. Für die disulfidhaltigen Domänen- oder Multidomänenproteine ist die vollständige Reduktion, Denaturierung und anschließende Befreiung des Reduktionsmittels vor dem Start ihrer Rückfältung und Modifikationen erforderlich.

Die simuliert dynamisch *in vitro* co- und posttranslationalen Modifikationen werden erfindungsgemäß wie folgt definiert:

- Erstens, dass ein vollständig denaturiertes disulfidfreies oder ein vollständig reduziertes, denaturiertes und vom Reduktionsmittel befreites disulfidhaltiges Protein, dessen Faltungsvorgang zuvor erfindungsgemäß charakterisiert ist, den *in vitro* posttranslationalen Modifikationen entweder chemisch und enzymatisch oder in einem Ansatz mit einer bestimmten Art von Zellen-Extraktion, die spezifisch für die *in vitro* posttranslationalen Modifikationen hergestellt wurde, unterzogen wird, wobei das rückfaltende und zugleich den *in vitro* posttranslationalen Modifikationen gefolgte Protein in zeitlichen Abständen portionsweise vom Ansatz herausgenommen, gelelektrophoretisch oder/und chromatographisch in Form von unterschiedlichen Intermediaten aufgetrennt wird und in weitergehenden Schritten des erfindungsgemäßen Verfahrens für die Charakterisierung des Vorgangs und der Ausmaße der *in vitro* posttranslationalen Modifikationen mit dem Faltungsvorgang ohne posttranslationale Modifikationen verglichen wird.

- Zweitens, dass die Charakterisierung des Vorgangs der *in vitro* posttranslationalen Modifikationen durch den Einsatz des gleichen Proteins mit $^{15}$N- und/oder $^{13}$C-Isotopenmarkierung und dem weitergehenden massenspektrometrischen Vergleich quantifiziert wird, wobei die beiden Proteine mit und ohne Isotopenmarkierung zuerst in einem bestimmtem Verhältnis zusammengemischt und dann der erfindungsgemäßen Charakterisierung unterzogen werden.

- Drittens, dass ein Protein, dessen Faltungsvorgang zuvor erfindungsgemäß charakterisiert ist, der *in vitro* Biosynthese in einem Ansatz mit einer bestimmten Art von Zellen-Extraktion, die spezifisch für die *in vitro* Biosynthese und womöglich erfolgten cotranslationalen Modifikationen des Proteins hergestellt wird, für die Charakterisierung des Vorgangs und Ausmaßes cotranslationaler Modifikationen des Proteins und zur Differenzierung der co- und posttranslationalen Modifizierung unterzogen wird, wobei das in *vitro* biosynthetisierte und gegebenenfalls cotranslational modifizierte Protein gelelektrophoretisch oder/und chromatographisch vom Ansatz abgetrennt, denaturiert, reduziert und vom Reduktionsmittel befreit, zur Rückfaltung und gegebenenfalls Reoxidiation gebracht wird und weitergehenden Schritten des erfindungsgemäßen Verfahrens unterzogen wird und sein dadurch charakterisierter Faltungsvorgang mit dem erfindungsgemäß charakterisierten Faltungsvorgängen ohne und mit posttranslationalen Modifikationen verglichen wird.

- Viertens, dass ein Protein, dessen Vorgang der *in vitro* simulierten co- und/oder posttranslationalen Modifikationen zuvor erfindungsgemäß charakterisiert ist, in gleichen zellfreien Ansätzen mit zusätzlichen Kandidaten-Substanzen unter Regulierung der physiologischen und biochemischen Bedingungen für das Scanning der chemischen und biologischen Beihilfe- oder Hemmstoffe derartiger Modifizierungen und der darauf wirkenden Einflussfaktoren dem erfindungsgemäßen Verfahren unterzogen wird, wobei die Wirkungen der Kandidaten-Substanzen durch Vergleich der beiden charakterisierten Vorgänge ermittelt werden.

- Alle hierbei eingeführten *in vitro* simulierten posttranslationalen Modifikationen können in Kopplung mit der Technologie der Proteinimmobilisierung und Proteinchipherstellung im Hochdurchsatzverfahren ausgeführt werden und erfindungsgemäß in Bezug auf die erweiterten Anwendungen je nach den Zielen und Wünschen neu kombiniert und weiter ausgearbeitet werden, zum Beispiel zur Bedingungsoptimierung für die Herstellung *der in vitro* posttranslational modifizierten Pharmatherapeutika.

Die dynamischen Modifizierungen bei *in vitro* simulierter Proteinfaltung werden erfindungsgemäß mit folgenden Ausführungsformen als Beispiele definiert:

- In erster Ausführungsform ist zu verstehen, dass ein vollständig denaturiertes disulfidfreies Protein oder ein vollständig reduziertes, denaturiertes und vom Reduktionsmittel befreites disulfidhaltiges Protein, dessen Faltungsvorgang zuvor erfindungsgemäß vollständig charakterisiert ist, in den parallelen Ansätzen mit den unterschiedlichen molekularen Umgebungsbedingungen in Form von Veränderung der Proteinkonzentration, der Temperatur (inklusive Frieren und Auftauen), der Lösungsmittel, der Ionenstärke, des pH-Werts und der zusätzlichen Substanzen für die Stabilisierung oder/und Destabilisierung des Proteins zum Zweck der Charakterisierung des Vorgangs der Aggregation des Proteins während seiner Rückfaltung zu einem simulierten *in vitro* Faltungsprozess gebracht werden kann. Der Ansatz ohne Stattfinden der Aggregation dient hier als Kontrast. Hierbei wird das rückfaltende Protein in den Ansätzen in zeitlichen Abständen portionsweise herausgenommen und der selektierten Modifizierung mit dem entsprechenden Seitenkettenreagenz unterzogen, anschließend für die weitere Charakterisierung dieses Vorgangs bereitgestellt.

- In einer zweiten Ausführungsform ist zu verstehen, dass zwei oder mehr als zwei vollständig denaturierte, entfaltete und gegebenenfalls vom Reduktionsmittel befreite Proteine, deren Faltungsvorgänge zuvor erfindungsgemäß vollständig charakterisiert sind, in den parallelen Ansätzen mit den unterschiedlichen molekularen Umgebungsbedingungen in Form von Veränderung der Proteinkonzentration, der Temperatur (inklusive Frieren und Auftauchen), der Lösungsmittel, der Ionenstärke, des pH-Werts und der zusätzlichen Substanzen für die Stabilisierung oder/und Desstabilisierung des Proteins zum Zweck der Charakterisierung des Vorgangs der Interaktionen bestimmter Proteine bei ihren Rückfaltungen zu einem simulierten *in vitro* Faltungsprozess gebracht werden kann. Der Ansatz ohne Stattfinden der Interaktionen dient hier als Kontrast. Hierbei werden die rückfältenden Proteine entweder getrennt in einzelnen Ansätzen oder in einem einzigen Ansatz in zeitlichen Abständen portionsweise herausgenommen, den getrennten einzelnen Proben zum Gemisch gebracht und jeweils der selektierten Modifizierung mit dem entsprechenden Seitenkettenreagenz unterzogen, anschließend für die weitergehende Charakterisierung dieses Vorgangs bereitgestellt.

- In einer dritten Ausführungsform ist zu verstehen, dass ein vollständig denaturiertes disulfidfreies Protein oder ein vollständig reduziertes, denaturiertes und vom Reduktionsmittel befreites disulfidhaltiges Protein, dessen Faltungsvorgang zuvor erfindungsgemäß vollständig charakterisiert ist, in den parallelen Ansätzen, die verschiedene biologische oder/und chemische potentielle Hemmstoffe oder/und Hilfsproteine enthalten, für die Suche nach den auf die Proteinfaltung einwirkenden biologischen oder chemischen Stoffen zu einem simulierten *in vitro* Faltungsprozess gebracht werden kann. Der Ansatz ohne die potentiellen Hemmstoffe und Hilfsproteine dient hier als Kontrast. Hierbei wird das rückfaltende Protein in den Ansätzen in zeitlichen Abständen portionsweise herausgenommen und der selektierten Modifizierung mit dem entsprechenden Seitenkettenreagenz unterzogen, anschließend durch Auftrennungen, weitere proteolytische Spaltungen und massenspektrometrische Messungen für die Charakterisierung und den Vergleich der Faltungsvorgänge bereitgestellt.

- In einer vierten Ausführungsform ist zu verstehen, dass ein vollständig denaturiertes disulfidfreies Protein oder ein vollständig reduziertes, denaturiertes und vom Reduktionsmittel befreites disulfidhaltiges Protein, dessen Faltungsvorgang zuvor vollständig charakterisiert ist, in den parallelen Ansätzen, die bekannte Foldasen oder/und Chaperone enthalten, für die Untersuchung der Wirkung der Foldasen und Chaperone auf der Proteinfaltung zu einem simulierten *in vitro* Faltungsprozess gebracht werden kann. Der Ansatz ohne Foldasen und Chaperone dient hier als Kontrast. Hierbei wird das in den Ansätzen rückfaltende Protein in zeitlichen Abständen portionsweise herausgenommen und der selektierten Modifizierung mit entsprechenden Seitenkettenreagenzien unterzogen, anschließend durch Auftrennungen, weitere proteolytische Spaltungen und massenspektrometrische Messungen für die Charakterisierung und den Vergleich der Faltungsvorgänge bereitgestellt.

- In einer fünften Ausführungsform ist zu verstehen, dass ein vollständig denaturiertes disulfidfreies Protein oder ein vollständig reduziertes, denaturiertes und vom Reduktionsmittel befreites disulfidhaltiges Protein, dessen Faltungsvorgang und darauf zurückzuführende Wirkungen bestimmter Foldasen oder/und Chaperone zuvor erfindungsgemäß charakterisiert sind, für die Suche nach einem effektiven Foldasen- und Chaperoninhibitor in den Foldasen oder/und Chaperone und ihrem Kandidat-Inhibitor enthaltenden Ansätze zum simulierten Faltungsprozess gebracht wird. Die verschiedenen Kandidat-Substanzen können gleichzeitig parallel untersucht werden, wobei ein Ansatz als Kontrast keine Kandidat-Substanzen enthält.

Hierbei wird das rückfaltende Protein in den Ansätzen in zeitlichen Abständen portionsweise herausgenommen und der selektierten Modifizierung mit entsprechenden Seitenkettenreagenzien unterzogen. Durch anschließende Charakterisierung und Vergleich aller Faltungsvorgänge sind die Wirkungen der Kandidat-Substanzen auf die Hemmung der Foldasen oder/und Chaperone festzustellen. Die bei den Untersuchungen gebrauchten Foldasen und Chaperone können erfindungsgemäß entweder direkt in den Ansätzen zugegeben oder durch Anwendung der Zell-Extraktion für die zellfreie Proteinsynthese vor dem Start des simulierten Faltungsvorgangs in die Ansätze gebracht werden. Die hierbei eingeführten dynamischen Modifizierungen bei simulierter *in vitro* Proteinfaltung können erfindungsgemäß zur Suche nach biologischen und chemischen Hemmstoffen und Hilfsproteinen der Proteinfaltung und des Proteinabbaus für die Entwicklung neuartiger Pharmatherapeutika dienen.

- In einer sechsten Ausführungsform ist zu verstehen, dass die vollständig denaturierten Polypeptidketten oder Proteine, deren Faltungsvorgang zuvor vollständig charakterisiert ist, in den parallelen Ansätzen mit biologischen oder/und chemischen Einflussfaktoren der Faltung und gegebenenfalls Reagenzien für die Bildung der Peptidbindung unter regulierten Bedingungen für die Untersuchung kontrollierter Selbstassemblierung und Polymerisation der Polypeptide oder Proteine bei ihrer Rückfältung zur Entwicklung und Herstellung der Nano-Proteinmaterialien zu einem simulierten *in vitro* Faltungsprozess gebracht werden. Der Ansatz ohne biologische oder/und chemische Einflussfaktoren und Reagenzien für die Bildung der Peptidbindung dient hier als Kontrast. Hierbei werden die rückfaltende, zugleich selbstassemblierende oder/und polymerisierende Proteine in den Ansätzen in zeitlichen Abständen portionsweise herausgenommen und der selektierten Modifizierung mit entsprechen Seitenkettenreagenzien unterzogen und weitergehend für die Charakterisierung ihres Faltungsvorgangs bereitgestellt.

- In einer siebten Ausführungsform wird gezeigt, dass die aufgrund falscher Faltung Krankheiten hervorrufende Proteine, deren Faltungsvorgang zuvor erfindungsgemäß charakterisiert ist, in den parallelen Ansätze mit den biologischen oder/und chemischen KandidateneFaltungsstabliisierungssubstanzen, die spezifisch an ungefaltete Proteine binden und somit die Proteinstruktur stabilisieren und die Faltung verbessern oder die hydrophobe Domänen fehlgefalteter Proteine maskieren und damit ihre Löslichkeit erhöhen und die Aggregation von ungefalteten Proteinen verhindern, für die Suche nach Pharmakologischen Chaperone gegen Proteopathy zu einem simulierten *in vitro* Faltungsprozess gebracht werden können. Der Ansatz ohne biologische oder/und chemische Kandidaten-Faltungsstabilisierungssubstanzen dient hier als Kontrast. Hierbei werden die bei Rückfaltung den Faltungsstabilisierungsfaktoren unterzogene Proteine in den Ansätzen in zeitlichen Abständen portionsweise herausgenommen und einer selektierten Modifizierung mit entsprechenden Seitenkettenreagenzien unterzogen, anschließende für die erfindungsgemäße Charakterisierung und Auswertung dieses Vorgangs bereitgestellt.

- In einer achten Ausführungsform wird gezeigt, dass ein bestimmtes Prion-Protein, dessen Faltungsvorgang zuvor erfindungsgemäß charakterisiert ist, in den parallelen Ansätzen mit den biologischen oder/und chemischen Faltungseinflusssubstanzen unter Regulierung der destabilisierenden Bedingungen wie z. B. Temperatur, pH-Wert und Ionenstärke für die Charakterisierung des Vorgangs zum einen der Umfaltung des $PrP^c$ ( Prion Protein cellular = zelluläres Prion-Protein) zu $PrP^{Sc}$ (Prion Protein Scrapie; pathogene Form des Prion-Proteins) einschließlich gefolgter Aggregation und zum anderen der Umkehrung des $PrP^{Sc}$ zu $PrP^C$ einschließlich des Abbaus der Aggregation zur Klärung der Pathogenese und der Suche nach Therapieoptionen und Möglichkeiten zur Prävention zu einem simulierten *in vitro* Faltungsprozess gebracht wird. Der Ansatz ohne biologische oder/und chemische Faltungseinflusssubstanzen dient hier als Kontrast. Hierbei wird das in den Ansätzen bei Rückfaltung den Faltungseinflusssubstanzen unterzogene Prion-Protein in zeitlichen Abständen portionsweise herausgenommen und einer selektierten Modifizierung mit entsprechen Seitenkettenreagenzien unterzogen und für die weitergehenden Durchführungen bereitgestellt.

- In einer neunten Ausführungsform wird gezeigt, dass ein gezielt vollständig denaturiertes, reduziertes und vom Reduktionsmittel befreites Protein, dessen Faltungsvorgang zuvor erfindungsgemäß charakterisiert ist, bei seiner Rückfaltung entweder einer katalytisch beschleunigten Isomerisierung, Deamidierung und Racemisierung durch Zufuhr der photochemischen und thermischen Energie oder einer von Radikaleinwirkung, oxidativem Stress und Umwelteinflüssen initiierten Modifikation unterzogen wird, wobei das rückfaltende Protein in den Ansätzen in zeitlichen Abständen portionsweise herausgenommen wird und anschließend der Modifizierung mit denselben Seitenkettenreagenzien unterzogen wird. Der Ansatz ohne Isomerisierung, Deamidierung und Racemisierung oder Radikaleinwirkung, oxidativem Stress und Umwelteinflüssen dient hier als Kontrast. Durch weitergehende Charakterisierung und Vergleich der Faltungsvorgänge sind die Vorgänge der aufgrund veränderter Ladungsverhältnisse und Konformationen zurückgeführten Proteinalterung zu untersuchen. Dies dient der Erforschung des Alterungsprozesses, der Entdeckung und Entwicklung der biologischen und chemischen Antioxidantien.

Unter der dynamischen Modifizierung bei der *in vitro* Proteinbiosynthese ist erfindungsgemäß zu verstehen, dass ein in einem zellfreien Ansatz biosynthetisierendes Proteins, dessen Faltungsvorgang erfindungsgemäß zuvor charakterisiert ist, während seiner schnellen Biosynthesephase in zeitlichen Abständen vom Ansatz portionsweise unter Kühlbewahrung herausgenommen, dann zusammengemischt, denaturiert, gegebenenfalls reduziert und vom Reduktionsmittel befreit und je nach definierten Längen der Aminosäureketten gezielt in unterschiedliche Polypeptidefragmente gelelektrophoretisch oder chromatographisch aufgetrennt, wobei jedes dieser Fragmente mit bestimmter Länge der Aminosäuresequenz womöglich über gleiche Molekularmassen, aber ungleiche strukturelle Gestaltung verfügt und als Mini-Intermediate bezeichnet wird. Diese aufgetrennten Polypeptid-Fragmente werden dann gegebenenfalls einer Modifikation mit entsprechenden Seitenkettenreagenzien für die Charakterisierung der Faltungsvorgänge der Biosynthese und cotranslationalen Modifikationen des Proteins unterzogen, indem die Länge der biosynthetisierten Mini-Intermediate durch den regulierenden Einsatz der benötigten Aminosäuren mit oder ohne Isotopenmarkierung erzielt wird und die Entstehungszeitpunkte solcher hergestellten Mini-Intermediate nach ihren Lange-Verhältnissen zur ganzen Aminosäuresequenz neu definiert werden.

Die erfindungsgemäß zur Modifizierung auszuwählenden Vorgehensweisen sind im Folgenden definiert:

- Die Singlemodifizierung: sie ist definiert als selektive Markierung der Reste einer einzigen Art der Aminosäure mit einem einzigen Reagenz bei optimierten Reaktionsbedingungen,

- Die Multimodifizierung: sie ist definiert als selektive Modifizierung der Reste bei mehr als einer Art der Aminosäure mit einer einzigen oder mehr als einer Art von Reagenz durch Veränderung der Reaktionsbedingungen. Sie kann auch in einem einzelnen oder in mehreren Ansätzen zuerst separat durchgeführt und dann zusammen gemischt werden.

- Die interne Crosslinker-Modifizierung: sie ist definiert als selektive interne Doppel-Modifizierung mit den bifunktionellen Reagenzien bei regulierten Reaktionsbedingungen in variierten Ausführungsformen.

Die bekannten zur Modifizierung anzuwendenden Methoden sind vielfältig und stehen je nach der gezielten Zeitskala der Proteinfaltung für die entsprechenden Modifikationen zur Verfügung. Die Auswahl der Methode entscheidet sich erfindungsgemäß von der Modifikationsschnelligkeit, die vorwiegend von der Reaktionsgeschwindigkeit der Seitenkettenreagenzien und den Eigenschaften der zu modifizierten Proteine abhängig ist. Die Methoden lassen sich daher je nach der angeführten Reaktionsschnelligkeit der Modifikation in drei Gruppen einteilen:

- Die Modifikationsgeschwindigkeit mit einer Zeitskala zwischen Nano-, Mikro- bis Millisekunden
  Dies eignet sich für die Untersuchungen der Proteinfaltung mit einer Zeitskala von Milli- bis Sekunden. Zu diesen schnellsten Phasen der Faltung gehören die Bildung von WasserstoffBrücken, die Ausbildung der sekundären Strukturelemente und des hydrophoben Kollapses der Polypeptidkette. Diese schnellsten Modifikationen können zum Beispiel durch Einsatz der spezifischen Seitenkettenreagenzien oder Zufuhr der photochemischen und thermischen Energie für die Isomerisierung von Prolin und Asparaginsäure und die Deamidierung von Asparagin erreicht werden.
- Die Modifikationsgeschwindigkeit mit einer Zeitskala zwischen Mikrosekunden bis einer Minute.
  Die durch Einsatz von Mikrowellen-Technik erreichbare Modifikationsgeschwindigkeit eignet sich für die Untersuchungen der Proteinfaltung mit einer Zeitskala von Millisekunden bis Minuten. Dabei geht es um die schnelle Faltungsphase und die Frühphase der Entstehung der wegezugehörigen Intermediate.
- Die Modifikationsgeschwindigkeit mit einer Zeitskala zwischen Millisekunden bis Minuten.
  Diese eignet sich für die Untersuchungen der Proteinfaltung mit einer Zeitskala von Sekunden bis zu Stunden oder Tagen, wobei es sich meist um die langsame Phase der Faltung und die Entwicklung der wegezugehörigen Intermediate, Entstehung der relativen stabilen kompakten Strukturen des Molten Globules und die weitere Faltung aller Intermediate bis in den nativen Zustand mit minimalem Energieniveau handelt.

Die Modifikationen in den Zeitskalen für die schnelle Phase der Faltung können in den entsprechenden Reaktionsansätzen je nach Bedarf erfindungsgemäß mit Quenched-Flow, Stopped-Flow und Continuous-Flow Methods sowie Turbulent Mixing Technik in Ankopplung mit der Mikrowellen-Miniapparatur und den spektroskopischen Detektion erfolgen. Die Modifikationen für die langsame Phase der Faltung können mit den konventionellen Methoden erfolgen. Das grundlegende Kriterium für eine erfolgreiche Modifikation des rückfaltenden Proteins liegt darin, ob alle damit modifizierten und abgefangenen Zwischenprodukte zu den strukturell relativ stabilen und aufzutrennenden Intermediaten mit individuellen Charakteristiken gebracht werden können.

Die erfindungsgemäß für die Modifizierung auszuwählenden Reagenzien umfassen z. B. die Folgenden:

- die bekannten und nicht bekannten, aber wirkenden Denaturierungsmittel der Proteine

- die bekannten und nicht bekannten, aber wirkenden Reduzierungsmittel für die disulfidhaltigen Proteine,

- die bekannten und nicht bekannten, aber wirkenden Reoxidationsmittel inklusive ihrer variablen Komponenten und Zusammensetzungen, spezifisch für die Modifizierung der disulfidhaltigen Proteine,

- die bekannten und nicht bekannten, aber gleich wirkenden seitenkettespezifischen Reagenzien, ohne und mit Isotopenmarkierung,

- die bekannten und nicht bekannten, aber gleich wirkenden Reagenzien mit den fluoreszenz- und spinmarkierten (spin label) Reportgruppen vorzugsweise der kleinen molekularen Reagenzien ohne Wirkung der Ladungsveränderung auf das Protein, insbesondere die bekannten Reagenzien für DIGE (Difference gel electrophoresis),

- die bekannten und nicht bekannten, aber gleich wirkenden nulllänge-, homo- und heterobifunktionellen Reagenzien zur internen Crosslinker-Markierung, einschließlich der Reagenzien für die Photomarkierung,

- die bekannten und nicht bekannten, aber gleich wirkenden Biotinylierungsreagenzien.

Die erfindungsgemäß benötigten und auszuwählenden Hilfssubstanzen zur Stabilisierung, Verbesserung und Erhöhung der nativen Faltungseffizienz bei der Modifikation und/oder Oxidation, insbesondere der großen Proteine umfassen folgende:

• die bekannte und nicht bekannte, aber gleich wirkende als Foldasen und Chaperone bezeichneten Faltungsfaktoren,

• die bekannten und nicht bekannten, aber gleich wirkenden Hilfssubstanzen zur optimalen Renaturierung der biotechnologisch erzeugten pharmatherapeutischen Proteine für die Reduzierung der Missfaltung, die zur Verminderung der Aggregation und/oder zur Erhöhung der thermischen Stabilität angewendeten biologischen und chemischen Substanzen. Sie finden die entsprechenden Anwendungen in allen erfindungsgemäßen Ausführungsformen der Proteinmodifizierung.

Zur Ausführung der vorstehenden Modifikationen in unterschiedlichen Vorgehensweisen und Ausführungsformen werden die Mittel erfindungsgemäß in Form von Serien-Assay-Kits, speziellen Laborgeräten und gegebenenfalls spezifischer Software gebraucht. Zur Begünstigung der Abtrennung der modifizierten Proteine von den Modifikationsansätzen können die Proteine je nach Notwendigkeit zuerst auf einen Träger chemisch immobilisiert, dann nach Modifikation durch Abspülen von übrigen Bestandteilen des Ansatzes abgetrennt, anschließend durch chemische oder photochemische Abspaltung vom Träger befreit und für die weitergehende Auftrennung der Intermediate bereitgestellt werden. Dies gilt für alle zu modifizierenden Proteine.

4. Auftrennung und Quantifizierung der Intermediate und zweidimensionale Darstellung ihrer hydrodynamischen Größen und Stoffmenge als Funktion ihrer Entstehungszeitpunkte

All diese vorstehend beschriebenen Modifikationen der Intermediate können erfindungsgemäß in zwei Durchführungsformen nach ihrer in zeitlichen Abständen erreichten hydrodynamischen Größen und Menge manuell oder erfindungsgemäß automatisch aufgetrennt, quantifiziert und mit ihren hydrodynamischen Größen oder gegebenenfalls thermodynamischen Größen und Mengen als Funktion ihrer Entstehungszeitpunkte eingeordnet und dem 4 phasigen Multifaltungswege-Modell entsprechend zweidimensional dargestellt werden. Ein auf die Aufbauphase der Faltungswege bezogenes Rückfaltungsfingerabdruckprofil eines Proteins kann dabei hergestellt werden.
Der ersten Durchführungsform liegen die Elektrophorese-Methoden zugrunde. Diese Durchführungsform bezieht sich neben der Kapillarelektrophorese bevorzugt auf die vom Erfinder modifizierte native Polyacrylamidgelelektrophorese, die vorzugsweise auf die globalen und hydrophilen Proteine ausgerichtet ist und je nach Art, Größe und Menge der zu behandelten Proteine in Form von variablen Konstruktionen gestaltet wird. Die hydrodynamischen Größen der Intermediate und ihre Menge werden hierbei z. B. nach der Elektrophorese direkt auf dem Gel in Form von Banden mit unterschiedlichen Färbungsstärken gegen ihre Entstehungszeit zweidimensional vorgelegt und für das Scanning zur Digitalisierung und für weitergehende Untersuchungen bereitgestellt. Die gleichen Gelbande, die aus den untersciedlichen Zeitpunkten entstehen und über gleiche hydrodynamischen Größe oder gleicher Bandenummer verfügen, vertreten hierbei die gleichen Intermediate.
Im Fall, dass mehr als ein Intermediat in einer einzigen Bande vorliegt, werden die Intermediate aus der Bande des Gels elektroeluiert und anschließend mit der von Erfinder konstruierten Mikrosäule chromatographisch nach ihren Differenzen der Verteilung der Ladungen, Hydrophobi- und Hydrophilizität auf der Moleküloberfläche aufgetrennt und anschließend spektrometrisch mit DLS (Dynamische Lichtstreuung), SLS (Statische Lichtstreuung), CD, Fluoreszenz und usw. differenziert.
Diese erfindungsgemäß modifizierte native Polyacrylamidgelelektrophorese richtet sich darauf, die Trennbreite zwischen den einzelnen Intermediaten zu vergrößern, die maximale Trennschärfe zu erzielen und die Dauer der Elektrophorese zu verkürzen, um im Gegensatz zur üblichen Proteinauftrennung die feinen Unterschiede der hydrodynamischen Größen der aus einzelnen Proteinen stammenden Intermediate zu differenzieren. Sie umfasst mindestens fünf technisch zusammenhängende Verbesserungen.

- Erstens, der Anschluss eines Zuführsystems der Pufferlösung für die während der Elektrophorese erfüllbaren Regulierung der Pufferstärke, -Zusammensetzung und des -pH-Werts entweder durch Einbetten eines durchlässigen Behälters, der mit der gewünschten Pufferlösung gefüllt ist und sich im Pufferreservoir der Kathode befindet oder durch einen Puffer-Verteiler, der sich im Pufferreservoir der Kathode befindet und durch einen dünnen Schlauch mit dem äußeren Gefäß verbunden ist. Dies ermöglicht die gewünschte Pufferlösung je nach gewünschtem Tempo kontinuierlich oder diskontinuierlich und dosierend zuzuführen.

- Zweitens, die dynamische Steuerung und Optimierung der Trennauflösung durch die während der Elektrophorese regulierend zugeführten Pufferlösung. Die dadurch erfolgten Einstellungen der Pufferstärke, -Zusammensetzung und des -pH-Werts führt zur Erhöhung der Differenz der Ladung und Polarität zwischen den Intermediaten eines Proteins und des Ionenstroms. Das verbesserte Trennvermögen ist hierbei nicht mehr nur von Ladungen und Formen eines Proteins allein, sondern auch von den dadurch neu eingeführten Differenzierungsfaktoren, nämlich der dynamisch veränderten Ladungs- und Polaritätsverteilung der Intermediate und ihrer differenzierten Wechselwirkungen mit dem zusätzlichen Ionenstrom, bestimmt.

- Drittens, die Anwendung der gepulst betriebenen Elektrophorese durch kurze Erhöhung der Spannung, kurze Veränderung der Polarität der Pufferbestandteile und/oder ihrer Konzentration und kurzes Umpolen kombiniert mit den stark hydrophoben Gegenionen zur Fokussierung der einzelnen Bande und Vergrößerung der Entfernung der Intermediate-Banden.

- Viertens, der Einsatz varianter Gelzusammensetzungen und -formen, z. B. durch das Porengradientengel zur Auftrennung der Intermediate von insbesondere großen Proteinen

- Fünftens, zum Unterdrücken der von Wärmeeffekten verursachten Diffusion der Intermediate zwischen den Banden soll die Gelelektrophorese bei einer Temperatur nicht über 10 °C durchgeführt wird, wobei die Elektrophoreseapparatur entweder an einem genügend effektiven Kühlthermostat direkt angeschlossen oder erfindungsgemäß mit einem aus den Peltier-Kühlplatten und einer zirkulierenden Flüssigkühlung bestehenden Vorrichtung zum gleichzeitigen Wärmeabtransport aus dem Gel und Wärmeableitung ins eishaltige Abkühlreservoir zusammengebaut werden kann.

Die zweite Durchführungsform ist die vom Erfinder konstruierte Mono- und Multisäulenflüssigchromatographie einschließlich der miniaturisierten Feldflussfraktionierung (FFF) und erforderlichenfalls der Mikrosäulenelektrochromatographie in Kopplung mit der spektrometrischen Differenzierung der hydrodynamischen Größen vorzugsweise mit DLS und SLS. Sie eignen sich für die Auftrennung und Größen-Differenzierung der Intermediate aller Proteine, richten sich aber vorwiegend auf die für die Elektrophorese-Methode nicht geeigneten stark sauren, stark basischen, hydrophoben und membranen Proteine, einschließlich der sehr großen Proteine, deren Intermediate mit Gelelektrophorese nicht effektiv aufgetrennt werden können. Die durch spektrometrische Differenzierung in Reihenfolge gebrachten hydrodynamischen Größen oder gegebenenfalls thermodynamischen Größen aller jeweils in den einzelnen Mikrogefäßen aufgetrennten intermediate werden als Funktionen ihrer Entstehungszeitpunkte für die weitergehenden Arbeitsschritte zweidimensional meist in einer Mikrotiterplatte eingeordnet und in der Datenbank erfasst.

Unter Monosäulenflüssigchromatographie versteht man hierbei den bevorzugten Einsatz der jeweils nach Wunsch oder Bedarf mit unterschiedlichen Trennträgern eingefüllten einzelnen Mikrogelfiltrationssäule oder des Mikrofeldflussfraktionierungskanals in Kopplung mit spektrometrischer Überprüfung zur direkten Ermittlung der Reihenfolge der hydrodynamischen Größen der dabei jeweils in den einzelnen Eluaten aufgetrennten Intermediate.

Die Multisäulenflüssigchromatographie ist hierbei definiert als Mikrosäulenkombinationen, die je nach Wunsch oder Bedarf in Serie und/oder parallel aus Gelfiltrations-, Hydroxyapatit-, Hydrophobe-, Ionenaustausch-, Reversedphase- und Affinitätschromatographie einschließlich des Mikrofeldflussfraktionierungskanals für die spezifische Trennung der Intermediate, die vor allem mit sehr ähnlichen hydrodynamischen Größen, entweder zu den gleichen oder zu unterschiedlichen Faltungswegen gehören. Die hydrodynamischen Größen der durch Multisäulenchromatographie in seriellen Mikrogefäßen oder meist in Mikrotiterplatten separierten Intermediate werden zuerst spektrometrisch differenziert, dann in die Reihenfolge ihrer Größe gebracht und in der Datenbank erfasst und anschließend als Funktion ihrer Entstehungszeitpunkte zweidimensional eingeordnet.

Die damit gekoppelte spektrometrische Differenzierung der hydrodynamischen Größen der jeweils in den einzelnen Mikrogefäßen aufgetrennten Intermediate und ihre Quantifizierung werden bevorzugt mit DLS (Dynamische Lichtstreuung) und SLS (Statische Lichtstreuung) durchgeführt und dies werden weiter mit Fluoreszenz, UV (Ultraviolettstrahlung)/VIS (sichtbare Spektrum), CD (Circulardichroismus), NMR (Kernspinreso-nanz), Fourier-Transforminfrarot und ESR (Elektronenspinresonanz) usw., die in vielfältiger Form in kommerziellen Produkten angeboten werden, ergänzt.

Die spezifische Differenzierung der in einer einzelnen Probe enthaltenden Intermediate, die sich entweder in unterschiedlichen hydrodynamischen Größen verteilen oder mit sehr ähnelnde hydrodynamischen Größen aber womöglich zu unterschiedlichen Faltungswegen gehören, können mit den erfindungsgemäß umgebauten DLS- und SLS-Geräten erfolgen, deren Differenzierungsvermögen durch mindestens eine der folgenden zusätzlichen Funktionen verbessert werden:

- Erhöhung der Differenzierungsauflösung durch verlängerte Messungszeit,

- Tm (Schmelzpunkt)-Differenzierung durch die allmähliche vom Programm gesteuerte Temperaturerhöhung,

- Konzentrationsänderung der Probe in einzelnen Mikrogefäßen oder in Mikrotiterplatten durch Verdünnung oder durch die eingebaute Vakuumverdunstung oder Belüftung erfüllte Aufkonzentrierung,

- Änderung des pH-Profils und Puffersystems durch Mikroautotitration oder manuelle Pipettierung gewünschter Pufferzusammensetzung,

- Bestimmung und Bewertung intrinsischer Viskosität und des Zeta-Potentials der Proben zur weitergehend strukturellen Differenzierung der Intermediate,

- Veränderung der biorheologischen Eigenschaften der Proben durch Energiezufuhr oder - abfuhr in Form von Bestrahlung, Erwärmung und Abkühlung, Ultraschall, Mikrowelle, elektrisches Feld und Magnetfeld,

- Bewertung und Anordnung der damit differenzierten Intermediate durch speziell dafür entwickelte Software.

Mit verbesserten Funktionen der DLS für die Verteilungsdarstellung der hydrodynamischen Größen der Intermediate kann ein Fingerabdruckprofil der Faltung eines Proteins, der den Vorgang des Aufbaus der Faltungskanäle in erfindungsgemäß definierter Phase der Faltung präsentiert, in Kopplung eines Instruments von Quench-Flow oder Stopped-Flow mit Tandem-Mixer unter definierten Bedingungen ermittelt und mithilfe der Graphiksoftware visualisiert werden. Dabei wird das optimal vollständig entfaltete Protein durch den ersten Mixer zum Start der Rückfaltung gebracht, anschließend in zeitlichen Abständen mit Hilfe des zweiten Mixers zur dynamischen Modifizierung gebracht und währenddessen den DLS-Messungen, um die in diesen Zeitabständen entstehenden Verteilungen der hydrodynamischen Größen der Intermediate zu ermitteln, unterzogen.

Diese Flüssigchromatographie in Kopplung mit der spektrometrischen Differenzierung der hydrodynamischen Größen wird je nach den physikochemischen Eigenschaften des betreffenden Proteins und der Zielsetzung der Untersuchung jeweils in den vom Erfinder definierten Mikro-, Analytik- und Semipräparativ-Skalen durchgeführt.

Die Durchführung in der Mikro-Skala braucht sehr wenig Protein und dient der schnellen Behandlung des weniger als $100\mu$g Protein enthaltenden Mikroansatzes der Proteinmodifizierung und erfolgt parallel mit den vom Erfinder konstruierten Mikrogelfiltrationssäulen im einzelnen, oder im seriellen, oder parallelen Anschluss. Die Eluate werden meist in den Mikrotiterplatten aufgefangen und nach der spektrometrischen Bestätigung ihrer hydrodynamischen Größen und Konzentrationsdifferenzen für die proteolytische Spaltung bereitgestellt.

Die Durchführung in der Analytik-Skala braucht bis zu 1mg Protein und richtet sich speziell auf die Auftrennung der Intermediate, die aufgrund ähnlicher hydrodynamischer Größen mit der Mikro-Skala-Durchführung noch nicht vollständig abgetrennt werden können oder deren Faltungswegezugehörigkeiten weitergehend differenziert werden sollen. Die Auftrennung der Intermediate in dieser Skala erfolgt mit der Multisäulenflüssigchromatographie und stellt ausreichend Proteinmaterial für die wiederholenden DLS-Bestimmungen, die spektrometrisch strukturellen Analysen, die anschließende proteolytische Fragmentierung und andere notwendige Untersuchungen zur Verfügung.

Die Durchführung in der Semipräparativ-Skala braucht mehr als 1 mg Protein und beruht auf der gleichen Konzeption der Auftrennung, die zuvor in der Mikro- oder Analytik-Skala erfolgreich nachgewiesen wurde, und dient vorwiegend zur Probenvorbereitung für die NMR- oder die Kristallstrukturbestimmungen der getrennten Intermediate, um die Faltungswegezugehörigkeit der Intermediate durch die individuellen Merkmale der strukturellen Veränderung genauer zu differenzieren. Diese Durchführung kann mit Hilfe von kommerziellen Produkten erfolgen, die dem Stand der Technik der Chromatographie entsprechen und entsprechend zusammengestellt werden. Die beiden Durchführungsformen können erfindungsgemäß durch selektive Zusammenstellung der Apparaturen, die dem Stand der Technik entsprechen, wie Elektrophorese, Flüssigchromatographie, Elektrochromatographie, Feldflussfraktionierung und Spektrometrie vorzugsweise mit DLS und SLS standardisiert, teilweise oder vollständig automatisiert, miniaturisiert und mit anderen Schritten des Erfindungsverfahrens online oder offline angeschlossen werden.

5. Fragmentierung der Intermediate durch In-Gel-Verdau oder In-Lösung-Verdau

Die Fragmentierung ist der erste Schritt für die weitere genauere Differenzierung der Faltungswegezugehörigkeit der Intermediate. Die Fragmentierung aller elektrophoretisch und chromatographisch aufgetrennten Intermediate, deren hydrodynamische Größe und Entstehungszeitpunkte bereits festgelegt wurden, erfolgt vorzugsweise durch enzymatische Spaltung mit Trypsin. Neben Trypsin finden unter anderem die Endoproteasen Lys-C, Glu-C und Asp-N Verwendung, die meist im Fall der möglicherweise durch Modifizierung verursachten Proteaseresistenz gegenüber Trypsin und zum Erhalt der zusätzlichen und begünstigten Spaltungsstellen eingesetzt werden. Die Exoprotease und chemische Spaltung als Alternative zu MALDI-TOF-MS/MS und MALDI-TOF-PSD (Post Source Decay) dienen dabei den Hydrolysen der Aminosäuren vom N- oder/und C-Terminus der enzymatisch gespaltenen Fragmente, um die unterschiedlichen Fragmente mit zufällig gleichem Molekulargewicht zu differenzieren. Alle proteolytischen Fragmentierungen können manuell oder mit einem kommerziellen Verdau-Roboter, oder mit der vom Erfinder spezifisch dafür konstruierten Mikrowellen-Verdau-Apparatur mit einem mittleren Probendurchsatz durchgeführt werden. Die fragmentierten Proben, die sich in Mikrotiterplatten befinden, können zur online- oder offline-Kopplung mit dem Massenspektrometer gebracht werden.

Die gelelektrophoretisch aufgetrennten Intermediate werden nach der Coomassie-Brillant-Blau- oder Silberfärbung in den Zeitabständen gefolgten Banden-Reihen mit unterschiedlichen Färbungsintensitäten zweidimensional auf dem Gel dargestellt, photographiert und zur Digitalisierung gescannt. Die quantitative Auswertung der Färbungsintensitäten und die Quantifizierung der Intermediate erfolgen mit dem Densiometer. Die kinetischen Zusammenhänge zwischen den Intermediaten können hierbei mit entsprechender Software qualitativ interpretiert werden. Die mit einzelnen oder unterschiedlichen Fluoreszenzfarbstoffen markierten und gelelektrophoretisch aufgetrennten Intermediate werden mit dem Fluoreszenzscanner detektiert und digitalisiert. Enthält eine Bande mehr als ein Intermediat, können die gemischten Intermediate zuerst chromatographisch mit der Mikrosäule aufgetrennt, dann separat fragmentiert werden. Alle Intermediate in den Banden werden erfindungsgemäß entweder mit dem spezifischen Werkzeug manuell oder mit einem Gelbandenpicker automatisch herausgestochen und mit der Standardmethode oder mit der erfindungsgemäßen Verdau-Apparatur in multifunktionelle Mikrotiterplatten für In-Gel-Verdau in Fragmente enzymatisch gespalten. Die zusätzlichen exoproteolytischen oder chemischen Spaltungen erfolgen in den übrigen Lösungen aus In-Gel-Verdau nach den ersten massenspektrometrischen Untersuchungen.

Die Fragmentierung der chromatographisch aufgetrennten Intermediate kann entweder mit der Standardmethode für In-Lösung-Verdau in Mikrotiterplatten erfolgen, oder analog des letzten Schrittes der Handhabung für die gelelektrophoretisch aufgetrennten Intermediate mit dem vom Erfinder spezifisch für den In-Gel-Verdau konstruierten Verdau-Apparat durchgeführt werden.

6. Massenspektrometrische Detektion der fragmentierten Intermediate

Die massenspektrometrische Detektion ist der zweite Schritt zur genaueren Differenzierung der Faltungswegezugehörigkeit der Intermediate. Die einzusetzenden Methoden umfassen ESI-TOF-MS (Elektrospray-Flugzeit-Massenspektrometrie), MALD-TOF-MS (Matrix-unterstützte Laser-desorptions/-Ionisations-Flugzeit-Massenspektrometrie), MALDI-TOF-MS/MS (Tandem-Massen-spektrometrie) und MALDI-TOF-MS-PSD (Post Source Decay-Massenspektrometrie) usw.

Die massenspektrometrische Detektion der fragmentierten Intermediate erfolgt durch die Messungen der Molekulargewichte aller einzelnen Fragmente und der durch Disulfidbrücken und/oder Crosslinker gebundenen großen Fragmente. Alle gesammelten Daten werden in einer Datenbank erfasst. In dieser Datenbank wird eine theoretische Fragmente-Analytik, die alle möglichen Intermediate und alle sich daraus ergebenen Fragmente einschließlich der Vernetzungen zwischen den Fragmenten einbezieht, mit kommerzieller oder erfindungsgemäß speziell dafür entwickelter Software ausgeführt und gespeichert.

7. Feststellung der Faltungswegezugehörigkeit der Intermediate

Der entscheidende Schritt für die Feststellung der Faltungswegezugehörigkeit des Intermediates ist der Vergleich der massenspektrometrisch erfassten Informationen über die Anzahl und Masse der vereinzelten kleinen Fragmente und der durch Disulfidbrücken und/oder Crosslinker gebundenen großen Fragmente mit dem in der Datenbank gespeicherten theoretischen Fragmenten-Massenerkennungsmuster. Hierbei werden die vorstehend beschriebenen Kriterien, die auf der Proteinevolutionstheorie und Proteinfaltungskinetik beruhen, zur Vervollständigung der Festlegung der Faltungswegezugehörigkeit der Intermediate gebraucht.

Die den Modifikationen zugrunde liegenden Merkmale jedes fragmentierten Intermediates werden durch den Vergleich erkannt und mit einer bestimmten Bezeichnung markiert. Die aufgetrennten Intermediate, die jeweils aus unterschiedlichen Zeitpunkten stammen, werden ebenso verglichen und je nach den individuellen Merkmalen mit den Bezeichnungen markiert. Dadurch wird eine Tabelle mithilfe der speziellen Software automatisch entstehen, in der die in der ersten Spalte eingetragene hydrodynamische Größe von oben nach unten abnimmt und die in weiteren Spalten angegebene Entstehungszeit von links nach rechts mit zeitlichen Abständen zunimmt. Alle zu untersuchenden Intermediate werden daher nach ihren hydrodynamischen Größen, Entstehungszeitpunkten und gegebenenfalls Mengen in dieser Tabelle definiert. In den weiteren Spalten der Tabelle werden die unterschiedlichen Markierungen oder Bezeichnungen für alle Intermediate angegeben. Die mit gleichen Markierungen bezeichneten Intermediate lassen sich in die Gruppen mit den natürlichen Zahlen einordnen. Die durch eine Zahl bezeichneten Gruppezugehörigkeiten aller Intermediate werden in der vorletzten Spalte eingetragen. Gehört ein Intermediat zu einer solchen Gruppe mit einer natürlichen Zahl, wird seine Faltungswegezugehörigkeit mit dieser Zahl festgestellt und in der letzten Spalte eingetragen.

Die aufgetrennten Intermediate, deren Faltungswegezugehörigkeit bis dahin noch nicht klar festzulegen sind, können weitergehend durch Einsatz der im vierten Verfahrensschritt beschriebenen Flüssigchromatographie in Analytik- und Semipräparativ-Skala und in Kopplung mit verbesserten DLS- und SLS-Geräten, die sich notfalls mit strukturellen und thermodynamischen Untersuchungen von CD, UV, NMR und Fluoreszenz usw. ergänzen, hinsichtlich ihrer Faltungswegezugehörigkeit differenziert werden.

Hierbei wird aufgewiesen, dass die Wegezugehörigkeit eines Intermediates nicht von sich allein vereinzelt definiert werden kann, sondern von seiner zugeordneten Gruppenzugehörigkeit zu einem bestimmten Faltungsweg identifiziert wird.

8. Identifizierung und Klassifizierung der Intermediate

Die Identifizierung eines Intermediates erfolgt durch Feststellung seiner zuvor festgestellten hydrodynamischen Größe, seines Entstehungszeitpunkts, seiner quantifizierten Menge und der hierbei ermittelten Wegezugehörigkeit der Faltung.

Alle identifizierten Intermediate lassen sich je nach Gemeinsamkeit ihrer individuellen Bezeichnung in unterschiedliche Gruppen zuordnen. Die gruppenzugehörigen Intermediate sollen bestimmte Merkmale besitzen. Die Merkmale können je nach Unterschiedlichkeit der Gruppen in verschiedene Formen wie in den folgenden Beschreibungen ersichtlich wird erscheinen.

Sie sollen über ein gleiches Modifikationsmuster verfügen. Ihre hydrodynamische Größe soll mit den zeitlichen Abständen in Richtung der nativen Struktur stufenweise abnehmen. Sie können mindestens ein signifikantes Intermediat besitzen, das eine relativ stabile kompakte Struktur hat, die als Molten Globule bezeichnet werden kann. Sie können auch die Intermediate beinhalten, deren hydrodynamische Größen sehr ähnlich sind und zwischen denen die intramolekularen Umlagerungen stattfinden. Desweiteren können sie auch noch die Intermediate enthalten, die zugleich zu mehr als einer Gruppe gehören, da die Weg-Abzweigung, -Erweiterung, -Kreuzung, - Überquerung und - das Zusammentreffen durch diese Intermediate eingeführt werden müssen.

Sie verfügen oft über die gleiche quantifizierte Menge, die über die kinetische Kompetenz dieses Faltungswegs entscheidet. Die sich bei der schnellen Faltung gruppierenden Intermediate involvieren oft die nativen Konformationen, und die bei der langsamen Faltung gruppierenden Intermediate umfassen meist hingegen die nicht-nativen Konformationen. Die zur Schnellfaltung gehörende Gruppe verfügt meist über die wenigsten Intermediate mit kaum intramolekularer Umlagerung. Die zur langsamen Faltung gehörende Gruppe hat meist mehrere Intermediate und wird fast immer dabei von den intramolekularen Umlagerungen begleitet, die zur Veränderung der strukturellen Mikroumgebung und dadurch auch zur Veränderung der Modifikationsmerkmale zwischen den Intermediaten führen. Die intramolekularen Umlagerungen finden oft beim Molten Globule Zustand statt. Die den intramolekularen Umlagerungen unterzogenen Intermediate besitzen ähnliche hydrodynamische Größen und lassen sich durch den auf die Modifikation zurückzuführenden Unterschied der strukturellen Merkmale differenzieren.

Alle in verschiedenen Gruppen zugeordneten Intermediate lassen sich hierbei weiterhin jeweils nach den eigenen Merkmalen der Gruppen in verschiedene Faltungswege klassifizieren. Dadurch wird die Wegezugehörigkeit aller Intermediate differenziert und dabei identifiziert. Die hydrodynamische Größe, der Entstehungszeitpunkt, die quantifizierte Menge, die Faltungswegezugehörigkeit und gegebenenfalls die NMR-Strukturen von allen Intermediaten werden hierbei in Tabellen definiert und für die anschließende Charakterisierung des Faltungsablaufs bereitgestellt.

9. Charakterisierung des Faltungsvorgangs

Die Charakterisierung des Faltungsvorgangs erfolgt erfindungsgemäß durch die parallele Darstellung aller sich um den Faltungsweg gruppierenden Intermediate in einem zweidimensionalen Koordinatensystem für den einfachsten Faltungsvorgang, wobei ihre hydrodynamischen Größen jeweils gegen den Zeitablauf ihrer Entstehungen eingetragen und einer systematischen Auswertung unterzogen werden. Läuft die Faltung in mehr als einem Weg ab, kann die Charakterisierung des Faltungsvorgangs auf dem gleichen Prinzip in einem multidimensionalen Koordinatensystem einschließlich der zusätzlich eingefügten Koordinaten als Faltungskanäle oder Faltungswege dargestellt werden.

In der Darstellung wird gezeigt, dass die Rückfaltung eines Proteins in vier Phasen verläuft, nämlich die superschnelle Faltung zur Herausbildung der Seedstrukturen der Faltungswege, den Aufbau der Faltungswege oder -kanäle, das nachfolgende Durchlaufen der Faltung entlang der aufgebauten Wege oder Kanäle und die weitergehende Restrukturierung durch die intramolekularen Umlagerungen zur Vervollständigung der nativen Struktur des Proteins. Der Faltungsvorgang eines Proteins kann vorwiegend durch die gesamten Charakteristiken aller in den ersten 2 Phasen der Faltung vorkommenden Intermediate charakterisiert werden und der Aufbau der in diesen 2 Phasen erfolgten Faltungswege oder -kanäle auch als Fingerabdruck der Faltung eines Proteins bezeichnet werden

Die verschiedenen Faltungswege als parallele Ereignisse in unterschiedlichen Geschwindigkeiten einschließlich der Weg-Abzweigung, -Erweiterung, -Kreuzung, -Überquerung und des - Zusammentreffens sind in der Darstellung anschaulich zu erkennen. Hierbei lassen sich von allen Intermediaten die Faltungswege, ihr kinetischer Ablauf, ihr prozentualer Beitrag zur Faltung, der Vorgang der Missfaltung, die Entstehung und der Ablauf der intramolekularen Umlagerung, die nativen und nicht-nativen Disulfidbildungen, die Reihenfolge der nativen Disulfidbildungen und die faltungstempobestimmten Intermediate usw. direkt ermitteln. Dadurch kann der Faltungsvorgang eines Proteins erfindungsgemäß vollständig charakterisiert werden.

Die hierbei zugleich ermittelten Daten über die veränderte Menge der Intermediate im Verhältnis zum Zeitablauf ihrer Rückfaltung stellen die Faltungskinetik des Proteins dar und können als zusätzliche Dimensionen für die verfeinerte Charakterisierung und Visualisierung aller Faltungswege eines Proteins eingesetzt werden.

Die Charakterisierung des Faltungsvorgangs kann auch auf der dreidimensional strukturellen Ebene aller Intermediate erfolgen. Hierbei sollen alle Intermediate eines Proteins, dessen Faltungsvorgang erfindungsgemäß charakterisiert wurde, nach dem gleichem Prinzip jeweils in benötigter Menge für die NMR- oder/und Kristallstrukturanalyse modifiziert, abgefangen, flüssigchromatographisch aufgetrennt und den Strukturbestimmungen unterzogen werden. Diese semipräparative Herstellung der Intermediate kann mit dem vom Erfinder speziell dafür entwickelten Verfahren in der Semipraparativ-Skala, die speziell auf die effiziente Auftrennung der abgefangenen Intermediate mit großen Konzentrationsunterschieden ausgerichtet ist, erfolgen.

Die Charakterisierung des Faltungsvorgangs mit dem erfindungsgemäßen Verfahren kann noch auf der funktionellen Ebene eines Proteins in unterschiedlichen Ausführungsformen für verschiedene Anwendungen vielfältig erweitert werden, wobei der Faltungsvorgang dieses Proteins zuerst erfindungsgemäß charakterisiert werden und dann weitergehend unter den Einflüssen der eigenen strukturellen Veränderungen oder der geänderten biologischen und chemischen Umgebungen zur Untersuchung veränderter Funktionalität und Aktivität dieses Proteins charakterisiert wird. Diese Erweiterung umfasst beispielsweise die Charakterisierung des Vorgangs der simuliert dynamischen *in vitro* post- und cotranslationalen Modifikationen, den Vorgang der dynamischen Modifizierungen bei simulierter in vitro Proteinfaltung und den Vorgang der modellierten in vitro Biosynthese des Proteins.

10. Graphische und visuelle Darstellung des Faltungsablaufs

Wenn ein Faltungsvorgang eines Proteins erfindungsgemäß charakterisiert ist, werden alle einbezogenen Intermediate jeweils mit ihren 4 individuellen Charakteristiken, nämlich der hydrodynamischen Größe, des Entstehungszeitpunktes, der Wegezugehörigkeit und des prozentualen Beitrags zur Rückfaltung, in einem Multikoordinatensystem 3- oder 4-dimensional genau definiert und digitalisiert. Zum Beispiel werden die Energiezustände der Intermediate entsprechend ihrer hydrodynamischen Größen und der Gelbanden als y-Ordinate, die Entstehungszeitpunkte als x-Ordinate, die Faltungswege oder -kanäle entsprechend ihrer Faltungswegzugehörigkeit als z-Ordinate und gegebenenfalls der prozentuale Beitrag zur Rückfaltung als integrierte 4te Dimension definiert. Dies ermöglicht es, dass der Faltungsvorgang mithilfe der entsprechenden Computergrafik abwechslungsreich visualisiert und/oder animiert wird.

Alle Faltungsvorgänge, die durch die Verwendung des erfindungsgemäßen Verfahrens in unterschiedlichen Ausführungsformen charakterisiert sind, können nach dem gleichen Prinzip multidimensional visualisiert und/oder nach Wunsch animiert werden.

Aus der erfindungsgemäßen Transformierbarkeit aller von den 4 Charakteristiken definierten Ordinaten und der Kombinierbarkeit dieser Ordinaten ergibt sich, dass die zahlreichen dadurch entstehenden Multikoordinatensysteme für die Darstellung des Faltungsvorgangs zur Verfügung stehen, und dass alle oben erwähnten charakterisierten Ausführungsformen und Vorgänge der Proteinfaltung dadurch in ihrer Vielfalt visuell dargestellt werden können. Unter der Transformierbarkeit der 4 Ordinaten versteht man z. B. den Austausch von hydrodynamischer Größe oder Gelbande mit dem Energiezustand der Faltung, dem Zeitablauf der Faltung mit der Konfigurationsabnahme der Faltung, der Faltungswegezugehörigkeit mit dem Wegeablauf der Faltung, der Menge des Intermediates mit dem prozentualen Beitrag der Faltung. Unter der Kombinierbarkeit ist eine Hybridisierung der Ordinaten zu verstehen, beispielsweise führen der Zeitablauf der Faltung und die Menge des Intermediates zur Kinetik der Faltung. Der Energiezustand und die Menge des Intermediates führen zum Beitrag zur Faltung, und der Wegeablauf und die Menge des Intermediates führen zur Kompetenz der Faltung usw..

Die sich daraus ergebende Beziehung zwischen räumlicher Struktur und Energiezuständen der Intermediate eines rückfaltenden Proteins kann in der Vielfalt seiner Formen vom multidimensionalen Energielandschafts-Modell quantitativ veranschaulicht dargestellt werden. Die Faltungswege eines Faltungsvorgangs in einem Oktant des Koordinatensystems können beispielsweise wie die vom Gipfel nach unten führenden Skispuren dargestellt oder in 2 oder 4 Oktanten je nach dem Beitrag zur Faltung verteilend definiert werden, wie die Spuren, die von einer hohen Ebene bis zur niedrigsten Stelle des Tals beim Fallen hinterlassen werden, und dem bekannten Trichter-Modell (Schultz, 2000) entsprechend visualisiert werden.

Man wird anerkennen, dass das erfindungsgemäße Verfahren durch NMR- oder Kristallbestimmung der 3-dimensionalen Strukturen aller oder nur der signifikanten Intermediate mit einer zusätzlichen 5ten Charakteristik für die Identifizierung der Intermediate erweitert wird und dass der Faltungsvorgang der Proteine dadurch auf Basis der 5 Charakteristiken der Intermediate, nämlich der hydrodynamischen Größe, dem Entstehungszeitpunkt, der Wegezugehörigkeit, des prozentualen Beitrags zur Rückfaltung und der dreidimensionalen Struktur charakterisiert und in einem multidimensionalen Koordinatensystem mit mindestens 5 Ordinaten dargestellt und in aller Vielfalt visualisiert wird.

**Vorteile des erfindungsgemäßen Verfahrens**

Die der Erfindung zugrunde liegenden Probleme des Stands der Technik werden gemäß den Merkmalen der Patentansprüche der vorliegenden Erfindung aufgehoben. Die Vorteile des erfindungsgemäßen Verfahrens im Vergleich zum Stand der Technik werden in folgenden Abschnitten zusammengefasst.

Dem erfindungsgemäßen Verfahren liegen mindestens 4 individuelle und digitalisierbare Charakteristiken der Intermediate zugrunde, nämlich die hydrodynamische Größe, der Entstehungszeitpunkt, die Faltungswegezugehörigkeit und die Menge. Dafür können umfangreiche Methoden der Bioanalytik durch flexible Technikzusammenstellung optimal und effizient eingesetzt werden. Dadurch wird ermöglicht, dass die Charakterisierung des Faltungsvorgangs nur wenig Proteinmaterialien braucht, leicht handhabbar, zeitsparend, standardisierbar und routinemäßig durchgeführt werden kann. Diese Durchführung kann weitergehend automatisiert und miniaturisiert werden.

Das erfindungsgemäße Verfahren basiert auf der Vielfalt der Modifikation für die Feststellung der Wegezugehörigkeit der Intermediate. Die Vielfalt der Möglichkeiten zur Modifikation des rückfaltenden Proteins gewährleistet hierbei, dass jedes dabei entstehende Intermediat entsprechend modifiziert und dadurch individuell markiert und daher von anderen differenziert werden kann. Dies bezieht daher alle Proteine ein. So werden zum Beispiel die kleinen und großen, die disulfidhaltigen und disulfidfreien, die stark sauren und die stark basischen, die hydrophilen und hydrophoben und die Membran- und Multidomänenproteine erfasst.

Die Auftrennung der Intermediate und die Feststellung ihrer hydrodynamischen Größen, der Entstehungszeitpunkte und der Menge können sowohl direkt durch die erfindungsgemäße Gelelektrophorese für die hydrophilen und globulären Proteine effizient erfüllt werden, als auch durch die Flüssigchromatographie und die anschließenden spektrometrischen Untersuchungen vorzugsweise mit DLS (Dynamische Lichtstreuung) leicht erzielt werden. Hierbei können alle Proteine, wie oben aufgeführt, zur Untersuchung miteinbezogen werden.

Das strukturierte proteolytische System aus Trypsin, daneben Lys-C, Glu-C Asp-N und Exoprotease sowie chemische Spaltung in Kopplung mit MALDI-TOF-MS/MS (Tandem-Massen-spektrometrie) und MALDI-TOF-MS-PSD (Post Source Decay-Massenspektrometrie) kann gewährleisten, dass alle Intermediate nach ihren Spaltungsbehandlungen in geeigneten Fragmenten mit passenden Modifikationsmerkmalen für die Herausbildung der Fragmente-Erkennungsmuster vorliegen, spektrometrisch erfaßt werden und zur weiteren Bearbeitung entsprechend digitalisiert werden können.

Der dadurch charakterisierte Vorgang der Faltung eines Proteins kann multidimensional in seiner Vielfalt dargestellt werden und die quantitative Veranschaulichung der dem Trichter-Konzept (Schultz, 2000) entsprechenden Beziehung zwischen räumlicher Struktur und Energielandschaft der Intermediate eines rückfaltenden Proteins wird hierbei ermöglicht.

Die Vorteile des erfindungsgemäßen Verfahrens liegen außerdem in seinen unterschiedlichen Anwendungen. In einer ersten bevorzugten Anwendung der Erfindung wird das Verfahren für die Charakterisierung der Faltung aller Arten, Typen und Größen von Proteinen, die Aufklärung des Faltungsmechanismus, einschließlich der Faltungswege, des Vorgangs der Missfaltung und des Ablaufs der intramolekularen Umlagerungen usw. sowie die Erkennung der Ursache bestimmter Proteopathy zur Verfügung gestellt.

In einer zweiten Anwendung der Erfindung wird das Verfahren für die Untersuchung, Überprüfung und Bewertung der Aktivitäts- und Funktionalitätsveränderungen eines rückfaltenden Proteins gegenüber seines Substrats zur Verbesserung oder Optimierung seiner biotechnologischen Herstellung benutzt, wobei die Faltungsvorgänge der zu untersuchenden Proteine zuerst ohne, dann mit Substrat in variierenden Molekularumgebungen erfindungsgemäß charakterisiert, verglichen und ausgewertet werden.

In einer dritten Anwendung der Erfindung wird das Verfahren für das Protein-Engineering benutzt, wobei z.B. die auf den Erkenntnissen der charakterisierten Faltungsvorgänge beruhende Modifizierung, das Redesign und die Fusion der Proteine und ihre darauf zurückführbaren Veränderungen der Funktionalität und Aktivität eines Proteins erfindungsgemäß überprüft, untersucht und bewertet werden.

In einer vierten Anwendung der Erfindung wird das Verfahren für die dynamische Charakterisierung und Quantifizierung der Vorgänge der simulierten *in vitro* Biosynthese und womöglich vorkommenden co- und posttranslationalen Modifizierungen beispielsweise zur Bedingungsoptimierung der biotechnologischen Herstellung der *in vitro* posttranslational modifizierten Proteintherapeutika und dem Scanning der chemischen und biologischen Beihilfe- oder Hemmstoffe der co- und/oder posttranslationalen Modifizierungen verwendet, wobei die Charakterisierung der Vorgänge der simuliert dynamisch *in vitro* co- und posttranslationalen Modifikationen erfindungsgemäß definiert durchgeführt und den entsprechenden Vergleichen und Auswertungen unterzogen wird.

In einer fünften Anwendung der Erfindung wird das Verfahren für die dynamische Charakterisierung des Rückfaltungsvorgangs der Proteine während ihrer *in vitro* simulierten bekannten *in vivo* posttranslationalen Modifizierungen zur Verfahrensentwicklung ihrer biotechnologischen Herstellung gebraucht. Die Charakterisierung dieser Vorgänge folgt den erfindungsgemäß definierten Schritten. Die zu untersuchenden Proteine können in Kopplung mit der Technologie der Proteinimmobilisierung und Proteinchipherstellung auf dem Träger immobilisiert, nach simulierten *in vivo* posttranslationalen Modifikationen von der chemischen, enzymatischen oder den Zellextrakt enthaltenden Reaktionslösung befreit, anschließend durch thermische, photochemische, chemische oder enzymatische Abspaltung von Träger abgetrennt und weitergehenden Schritten des erfindungsgemäßen Verfahrens unterzogen werden.

In einer sechsten Anwendung der Erfindung wird das Verfahren für die Suche nach biologischen oder chemischen Wirkstoffen auf die Proteinfaltung benutzt, wobei die ausgewählten biologischen oder/und chemischen Kandidaten-Hemmstoffe oder/und -Hilfsproteine bei der erfindungsgemäßen Charakterisierung der Rückfaltung des gesuchten Proteins mit einbezogen werden.

In einer siebten Anwendung der Erfindung wird das Verfahren für die Untersuchung der Wirkung der Foldasen oder Chaperone auf die Proteinfaltung benutzt, wobei die Wirkung durch den Vergleich der erfindungsgemäß charakterisierten Faltungsvorgänge mit und ohne die zu untersuchenden Foldasen oder Chaperone definiert wird.

In einer achten Anwendung der Erfindung wird das Verfahren für die Suche nach neuartigen Pharmatherapeutika, die zu den biologischen und chemischen Hemmstoffen der Foldasen/-Chaperonen und den Proteinen mit Einfluss auf den Proteinabbau gehören, gebraucht, wobei das zu untersuchende Protein in parallelen, z. B. Foldasen oder Chaperone enthaltenen Ansätzen jeweils mit und ohne Kandidat-Inhibitoren der Foldasen oder Chaperone den erfindungsgemäßen Charakterisierungen der Faltungsvorgänge unterzogen wird. Durch den anschließenden Vergleich der Faltungsvorgänge sind die Wirkungen der Kandidat-Substanzen auf die Hemmung der Foldasen/Chaperone und den Proteinabbau festzustellen.

In der neunten Anwendung der Erfindung wird das Verfahren für die Untersuchung kontrollierter Selbstassemblierung und Polymerisation der Polypeptide oder Proteine bei ihrer Rückfaltung zur Entwicklung und Herstellung der Nano-Proteinmaterialien verwendet, wobei der anfängliche Vorgang der Selbstassemblierung und Polymerisation eines zu untersuchenden Proteins unter biologischen und/oder chemischen Einflussfaktoren erfindungsgemäß charakterisiert wird.

In einer zehnten Anwendung der Erfindung wird das Verfahren zur Suche nach pharmakologischen Chaperonen gegen Proteopathy eingesetzt, wobei die Wirkung bestimmter biologischer und/oder chemischer Faltungsstabilisierungssubstanzen, die sich spezifisch an ungefaltete Proteine binden, die die Faltung verbessern und die Proteinstruktur stabilisieren oder die hydrophoben Domänen fehlgefalteter Proteine maskieren und die Löslichkeit erhöhen und damit die Aggregation ungefalteter Proteine verhindern, auf die Rückfaltung der proteopathischen Proteine durch die erfindungsgemäße Charakterisierung festgestellt wird.

In einer elften Anwendung der Erfindung wird das Verfahren für die Charakterisierung des Vorgangs zum einen der Umfaltung des PrP$^c$ ( Prion Protein cellular) zu PrP$^{Sc}$ (Prion Protein Scrapie; pathogene Form des Prion-Proteins) einschließlich gefolgter Aggregation und zum anderen der Umkehrung des PrP$^{Sc}$ zu PrP$^c$ einschließlich des Abbaus der Aggregation zur Klärung der Pathogenese und Suche nach Therapieoptionen und Möglichkeiten zur Prävention verwendet. Die Faltungsvorgänge eines Prion-Proteins werden dabei in parallelen Ansätzen mit biologischen oder/und chemischen Faltungseinflusssubstanzen unter Regulierung der destabilisierenden Bedingungen z. B Temperatur, pH-Wert und Ionenstärke erfindungsgemäß charakterisiert und mit dessen zuvor charakterisierten Faltungsvorgang ohne die Faltungseinflusssubstanzen verglichen.

In einer zwölften Anwendung der Erfindung wird das Verfahren für die dynamische Charakterisierung des Faltungsvorgangs eines gezielten Proteins zur Untersuchung der auf Isomerisierung, Deamidierung und Racemisierung zurückgeführten Proteinalterung und des durch Radikaleinwirkung, oxidativen Stress und Umwelteinflüssen verursachten Proteinverschleißes eingesetzt. Hierbei wird das gezielte Protein bei seiner Rückfaltung entweder einer katalytisch beschleunigten Isomerisierung, Deamidierung und Racemisierung durch Zufuhr von photochemischer und thermischer Energie oder einer von Radikaleinwirkung, oxidativem Stress und Umwelteinflüssen initiierten Modifikation unterzogen. Sein dadurch veränderter Faltungsvorgang wird anschließend in weitergehenden Schritten des erfindungsgemäßen Verfahrens charakterisiert und mit dem Faltungsvorgang ohne diese charakteristischen Einflussfaktoren verglichen wird.

In einer dreizehnten Anwendung der Erfindung wird das Verfahren für die Klassifizierung (Taxonomie) der Proteine und die Erforschung der Proteinevolution auf einer neuen Ebene der dynamischen Proteinfaltung eingesetzt, wobei der erfindungsgemäß charakterisierte Vorgang der Faltung als individueller Fingerabdruck jedes Proteins die funktionellen und evolutionären Zusammenhänge der Proteine liefert und als zusätzliches Kriterium in die bisherige Klassifizierung, die auf Struktur, Topologie, Homologie und evolutionäre Verwandtschaft basierte, integriert werden kann.

In einer vierzehnten Anwendung der Erfindung wird das Verfahren für die dynamische Charakterisierung des Faltungsvorgangs der Proteine bei ihrer Nukleo-, Glyko- und Lipo-Komplexbildung zur Untersuchung ihres Entstehungsvorgangs und dabei begleiteter Aktivitäts- und Funktionalitätsveränderung und der Suche nach den auf die Komplexbildung wirkenden chemischen und biologischen Substanzen gebraucht. Dabei werden die Faltungsvorgänge z. B. eines Proteins und seines Protein-Nukleinsäure-Komplexes zunächst jeweils vereinzelt erfindungsgemäß charakterisiert und dieses Protein und die betreffende Nukleinsäure dann während ihrer Rückfaltung in zeitlichen Abständen in nacheinander folgenden Ansätzen zur Komplexbildung zusammen gebracht und den erfindungsgemäßen Charakterisierungen, Vergleich und den Auswertungen ohne und mit Zufuhr des Substrats des Protein-Nukleinsäure-Komplexes unter variierenden chemischen und biologischen Einflussfaktoren wiederholt unterzogen. Hierbei werden die zusätzlichen Massen der Nukleinsäure-, Kohlenhydrat- und Lipidanteile beim Erstellen des Fragmente-Massenerkennungsmusters entsprechend mitgerechnet. Für die qualitative und quantitative Bestimmung der nicht-kovalenten Anteile dieser Proteine sollen die Ansätze nach der proteolytischen Spaltung und vor den massenspektrometrischen Messungen zunächst einer chromatographischen Auftrennung und dann einer spektrometrischen Festlegung der hydrodynamischen Größen und Massen der Fragmente unterzogen werden.

In einer fünfzehnten Anwendung der Erfindung wird das Verfahren für die Diagnostik und Prognose der von Proteinmissfaltung verursachten Erkrankungen angewendet, wobei die Veränderungen des Faltungsvorgangs der krankheitsbezogenen Proteine charakterisiert, in Form eines Fingerabdruckprofils der Faltung dargestellt und als Kriterien der Diagnostik und ihrer Prognosen bestimmter Erkrankungen definiert werden.

In einer sechzehnten Anwendung der Erfindung wird das Verfahren für die dynamische Charakterisierung des anfänglichen Vorgangs der Aggregation derselben Proteine oder unterschiedlicher Proteine während ihrer Rückfaltung zwischen den faltenden Proteinen oder zwischen den faltenden und nativen Proteinen zur Aufklärung des Mechanismus der Aggregation und Suche nach chemischen und biologischen Hemmstoffen der Proteinaggregation eingesetzt, wobei der Faltungsvorgang des zu untersuchenden Proteins zuerst ohne und dann unter dem Einfluss von anderen Proteinen und/oder chemischen und biologischen Hemmstoffen in unterschiedlichen molekularen Umgebungsbedingungen jeweils erfindungsgemäß charakterisiert, verglichen und ausgewertet wird.

In einer siebzehnten Anwendung der Erfindung wird das Verfahren für die dynamische Charakterisierung des Faltungsvorgangs in der interaktion zwischen den rückfaltenden Proteinen und/oder zwischen den rückfaltenden und nativen Proteinen, zur Untersuchung des Konformationsverhaltens und der Katalyseeigenschaften spezifischer Proteine auf der Ebene ihrer Rückfaltung, zur Suche nach therapeutischen Targetproteinen, zur Ermöglichung eines rationalen Designs von Wirkstoffen und zur Optimierung biotechnologischer Prozesse eingesetzt, wobei die konzipierten Faltungsvorgänge des zu untersuchenden Proteins zuerst ohne und dann unter dem Einfluss von anderen Proteinen in optimierten Molekularumgebungen jeweils erfindungsgemäß charakterisiert, verglichen und ausgewertet werden.

In der achtzehnten Anwendung der Erfindung wird das Verfahren für die dynamische Charakterisierung des Vorgangs der Antigen-Antikörper-Reaktion und des Abbauprozesses des Antigen-Antikörper-Komplexes zur Optimierung und Rationalisierung des Antiköperengineering gebraucht. Dabei werden die neu konstruierten Antikörper und Antigene den erfindungsgemäßen Charakterisierungen, dem Vergleich und den Auswertungen zunächst vereinzelt, dann zusammen in einer optimierten physiologischen und biochemischen Molekularumgebung ohne und/oder mit den chemischen und biologischen Einflussfaktoren zur Untersuchung der Selektivität, Spezifität, Affinität, Faltungseffizienz, thermodynamischen Stabilität, pharmakologischen Kinetik und biotechnologischen Produktivität des redesignten Antikörpers und der Antigenität des Antigens unterzogen.

In der neunzehnten Anwendung der Erfindung wird das Verfahren für die Charakterisierung des Faltungsvorgangs des *in vitro* biosynthetisierenden und womöglich cotranslational modifizierenden Proteins zur Aufklärung initierter Faltung des Proteins während seiner nascenten Biosynthese und zur Suche nach den chemischen und/oder biologischen Substanzen mit Einfluss auf die initiierte Faltung des Proteins verwendet, wobei die in den zellfreien Ansätzen in unterschiedlichen Längen synthetisierten Polypeptid-Fragmente eines Proteins, die unter optimierten biologischen und physikochemischen Einflussfaktoren durch regulierende Zufuhr der notwendigen Aminosäuren mit oder ohne Isotopenmarkierung gewonnen und als Mini-Intermediate bezeichnet werden, zuerst zusammen gesammelt und dann gezielt chromatographisch aufgetrennt und anschließend den Charakterisierungen und dem Vergleich ihrer Faltungsvorgänge erfindungsgemäß unterzogen werden. Hierbei werden die Entstehungszeitpunkte der Mini-Intermediate nach ihren Länge-Verhältnissen zur ganzen Aminosäuresequenz neu definiert.

In einer weiteren Anwendung der Erfindung wird das Verfahren für den Aufbau von Datenbanken, die auf den charakterisierten Faltungsvorgängen der Proteine und dadurch eingeführten Anwendungen basieren und als Servicezentren für die weitergehende Erschließung und Eröffnung neuer Verwendungen zur Verfügung stehen werden, benutzt. Zu diesen Verwendungen gehört zum Beispiel die den Ergebnissen der charakterisierten Proteinfaltung zugrunde liegende neue Modifizierung, Redesign und Fusion der therapeutischen Proteine und die Vorhersage des Faltungsabdruckprofils dieser neu konstruierten Proteine.

Ein besonderer Vorteil der Erfindung besteht darin, dass die Ausführungsformen des Verfahrens je nach Zielsetzung durch ihre Kombinationen und zusätzlichen Technik- und Methodenzusammenstellungen erweitert werden.

Die Erzeugnisse des Verfahrens, die gekennzeichnet durch die in Vielfalt modifizierten Intermediate des Proteins und die dadurch in unterschiedlichen Anwendungen bei unterschiedlichen molekularen Umgebungen unter verschiedenen physikochemischen und biochemischen Einflussfaktoren ermittelten und in einer Datenbank systematisch erfassten Erkenntnisse über die Faltungsvorgänge aller charakterisierten Proteine, können konzipiert und verwendet werden für das Screening der chemischen und biochemischen Wirkstoffe auf die Proteinfaltung, für die Verbesserung und Optimierung der biotechnologischen Herstellung, für die optimale Renaturierung und Aufbewahrung der Targetproteine, für die effiziente Modifizierung, für das erneute Design, für die rationale Fusion der Proteine und zur Verbesserung ihrer strukturellen Funktionalität, Aktivität und pharmakokinetischen sowie pharmakologischen Eigenschaften.

Das erfindungsgemäße Verfahren betrifft die Verwendung der neuartigen Materialien, nämlich die nach optimaler vollständiger Entfaltung rückfaltenden und dabei vielfältig, in zeitlichen Abständen modifizierten Proteine in Form von Intermediaten jeweils mit mindestens 4 eigenständigen Charakteristiken. Diese erfindungsgemäß in unterschiedlichen Ausführungsformen gewonnenen Proteinmaterialien werden verwendet für die Charakterisierungen und multidimensionalen Darstellungen ihrer in variierten molekularen Umgebungen unter verschiedenen physiochemischen und biochemischen Bedingungen erfolgten Faltungsvorgängen zur Aufklärung des Mechanismus der Faltung, Missfaltung, Aggregation, Interaktion, Selbstassemblierung, Polymerisation, Alterung, des Verschleißes und der Biosynthese der Proteine, Verbesserung der Proteinaktivität und - funktionalität, Optimierung der biotechnologischen Herstellung, Entwicklung der Nano-Proteinmaterialien, Anreicherung der Proteintaxonomie und Suche nach neuartigen auf den Einfluss der Proteinfaltung beruhenden biologischen und chemischen Wirkstoffen und Proteintherapeutika.

Mittel zur Ausführung des Verfahrens

Zur Ausführung des erfindungsgemäßen Verfahrens werden die Mittel in Form von Serien-Assay-Kits, speziellen Laborgeräten und spezifischen Softwaren gebraucht. Die Ausführung kann sowohl Schritt für Schritt manuell als auch mittels der teilweise oder/und vollständig automatisierten und weiterhin miniaturisierten Geräte, die nach der Konzeption und dem Entwurf der Erfinder speziell dafür entwickelt und hergestellt werden, erfolgen.

Serien-Assay-Kits

Die Serien-Assay-Kits bestehen überwegend aus den konventionellen Chemikalien, Materialien, den Komponenten und Gebrauchsanweisungen. Sie sind Produkte der Entwicklung, Optimierung und Standardisierung der Versuchsbedingungen und Handhabungen jeder Versuchsschritte bis zur systematischen Durchführung der Charakterisierung der Rückfaltung aller Arten und Typen von Proteinen. Sie werden je nach den Durchführungsschritten, Vorgehensweisen, Ausführungsformen und Anwendungen des Verfahrens jeweils in unterschiedlich einsatzbereiten Assay-Kits im Folgenden eingeordnet und zum Einsatz gebraucht:

- für die Herstellung der optimal entfalteten Proteine mit maximierten hydrodynamischen Größen,

- für die dynamische Modifizierung der immobilisierten Proteine bei ihrer Rückfaltung jeweils mit variiertem Seitenkettenreagenzien,

- für die dynamische Modifizierung des rückfaltenden Proteins mit einzelnen und/oder unterschiedlichen Fluoreszenzfarbstoffen,

- für die dynamische Modifizierungen der rückfaltenden Proteine mit isotopischen oder spinmarkierten Seitenkettenreagenzien,

- für die dynamischen Modifikationen durch die Biotinylierungen bei der Rückhaltung eines Proteins,

- für die dynamische Modifizierung der disulfidhaltigen Proteine jeweils mit variierten Seitenkettenreagenzien und die Erstellung ihrer Rückfaltungsfingerabdruckprofile,

- für die dynamische Single-, Multi- und/oder internen Crosslinkermodifizierungen der rückfaltenden disulfidfreien Proteine jeweils mit variierten Seitenkettenreagenzien inklusive der nulllängen-, homo- und heterobifunktionellen Reagenzien und der Reagenzien für die Photomarkierung und die Erstellung ihrer Rückfaltungsfingerabdruckprofile,

- für die dynamische Modifizierung der rückfaltenden globulären und hydrophilen Proteine jeweils mit variiertenSeitenkettenreagenzien und die Erstellung ihrer Rückfaltungsfingerabdruckprofile,

- für die dynamische Modifizierung der rückfaltenden stark hydrophoben und der Membranproteine mit variierten Seitenkettenreagenzien und die Erstellung ihrer Rückfaltungsfingerabdruckprofile,

- für die dynamische Modifizierung der rückfaltenden stark sauren oder stark basischen Proteine mit variierten Seitenkettenreagenzien und die Erstellung ihrer Rückfaltungsfingerabdruckprofile,

- für die dynamische Modifizierung der isolierten Proteindomäne aus den untereinander unabhängigen Multidomänenproteinen, während ihrer Rückfältung mit variierten Seitenkettenreagenzien und die Erstellung ihrer Rückfaltungsfingerabdruckprofile

- für die dynamische Modifizierung der rückfaltenden untereinander abhängigen und zusammengesetzten Multidomänenproteine und die Erstellung seines Rückfaltungsfingerabdruckprofils,

- für die dynamische Charakterisierung eines rückfaltenden Proteins in den Ansätzen mit seinem Substrat oder seinem potenziellen Substrat unter unterschiedlich auszuwählenden physikochemischen und biochemischen Bedingungen zur Untersuchung und Überprüfung seiner Aktivitäts- und Funktionalitätsveränderungen während seines Rückfaltungsvorgangs,

- für die dynamische Charakterisierung der Rückfaltung der redesignten, oder biotechnisch modifizierten oder/und fusionierten Proteine auf Basis ihrer charakterisierten authentischen Rückfaltungen zur Überprüfung der darauf zurückgeführten Veränderungen der Funktionalität und Aktivität des betreffenden Proteins,

- für die dynamische Charakterisierung der Vorgänge der simulierten *in vitro* Biosynthese und womöglich vorkommenden co- und posttranslationalen Modifizierungen eines Proteins in den zellfreien Ansätzen unter auszuwählenden physikochemischen und biochemischen Molakularumgebungen zur Bedingungsoptimierung der biotechnologischen Herstellung der *in vitro* posttranslational modifizierten Proteintherapeutika,

- für die dynamische Charakterisierung des Rückfaltungsvorgangs der Proteine während ihrer *in vitro* simulierten auszuwählenden *in vivo* posttranslationalen Modifizierungen in den biologischen oder zellfreien Ansätzen unter auszuwählenden physikochemischen und biochemischen Bedingungen zur Verfahrensentwicklung ihrer biotechnologischen Herstellung,

- für die dynamische Charakterisierung des Rückfaltungsvorgangs der Proteine unter Einfluss der auszuwählenden biologischen oder/und chemischen Substanzen zur Untersuhung und Überprüfung der potenziellen Wirkstoffe auf die Proteinfaltung,

- für die dynamische Charakterisierung des Rückfaltungsvorgangs der Proteine in den Ansätzen mit auszuwählenden Foldasen oder Chaperonen zur Untersuchung und Überprüfung ihrer Hilfswirkung auf die Proteinfaltung,

- für die dynamische Charakterisierung des Rückfaltungsvorgangs der Proteine in den Ansätzen mit ausgewählten Foldasen oder Chaperone und ihrer potenziellen biologischen und chemischen Hemmstoffe zur Untersuchung und Überprüfung ihrer Hemmwirkung auf die Proteinfaltung,

- für die dynamische Charakterisierung des Rückfaltungsvorgangs der Polypeptide oder der kleinen Proteine in den Ansätzen mit zusätzlichen biologischen und/oder chemischen Einflussfaktoren zur Untersuchung ihrer kontrollierten Selbstassemblierung und Polymerisation,

- für die dynamische Charakterisierung des Rückfaltungsvorgangs eines proteopathischen Proteins in den Ansätzen mit bestimmten biologischen und/oder chemischen potentiellen Faltungsstabilisierungssubstanzen zur Untersuchung und Überprüfung ihrer pharmakologischen Chaperonwirkung gegen Proteopathy,

- für die dynamische Charakterisierung des Rückfaltungsvorgangs eines Prion-Proteins in parallelen Ansätzen mit biologischen oder/und chemischen Faltungseinflusssubstanzen unter Regulierung der destabilisierenden Bedingungen zur Untersuchung seiner Pathogenese und Überprüfung der Optionen einer möglichen Therapie und Prävention,

- für die dynamische Charakterisierung des Rückfaltungsvorgangs eines gezielten Proteins in den Ansätzen jeweils unter photochemischer und/oder thermischer Energiezufuhr, der Radikaleinwirkung, oxidativern Stress und dem Einfluss der veränderten molekularen Umgebung zur Untersuchung der auf Isomerisierung, Deamidierung und Racemisierung zurückgeführten Proteinalterung und des durch Radikaleinwirkung, oxidativen Stress und Umwelteinflüsse verursachten Proteinverschleißes,

- für die dynamische Charakterisierung des Rückfaltungsvorgangs der Proteine in den Ansätzen mit optimierten Molekularumgebungen unter den nach Arten, Typen und Größen der Proteine standardisierten physikochemischen und biochemischen Bedingungen zur Gewinnung der Informationen über die Proteinklassifizierung (Taxonomie) und Proteinevolution,

- für die dynamische Charakterisierung des Rückfaltungsvorgangs eines Proteins jeweils in den Ansätzen seiner Nukleo-, Glyko- und Lipo- Komplexbildungen unter variierten chemischen und biologischen Einflussfaktoren und regelmäßiger Zufuhr seiner potenziellen Substrate zur Untersuchung ihres Entstehungsvorgangs und der dabei begleiteten Aktivitäts- und Funktionalitätsveränderung sowie der auf die Komplexbildungen ausgeübten Wirkungen der zugegebenen chemischen und biologischen Substanzen,

- für die dynamische Charakterisierung des Rückfaltungsvorgangs eines krankheitsbezogenen Proteins in den Ansätzen unter optimierten und standardisierten physicochemischen und biochemischen Bedingungen zur Erstellung seines Rückfaltungsfingerabdruckprofils als Kriterien für seine Diagnostik und Prognose,

- für die dynamische Charakterisierung des Rückfaltungsvorgangs eines Proteins in den Ansätzen ohne und mit anderen Proteinen unter variierten molekularen Umgebungsbedingungen und regelmäßigem Zugeben der chemischen und biologischen Hemmstoffen (inklusiv potenzieller Hemmstoffe) der Proteinaggregation zur Überprüfung und Untersuchung des Aggregationsmechanismus derselben Proteine oder unterschiedlicher Proteine während ihrer Rückfaltung zwischen den faltenden Proteinen oder zwischen den faltenden und nativen Proteinen,

- für die dynamische Charakterisierung des initiierten Vorgangs der Interaktionen zwischen den rückfaltenden Proteinen oder/und zwischen den rückfaltenden und nativen Proteinen in den Ansätzen ohne und mit den potenziellen Substraten oder Einflussfaktoren unter optimierter Molekularumgebung zur Untersuchung und Überprüfung des Konformationsverhaltens, der Katalyseeigenschaften und der therapeutischen Target-Wirkungen während ihrer Rückfaltung,

- für die dynamische Charakterisierung des Vorgangs der Antigen-Antikörper-Reaktionen und Abbauprozesses des Antigen-Antikörper-Komplexes in den Ansätzen mit den chemischen und biologischen Einflussfaktoren zur Untersuchung und Überprüfung der Veränderungen der Selektivität, Spezifität, Affinität, Faltungseffizienz, thermodynamischen Stabilität, pharmakologischen Kinetik und biotechnologischen Produktivität des betreffenden Antikörpers und der Antigenität des Antigens,

- für die Charakterisierung des Faltungsvorgangs des *in vitro* biosynthetisierenden und womöglich cotranslational modifizierenden Proteins in den zellfreien Ansätzen unter optimierten biologischen und physikochemischen Einflussfaktoren und regulierter Zufuhr der notwendigen Aminosäuren ohne oder mit Isotopenmarkierung zur Untersuchung und Überprüfung initiierter Faltung des Proteins während seiner nascenten Biosynthese und der darauf wirkenden chemischen und/oder biologischen Substanzen,

- für die Herstellung nativer Polyacrylamidgele und die elektrophoretische Auftrennung der bei Rückfaltung des Proteins dynamisch modifizierten Intermediate,

- für die elektrophoretische Auftrennung der bei Rückfaltung des Proteins dynamisch modifizierten Intermediate mit den einsatzbereiten nativen Polyacrylamidgelen,

- für die multidimensionalen flüssigchromatographischen Auftrennungen der bei der Rückfaltung des Proteins modifizierten Intermediate mit parallelen und seriellen Einweg-Mikrosäulen und/oder -Mikrosäulenmodul,

- für die Auftrennungen der bei der Rückfaltung des Proteins modifizierten Intermediate mit einzelnen, parallelen und seriellen Mikrosäulenelektrochromatographie,

- für die Pufferwechsel der bei der Rückfaltung des Proteins modifizierten Intermediate mit Einweg-Mikrosäulen und/oder -Mikrosäulenmodul,

- für die Aufkonzentrierung der bei der Rückfaltung des Proteins modifizierten und aufgetrennten Intermediate mit Einweg-Mikroultrafiltrationssäulen und/oder -Mikroultrafiltrationssäulenmodul,

- für den mikrowellenunterstützten In-Gel-Verdau der Intermediate in den speziell dafür hergestellten multifunktionellen Mikrotiterplatten mit spezifischen Werkzeugen zur manuellen Behandlung der trennenden Gelbanden,

- für den multiplen proteolytischen In-Gel-Verdau und der zusätzlichen chemischen Spaltung der Intermediate in den multifunktionellen Mikrotiterplatten,

- für den mikrowellenunterstützten In-Lösung-Verdau der Intermediate in den multifunktionellen Mikrotiterplatten.

Jedes der oben beschriebenen einsatzbereiten Assay-Kits enthält mindestens eine der folgenden Chemikalien, bzw. Materialien und den Komponenten in variierten Formen,

- die unterschiedlichen Pufferlösungen, Denaturierungsmittel, Reduktionsmittel, Reoxidationsmittel inklusiver ihrer variablen Komponenten und Zusammensetzungen, seitenkettespezifische Reagenzien ohne oder mit Isotopen-, Spin- und Fluoreszenzmarkierung, Biotinylierungsreagenzien, Reagenzien zur internen Crosslinker-Markierung, gelelektrophoretische Reagenzien, als Foldasen und Chaperone bezeichnete Faltungsfaktoren, potenzielle Inhibitoren der Foldasen und Chaperone, biologische und chemische Hilfs- und Hemmstoffe der Proteinfaltung und des -abbau, Stabilisierungssubstanzen der Proteinfaltung, spezifische Zellen-Extraktionen für die *in vitro* Proteinsynthese und posttranslationalen Modifikationen usw.,

- die unterschiedlichen Flaschen, Probenröhrchen, Reaktionsgefäße, multifunktionellen Mikrotiterplatten, die Gelbandenpicker, spezielle Spritzen für die Herstellung des Gradient-Gels, vorgefertigtes natives Gel, die Einweg-Mikrosäulen und -Mikrosäulenmodule der Flüssigchromatographie und der Elektronchromatographie, die Einweg-Mikroultrafiltrationssäulen und - Mikroultrafiltrationssäulenmodule, die Proteinimmobilisierungsträger und entsprechende Hilfssoftware uws..

Die Assay-Kits werden nicht auf oben genannte Beispiele beschränkt sein, da die neuen Assay-Kits je nach Bedarf zur Ausführung bestimmter Schritte des erfindungsgemäßen Verfahrens durch die Kombination und Komplementierung oder Aufteilung der fünktionsgemäßen Komponenten der vorhandenen Assay-Kits weiter entwickelt und hergestellt werden können.

Spezielle Laborgeräte

Die für die Ausführung des Verfahrens zur Charakterisierung der Proteinfaltung einzusetzenden Laborgeräte involvieren

- den mikrowellenunterstützten quenched-flow-Apparat für die dynamische Reoxidierung und zugleich Modifizierung der Intermediate,

- die Mikrowellen-Miniapparatur für die Modifikation der Intermediate anschließbar mit quenchedflow Probenkopf,

- die Gelelektrophorese-Apparatur für die elektrophoretische Auftrennung der modifizierten Intermediate,

- die Apparatur für die elektrochromatographische Auftrennung der modifizierten Intermediate,

- den digitalen Gelscanner für die graphische Aufnahme und qualitative Quantifizierung der getrennten und gefärbten Gelbanden,

- den digitalen Mikrofluoreszenzscanner für die graphische Aufnahme und die qualitative Quantifizierung der getrennten und fluoreszierenden Gelbanden und insbesondere für die digitale Differenzierung der unterschiedlichen Intermediate in ähnliche hydrodynamische Größen,

- den Gelbandenpicker für die Probenvorbereitung des In-Gel-Verdaus,

- die Mikrowellen-Verdau-Apparatur für In-Gel- und In-Lösung-Verdau,

- das automatisierbare Module-Set der seriellen Mikrofiltrationssäule für die chromatographische Auftrennung der modifizierten Intermediate,

- die verbesserte DLS- und SLS-Messapparatur ( dynamische und statische Lichtstreuung) für die systematische Bestimmung und Differenzierung der hydrodynamischen Größe der Intermediate in den multifunktionellen Mikrotiterplatten,

Konzeption und Entwurf für die automatisierte Ausführung des Verfahrens

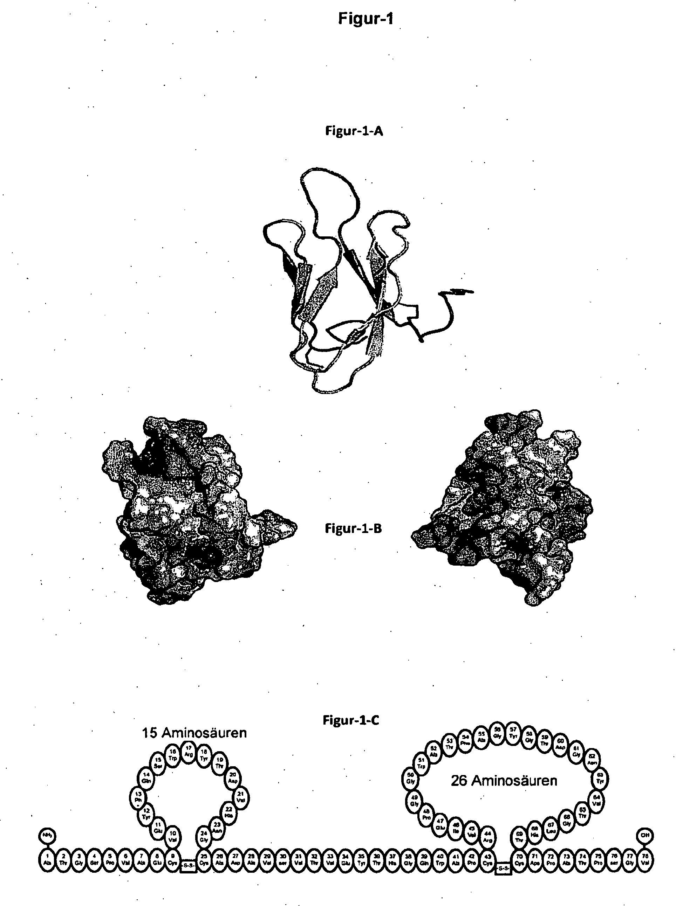

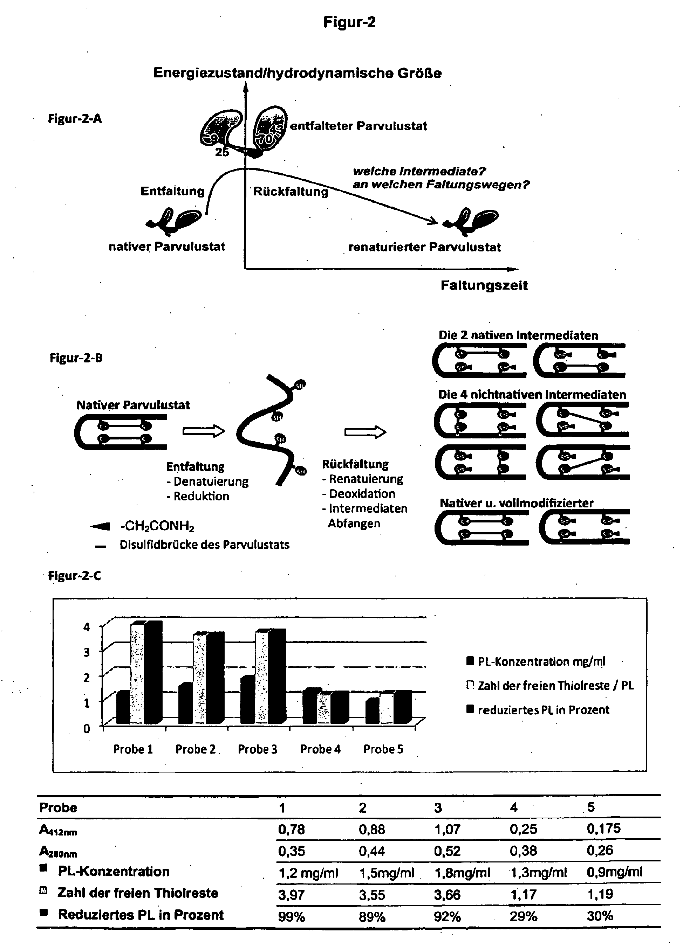

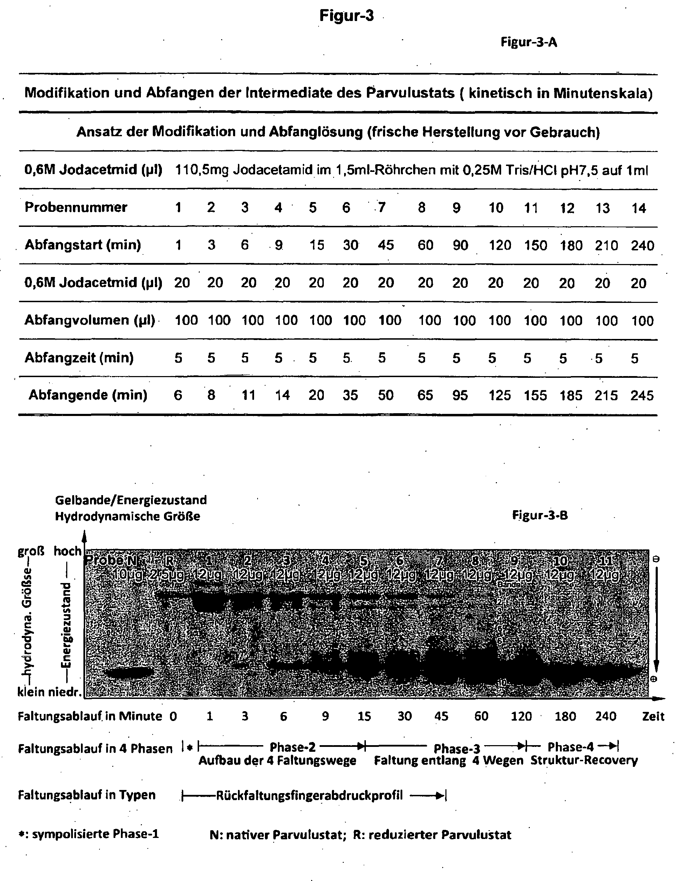

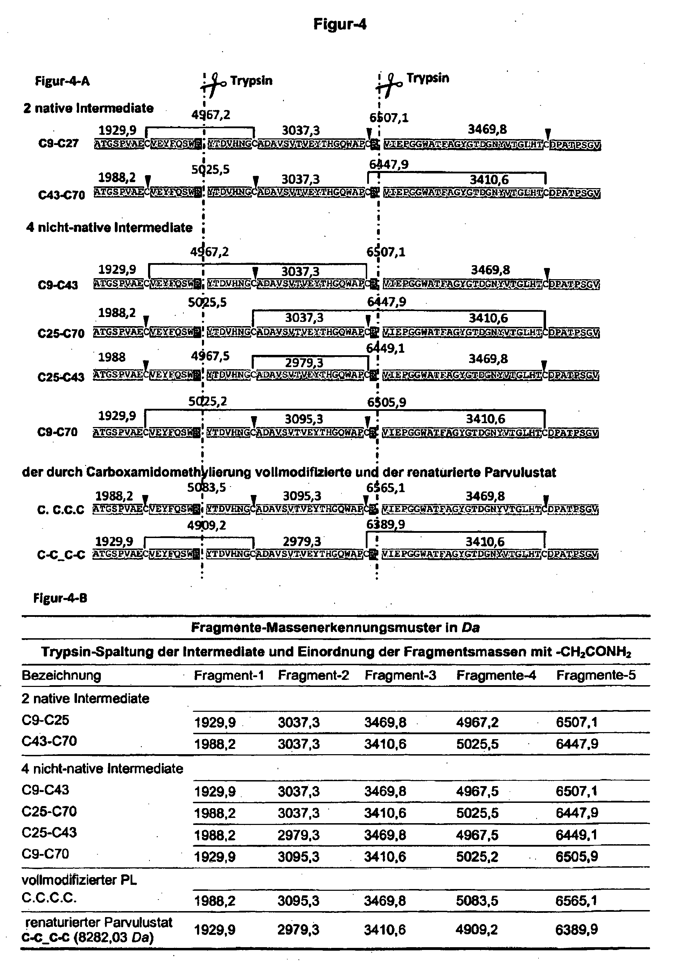

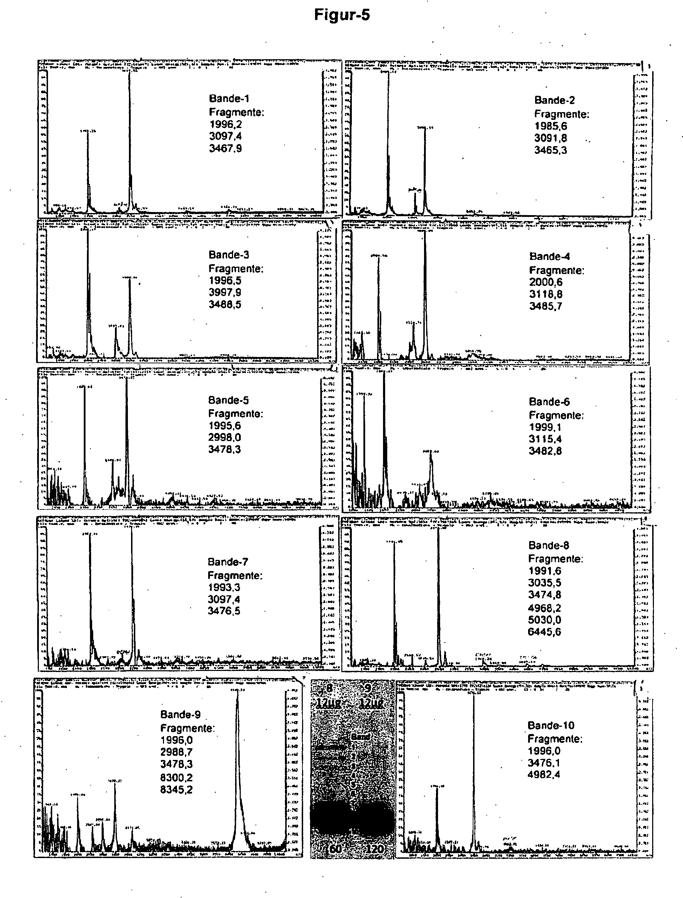

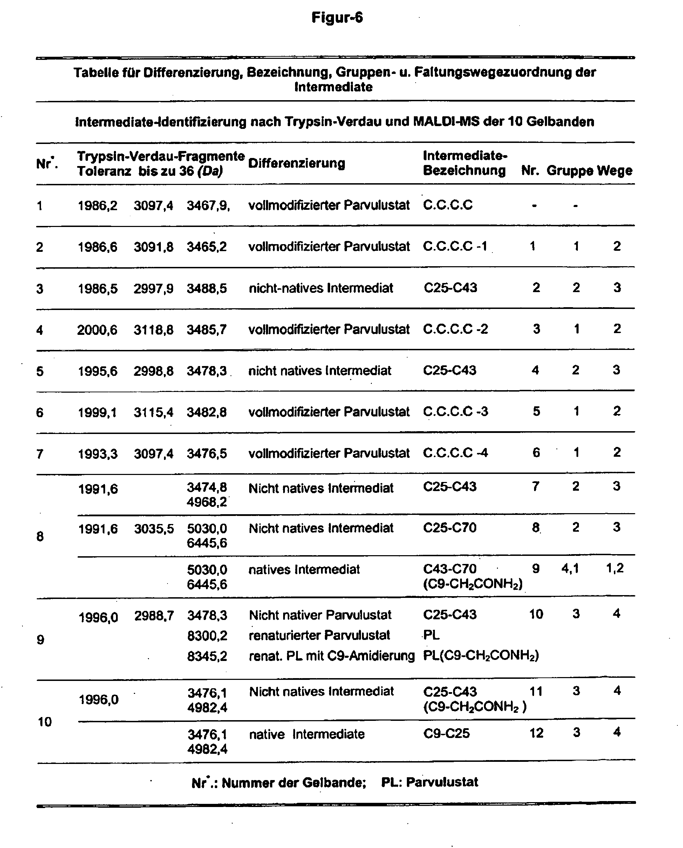

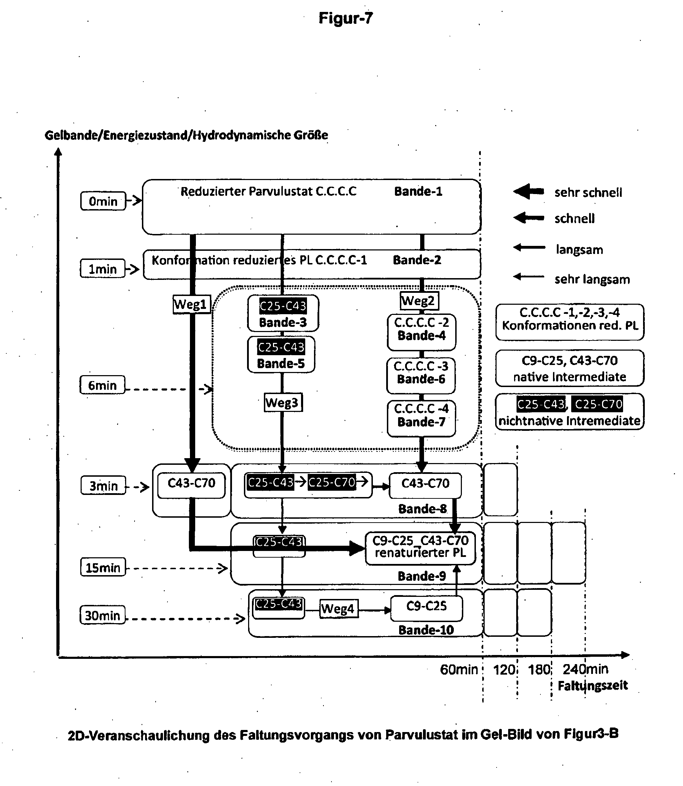

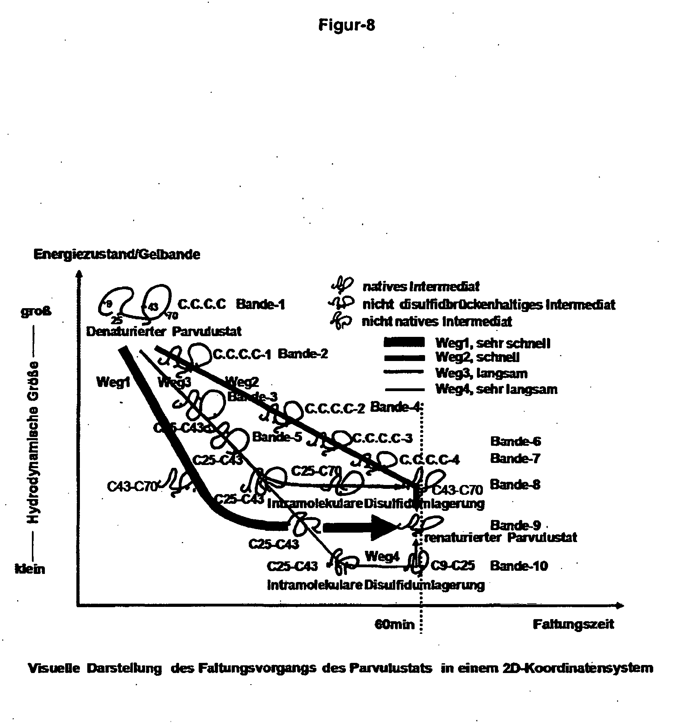

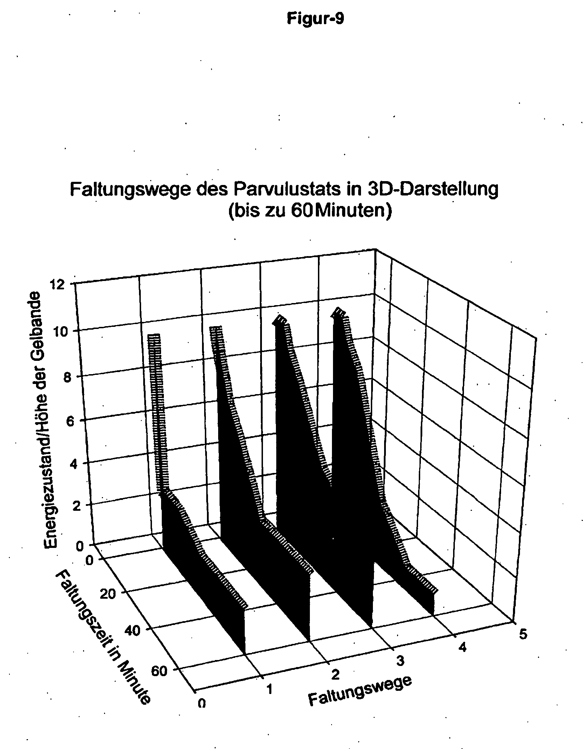

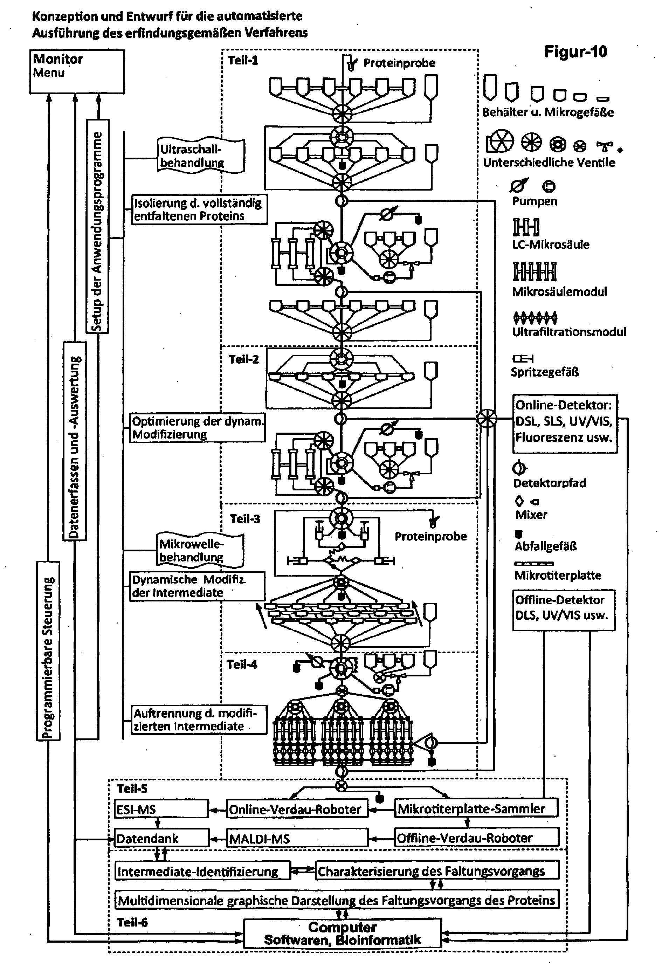

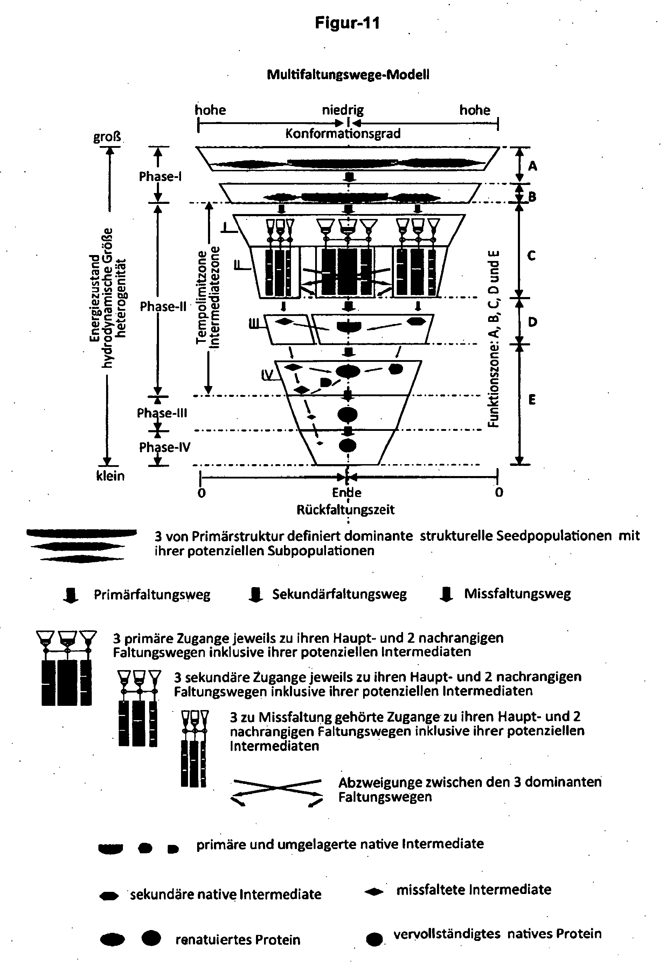

Die Ausführung des Verfahrens kann in einem gemäß der Konzeption und dem Entwurf des Erfinders hergestellten Automat automatisiert werden. Der Automat soll aus 6 jeweils mit Teil-1 bis - 6 in Figur 10 gezeichneten Funktionseinheiten bestehen. Ihre Funktionen werden im Folgenden detailliert.

Funktion-Teil-1 steht zur Isolierung des optimal entfalteten Proteins. Zum Funktionsträger zählen die Pumpe, die Ventile, die Chromatographiesäule oder die Feldflussfraktionierungskanäle und die seriellen, nachfüllbaren oder/und Einweg-Gefäße für die Bereitstellung unterschiedlicher Denaturierungslösungen und Entfaltungsansätze. Die Ansatzgefäße können hierbei auch mit Thermostat versehen werden. Das bereits angemessen in Denaturierungsmittel gelöste Protein wird zunächst durch die Pumpe in einem Zugang des zweiten Ventils zugeführt und dann durch Umschalten auf das erste Ventil und das daher erfolgte Einfließen von gewählter Denaturierungslösung in ein Ansatzgefäß gebracht und dem Ultraschall oder Schütteln zur Mischung unterzogen. Das hierbei nach den programmierten Bedingungen denaturierte Protein verfügt über unterschiedliche Strukturgröße und wird durch die Pumpe in die Chromatographiesäule oder den Feldflussfraktionierungskanal gebracht. Die hydrodynamischen Größen und ihre Verteilung der dadurch aufgetrennten Proteingruppe werden spektrometrisch mit DLS in einer Datenbank erfasst. Dieser Prozess wird mit unterschiedlichen Denaturierungslösungen unter vorprogrammierten Bedingungen wiederholt. Danach wird ein Ansatz mit den besten Reaktionsbedingungen für das Gewinnen des Anteils des optimal entfalteten Proteins, das über eine relativ stabile maximale hydrodynamische Größe verfügt, festgelegt. Zum Schluss wird der vom Computer erkannte optimale Ansatz nochmal wiederholt durchgeführt und das dadurch gewonnene Eluat mit maximaler hydrodynamischer Größe des Proteins von DLS detektiert und von der Pumpe zur Funktion-Teil-2 eingeleitet.

Funktion-Teil-2 steht zur Optimierung der dynamischen Modifizierung des Proteins. Die Funktionsträger sind so wie in Funktion-Teil-1, aber im viel kleineren Volumenstil. Das kleine Einweg-Gefäßemodul kann hierbei auch für die Bereitstellung der vielfältigen Modifikationsreagenzien angeschlossen werden. Das aus Funktion-Teil-1 eingeleitete Eluat wird zunächst mit Orientierung der Aufbauphase der Rückfaltungskanäle in Zeitskalen von Sekunden bis Minuten nacheinander jeweils mit verschiedenen zugeschalteten Modifizierungsreagenzlösungen in den Ansatzgefäßen verteilt und zugleich zur Modifizierung gebracht. Die Modifizierungsansätze werden dann in der Reihenfolge zur flüssigchromatographischen Auftrennung des rückfalteten Proteins mit unterschiedlichen hydrodynamischen Größen weiter eingeleitet. Durch die spektrometrische Detektierung der Eluate werden die Verteilungsprofile der hydrodynamischen Größen für alle durchgeführten Modifizierungsansätze gewonnen, wobei der beste Ansatz mit optimalem Modifizierungsreagenz, der zur breiten Verteilung der relativ stabilen und chromatographisch abtrennbaren Intermediate führt, festgelegt und für die dynamische Quench-Flow-Modifizierung in Funktion-Teil-3 reserviert wird.

Funktion-Teil-3 steht zur dynamischen Quench-Flow-Modifizierung des Proteins. Die Funktionsträger umfassen die Quench-Flow-, Mikrowelle-, Probenauffang-Module und die Ventile usw.. Das optimale Eluat aus Funktion-Teil-1 wird direkt in einem von 4 Spritzegefäßen des Quench-Flow-Moduls eingeleitet. Die Rückfaltungspufferlösung oder die Reoxidationslösung, die von Funktion-Teil-2 festgelegte optimale Modifizierungsreagenzlösung und die Stabilisierungslösung werden jeweils in andere Spritzegefäße eingeleitet. Die Rückfaltung des Proteins wird im ersten Mixer gestartet. Die dynamische Modifizierung erfolgt in zeitlichen Abständen durch Mischung der Modifizierungsreagenzlösung im zweiten Mixer, wobei der Mixer und die Delay-Tube zur Beschleunigung der Reaktionen mit einer speziellen Mikrowelleneinheit behandelt werden. Die nacheinander portionsweise modifizierten und zugleich in der Delay-Tube abgefangenen Intermediate können entweder zur strukturellen Stabilisierung weiter durch den dritten Mixer oder direkt zu den Mikrogefäßen des autobewegbaren Probenauffang-Moduls geleitet und für die weitere Auftrennung in Funktion-Teil-4 bereitgestellt werden.

Funktion-Teil-4 steht zur flüssigchtomatographischen Auftrennung der dynamisch modifizierten Intermediate. Die Funktionsträger bestehen aus dem Einweg-Mikrosäule- und Einweg-Ultrafiltrationsmodul, den Mikrotiterplatten, den Pumpen und Ventilen usw. Die in den Mikrogefäßen von Funktion-Teil-4 gelagerten Modifizierungsansätze können in der ersten Vorgehensweise zuerst einer nach dem anderen in die Mikrosäule zugeführt werden, dann den chromatographischen Auftrennungen der Intermediate und den Aufkonzentrierungen der abgetrennten Intermediat-Eluate unterzogen werden, zuerst im Einweg-Mikrosäulen-Modul- und danach im Einweg-Ultrafiltrationsmodul, wobei die von DLS detektierten und von der Ultrafiltrationssäule konzentrierten Eluate weitergehend zu Funktion-Teil-4 online geliefert werden. In der anderen Vorgehensweise werden die Modifizierungsansätze zunächst vereinzelt in die Mikrosäulen des Einweg-Moduls eingeleitet. Dann werden die von der Pumpe getriebenen gleichzeitigen Auftrennungen der Intermediate und anschließend die Aufkonzentrierungen der Elutionen in den verstärkten Ultrafiltrationssäulen des Einweg-Moduls ohne den DLS-Detektiv vollzogen, wobei die Elutionen direkt in den Mikrotiterplatten aufgenommen werden und für die nächsten Offline-Operationen zur Verfügung stehen. Funktion-Teil-5 steht zur proteolytischen Spaltung und massenspektrometrischen Datenerfassung der fragmentierten Intermediate. Die nacheinander zusammengestellten Funktionsträger sind der Online- und Offline-Verdau-Roboter mit Ankoppelung der Mikrowelleneinheit, der ESI-MS, der MALDI-MS, die Mikrotiterplatten, angeschlossen mit DLS-Detektor usw.. Die aus Funktion-Teil-4 mit erster Vorgehensweise gelieferten Eluate werden zunächst zur proteolytischen Spaltung im Online-Verdau-Roboter gebracht, Die dadurch fragmentierten Intermediate werden anschließend online zur ESI-MS für die Datenerfassung der Anzahl und der Massen der Fragmenten der Intermediate in allen Eluaten eingeleitet. Die aufgetrennten Intermediate in den Eluaten, die durch die zweite Vorgehensweise von Funktion-Teil-4 in Mikrotiterplatten aufgefangen worden sind, werden hierbei durch die angekoppelten DLS- und UV-Messungen erkannt, differenziert, quantifiziert und anschließend einer proteolytischen Spaltung im Offline-Verdau-Roboter unterzogen. Die Datenerfassung der Fragmente aller Intermediate erfolgt danach durch offline MALDI-MS-Messungen der selektierten Eluate in Mikrotiterplatten. Die beiden Verdau-Roboter sollen jeweils mit einer Mikrowelleneinheit versehen werden.

Funktion-Teil-6 steht zur Ausführung der drei Aufgaben, nämlich des Setups der Anwendungsprogramme, der Prozesssteuerung, der Datenauswertung und der graphischen Darstellung der Ergebnisse. Die Funktionsträger umfassen die Kontroll- und Anschlusskomponenten, Computersystem und Software für die Automatisierung, die Auswertung und die Darstellung. Beim Setup des gewählten Anwendungsprogramms wird ein Prozessplan erstellt, geprüft, als ein im laufenden Betrieb veränderbares Flussdiagramm visualisiert und automatisch ausgeführt. Der Verlauf des Prozessplans wird ebenso visualisiert und während seiner Überwachung und Kontrolle protokoliert und archiviert. Die sich aus DLS- UV- und Massenspektrometrie-Messungen ergebenen Daten werden automatischen erfasst und auf vielfältige Weise ausgewertet Alle Intermediate werden hierbei nach ihren 4 Charakteristiken identifiziert und nach ihren Faltungswegezugehörigkeiten klassifiziert. Die darauf beruhende Charakterisierung der Faltungsvorgänge des Proteins und ihre multidimensionalen Visualisierungen erfolgen mit spezieller Software ebenso automatisch.

Ein Vorteil dieser Konzeption und dieses Entwurfs liegt darin, dass Funktion-Teil-1 bis -5 jeweils durch die vereinzelte Zusammenstellung mit dem Funkition-Teil-6 zu 5 kleinen untereinander anschließbaren Automaten für die eigenständige Ausführung bestimmter Schritte des erfindungsgemäßen Verfahrens aufgebaut werden können. Auf Basis dieser Konzeption und des Entwurfs können die Automaten weiterhin miniaturisiert werden.

Spezielle Software

Die speziell für die Durchführung der jeweiligen Schritte des erfindungsgemäßen Verfahrens gebrauchte Software umfasst:

- das Programm zur Klassifizierung des Proteins je nach Größe des Molekulargewichts, der Art des Proteins, disulfidhaltig oder disulfidfrei, des Types, hydrophil oder hydrophob, der Eindomäne- oder Multidomänenproteine und der physikochemischen Eigenschaften der Proteine und der darauf beruhenden Entscheidungen für die Auftrennungsmethode, Puffersysteme und die physikochemischen und biochemischen Bedingungen,

- das Programm für die Auswahl der seitenkettenspezifischen Modifikationsreagenzien auf Basis der primären und dreidimensionalen Struktur des Proteins,

- das Programm für den Betrieb der Mikrowellen-Miniapparatur zur schnellen Modifikation der Intermediate in Kopplung mit dem Quenched-Flow-Apparat,

- das Programm für den Betrieb des spezifisch verbesserten Gelelektrophorese-Apparats und die Einkopplung mit dem digitalen Geiscanner, Mikrofluoreszenzscanner und den Gelbandenpicker,

- das Programm für den Betrieb der chromatographischen Auftrennung der modifizierten Intermediate mit dem Einweg-Module-Set der seriellen Mikrofiltrationssäule und der Mikroultrafiltrationssäule,

- das Programm für die Differenzierung, Einordnung und die zweidimensionale Graphikdarstellung der hydrodynamischen Größe und Menge als Funktion des Faltungszeitablaufs der Intermediate, basierend auf den spektrometrischen Messdaten,

- das Programm für die Heraustellung und die in Vielfalt visualisierten Darstellungen des Rückfaltungsfingerabdruckprofils eines Proteins auf Basis der Messdaten aus der erfindungsgemäß definierten ersten 2 Phasen oder allen 4Phasen seiner Rückfaltung,

- das Programm für den Betrieb der Mikrowellen-Verdau-Apparatur für den mittleren Proben-durchsatz von In-Gelund In-Lösung-Verdau in Kopplung mit Gelbandenpicker und Einweg-Module-Set der seriellen Mikrofiltrationssäule und Mikroultrafiltrationssäule,

- das Programm für die Heraustellung der theoretischen Fragmente-Erkennungsmuster auf Basis der primär- und 3-dimensionalen Struktur des Proteins und der proteolytischen Spaltung seiner modifizierten Intermediate, für die darauf beruhenden Vergleiche mit den spektrometrisch erfassten Messdaten und die weitergehenden Auswertungen,

- das Programm für die Herausstellung der theoretischen Fragmente-Erkennungsmuster der *in vitro* posttranslational modifizierten und proteolytisch gespaltenen Intermediate, für die darauf beruhenden Vergleiche mit den spektrometrisch erfassten Messdaten und die weitergehende Auswertungen,

- das Programm für die Herausstellung der theoretischen Fragmente-Erkennungsmuster der bei der *in vitro* simulierten Rückhaltung der unter den chemischen und/oder biologischen Einflussfaktoren modifizierten und proteolytisch gespaltenen Intermediate, desweiteren für die darauf beruhenden Vergleiche mit den spektrometrisch erfassten Messdaten und die weitergehenden Auswertungen,

- das Programm für die Herausstellung, Differenzierung und Auswertung der theoretischen Fragmente-Erkennungsmuster der proteolytisch gespaltenen Intermediate in Bezug auf die Single-, Multi- und internen Crosslinker-Modifizierungen jeweils mit allen bekannten und nicht bekannten, aber gleich wirkenden seitenkettenspezifischen Reagenzien, ohne oder mit Isotopen-, Fluoreszenz- und Spinmarkierung einschließlich der nulllängen-, homo- und heterobifunktionellen Reagenzien und der Reagenzien für die Photomarkierungen und Biotinylierungen,

- das Programm für die Klassifizierung der Gruppenzugehörigkeit der identifizierten Intermediate,

- das Programm für die Feststellung der Wegezugehörigkeit der in Gruppen klassifizierten Intermediate,

- das Programm für die in Vielfalt visualisierten Darstellungen des charakterisierten Faltungsvorgangs des Proteins im multidimensionalen Koordinatensystem,

- das Programm für die multidimensionalen Animationen des Ablaufs einer charakterisierten Proteinfaltung,

- das Programm für die multidimensionalen Animationen des Ablaufs einer charakterisierten Proteinfaltung und der dabei erfolgten *in vitro* posttranslationalen Modifizierungen,

- das Programm für die Simulierung und Vorhersage des Faltungsvorgangs eines Proteins, basierend auf der charakterisierten Proteinfaltung seines homologen Proteins und der *de novo* Vorhersage der Strukturelemente von Intra- und Internetserver,

- das Programmpaket für den Automaten der Charakterisierung der Proteinfaltung.

Diese Programme können je nach den Anwendungszwecken jeweils als unabhängige Produkte vereinzelt oder als Bestandteil der oben genannten Assay-Kits und Laborgeräte in unterschiedlichen Paketen angeboten werden.

**Ausführungsbeispiele**

Die vorliegende Erfindung wird nunmehr durch die folgenden Beispiele veranschaulicht. Diese dienen dazu, bestimmte bevorzugte Ausführungsformen und Aspekte der vorliegenden Erfindung zu veranschaulichen, aber nicht so auszulegen sind, dass sie deren Umfang einschränken.

Das Ausführungsbeispiel betrifft die Charakterisierung des Faltungsvorgangs des in *Streptomyces lividans* TK24 überexprimierten $\alpha$-Amylaseinhibitors Parvulustats (Z-2685) aus *Streptomyces parvulus* FH-1641. Parvulustat ist aufgrund seiner klar definierten Pharmakophorstruktur, Irreversibilität der Bindung an das Enzym und niedrigen Dissoziationskonstante von $2,8 \times 10^{-11}$ M/L ein effektiver Hemmstoffe der $\alpha$-Amylase, der die Aufnahme von Glukose über den Darm ins Blut reduziert und verlangsamt und somit ein potenzielles Antidiabetikum gegen Diabetes Typ **II** ist. Seine dreidimensionale Struktur ist bereits durch NMR-Bestimmung aufgeklärt (Rehm et. al., 2009; pdb, 2KER). Die Charakterisierung des Faltungsvorgangs des Parvulustats ist für seine biotechnologische Herstellung und die Erforschung seiner pharmakokinetischen Eigenschaften von wichtiger Bedeutung.

Beispiel 1

Analyse der strukturellen und physikochemischen Eigenschaften des Parvulustats

Parvulustat (Figur-1-A) besteht aus 78 Aminosäuren mit einem Molekulargewicht von 8282.09 Da. Seine Ami-

nosäurensequenz lautet ATGSPVAECVEYFQSWRYTDVHNGCADAVSVTVEYTHGQW-APCRVIEPGGWATFA-GYGTDGNYVTGLHTCDPATPSGV. Er verfügt über 4 Cysteine, 5 Proline, 2 Arginine, 3 Tryptophane, 5 Tyrosine, 2 Phenylalanine, 3 β-Faltblätter, 6 β-Turns und zwei Loopstrukturen resultierend aus den 2 Disulfidbrücken, die jeweils von Thiolen des Cysteins-9, -25 und -43, -70 gebunden sind **(Figur-1-C)**. Er hat 8 negativ und 2 positiv geladene Reste jeweils aus 4 Asparaginsäuren, 4 Glutaminsäuren und 2 Argininen. Sein α-Amylase hemmendes Aktivzentrum der Triade Trp[16]-Arg[17]-Tyr[18] als Pharmakophor befindet sich am β-Turn der β-Faltblattstruktur in erster Loopstruktur. Sein isoelektrischer Punkt ist 4.3. Bei pH7.0 wird er von 5.9 netto Negativladungen beladen. Das Verhältnis zwischen den hydrophilen und hydrophoben Resten liegt bei 1:3. Seine molekulare Oberfläche ist hydrophil und polarisiert **(Figur-1-B).** Parvulustat ist ein kleines kompaktes Protein, das sich durch totale Denaturierung in 7M Guanidiniumhydrochlorid-Lösung und anschließende Renaturierung ohne Aktivitätsverlust in den nativen Zustand zurückfalten kann (**Figur-2-A**).

Ausgehend vom Ergebnis der computerunterstützten Analyse, dass Parvulustat über 8 theoretisch mögliche durch native und nicht native Disulfidbildung resultierten Intermediate bei der Rückfaltung verfügen kann **(Figur-2-B),** wurde das Fragmente-Massenerkennungsmuster **(Figur-4-B)** hergestellt. Es wurde noch entschieden, dass die Auftrennung der Intermediate, die Differenzierung ihrer hydrodynamischen Größen und Festlegung der Reihenfolge dieser Größen mit nativer Polyacrylamidgelelektrophore gleichzeitig erfolgen und dass die Modifikation des rückfaltenden Parvulustats mit dem ladungsneutralen seitenkettenspezifischen Reagenz von Jodacetamid an Thiolgruppen der Cysteine durchgeführt wird und dass die anschließende proteolytische Fragmentierung der aufgetrennten Intermediate in Gelbanden mit Trypsin In-Gel-Verdau erfolgt **(Figur-4-A).** Der Faltungsvorgang des Parvulustats wurde auf dieser Basis weitergehend erfindungsgemäß charakterisiert.

Beispiel 2

Auftrennung optimal entfalteter Proteinprobe mit maximierten hydrodynamischen Größen

Parvulustat wurde vollständig denaturiert, reduziert und von Reduktionsmittel befreit. Dabei wurde der Anteil des optimal entfalteten Parvulustat, der über eine maximale hydrodynamische Größe verfügt und dessen Disulfidbindungen zu freien Thiolgruppen komplett reduziert wurden, flüssigchromatographisch aufgetrennt und für die Reoxidation und die dynamische Modifikation in den nächsten Schritten bereitgestellt.

Denaturierung und Reduzierung des Parvulustats erfolgen mit dem Denaturierungspuffer von 6M bekanntem Denaturierungsmittel GdmCl (Guanidiniumchlorid), 0,2M Tris, 1mM EDTA, pH8.7 und Reduktionspuffer von 0,2M bekanntem Reduktionsmittel DTE (1,4-Dithioerythrit), 6M GdmCl, 0,2M Tris, 1mM EDTA, pH8.7. Parvulustat ist aufgrund seiner stark polarisierten Ladungen auf der molekularen Oberfläche leicht in Lösung zu aggregieren. Seine höchste Konzentration bei der Denaturierung in 6M Guanidinhydrochlorid GdmCl Lösung darf daher nicht 2,4mg/ml überschreiten, um Parvulustat vollständig zu denaturieren, reduzieren und die intermolekularen Disulfidbildung zu vermeiden. 5mg Parvulustat in einem 1,5ml Eppendorf Cap wurde zuerst mit 1,25ml Denaturierungspuffer versetzt, 2 min vortext und 15 min mit Ultraschall bei 37 °C behandelt, dann in ein 4ml-Röhrchen in dem zuvor 1,25ml Reduktionspuffer eingefüllt wurde überführt und nach Mischung und 5 minütiger Ultraschallbehandlung weiter 1 Stunde bei Raumtemperatur zur vollständigen Denaturierung und Reduzierung der Disulfidbrücken gebracht. Die Konzentration des Parvulustats in dieser Lösung beträgt 2mg/ml.

Nach der Denaturierung und Reduktion muss der reduzierte Parvulustat vom Reduktionsmittel DTE gründlich abgetrennt werden. Jede Spur von DTE in der Parvulustatiösung würde die anschließenden Untersuchungen schwer beeinträchtigen. Die Befreiung von Reduktionsmittel DTE erfolgt mit zwei seriellen Gelfitrationssäulen. Zwei PD-10 Säulen wurden zuerst jeweils mit 20ml Auftrennungspuffer bei pH2 äquilibiert. Dann wurde 2,5ml reduzierte Parvulustatiösung in die erste Säule überführt. Nach dem Eintauchen der Lösung wurde 2,5ml Auftrennungspuffer nachgeführt und zugleich wurde die 2,5ml eluierte Parvulustatlösung direkt in die zweite PD-10 Säule herein getropft. Zum Schluss wurde noch 2,5ml Auftrennungspuffer in die zweite Säule eingeführt und dabei wurde die Parvulustatlösung portionsweise jeweils 0,5ml in 5x1,5ml-Röhrchen nacheinander aufgenommen und anschließend die Konzentration des reduzierten Parvulustats und die Anzahl reduzierter Cystein-Thiol-Reste (-SH) bestimmt.

Die Konzentrationsbestimmung des reduzierten Parvulustats dient der Kontrolle des Materialgehalts des Parvulustats in der Lösung und zur Abrechnung der Zahl der freien Thiolgruppen in dem Molekül. Hierbei wurde die photometrische Messung im UV-Bereich 280nm als bevorzugte Methode verwendet. Diese Methode bedient sich der Absorption der aromatischen Aminosäuren von 3 Tryptophanen, 5 Tyrosinen und zwei Disulfidbrücken des Parvulustats. Sein $A_{280}$-Wert bei einer Konzentration von 1 mg/ml liegt bei 2,92, was viel höher ist als die von den meisten Proteinen erzeugten Werte von 0,5 bis 1,5. Zur Beibehaltung der Linearität der Abhängigkeit des Absorptionswertes von der Konzentration des Parvulustats soll der gemessene $A_{280}$-Wert dabei 1,0 nicht überschreiten. Es entspricht der Konzentration des zu messenden Parvulustats in der Pufferlösung im Bereich von 20 bis 500µg/ml, die meistens durch die entsprechende Verdünnung der Lösung erreicht wird. Bei den Messungen wurden die Parvulustatproben zuerst zehnfach verdünnt. Die jeweils 0,1ml reduzierte und von DTE befreiten Parvulustatlösungen von 5 Proben wurden in 5x1,5ml-Röhrchen, die zuvor mit 0,9ml pH7,5-Phosphatpuffer aufgefüllt wurden, überführt. Nach der Mischung wurden die Absorptionswerte $A_{280}$ der 5 Lösungen im UV-Spektrometer gemessen. Den Absorptionskoeffizient $\varepsilon_{280}$ kann man mit der folgenden ersten Gleichung (Pace et al., 1995) ermitteln. Die Konzentrationen des Parvulustats wurden mit der zweiten Gleichung unten ohne Anwendung der Eichmethode direkt berechnet.

$$\varepsilon_{280} = (Trp)(5,500) + (Tyr)(1,490) + (Disulfidbrücken)\ (125)$$

$$= (3)(5,500) + (5)(1,490) + (2)\ (125) = 24200\ (M^{-1}cm^{-1})$$

$$C\ (mg/ml) = 10.A_{280}/\varepsilon_{280}.d = A_{280} \times 82860mg\ M^{-1}cm^{-1} / 24200\ M^{-1}cm^{-1} = 3,42x\ A_{280}$$

Es ist entscheidend für die erfolgreiche Charakterisierung des Faltungsvorgangs, das zu untersuchende Protein vollständig zu denaturieren und seine Disulfidbrücken zu freien Thiolgruppen komplett zu reduzieren. Die Bestimmung der freien Thiolgruppen der 5 Proben dient dazu, die beste der 5 Proben mit sicher vollständig reduziertem Parvulustat, das über alle vier freie Thiolgruppen (-SH) und damit die maximale hydrodynamische Größe verfügt, als Ausgangsmaterial für die anschließende Reoxidation, Modifikation und das Abfangen der Intermediate zu verwenden. Die Bestimmung der freien Thiolgruppen erfolgt mit Ellmansreagenz aus 2mg 5,5'-Dithiobis(2-nitrobenzoesäure) (DTNB) /ml, 0,1mM EDTA und 0,1 M Phosphatpuffer pH7,5. Jeweils 0,1ml reduzierte Parvulustatlösung von 5 Proben wurde in fünf 1,5ml-Röhrchen, die zuvor mit 0,8ml pH7,5-Phosphatpuffer aufgefüllt wurden, überführt und durch leichtes Umkippen gemischt. Dann wurde jeweils 0,1ml Ellmansreagenz in diese 5 Röhrchen zugegeben. Nach der Mischung wurden die Absorptionswerte der 5 Lösungen bei einer Wellenlänge von 412nm im Spektrometer gemessen. Hierbei wird Ellmansteagenz (DTNB) durch eine SH-SS-Austauschreaktion mit reduziertem Parvulustat zum gleichen stöchiometrischen Produkt von $TNB^{2-}$ mit einer intensiv gelben Färbung und einem molaren Absorptionskoeffizienten von $13600M^{-1}cm^{-1}$ bei 412nm gebracht. Die Intensität der Färbung ist proportional zu den freien Thiolgruppen im Parvulustat-Molekül. Die Zahl der freien Thiolreste des Parvulustats in der Probenlösung ist daher durch Dividieren der Konzentration von $TNB^{2-}$ und Parvulustat in der Lösung stöchiometrisch in der folgenden Gleichung zu ermitteln( Riddles *et al.,* 1983). Es wurde dadurch festgelegt, dass Parvulustat im ersten 0,5ml Eluat mit einer Konzentration von 1,2mg/ml über vier vollständig reduzierte Thiolgruppen und daher die größte molekulare hydrodynamische Größe verfügt **(Figur-2-C)**. Die anschließenden Untersuchungen wurden daher mit dieser Probe durchgeführt.

$$Anzahl\ der\ freien-SH = \frac{Konz.\ von\ TNB^{-2}}{Konz.\ von\ Parvulustat} = \frac{A_{TNB^{-2}(412nm)}/13600\ M^{-1}cm^{-1}}{A_{Parvulustat(280nm)}/24200\ M^{-1}cm^{-1}}$$

Beispiel 3

Reoxidation und dynamische Modifikation des reduzierten Parvulustats

Die dynamische Modifizierung des disulfidhaltigen Parvulustats mit 4 vollständig reduzierten Thiolgruppen erfolgt bei seiner Reoxidation. Die Reoxidation dient zur Bildung der nativen und gegebenenfalls nicht nativen Disulfidbrücken des Parvulustats aus den reduzierten freien Cystein-Thiolgruppen bei seiner Rückfaltung. Diese Bindung zwischen den Thiolen ist nicht spontan, selbst wenn sie unmittelbar benachbart sind. Sie hängt vor allem vom Redoxpotential ab, das heißt, von der effektiven Konzentration passender Elektronen-Donatoren und Akzeptoren in der Umgebung der Thiole. Zur Bildung der Disulfidbrücken muß ein geeigneter Elektronenakzeptor vorhanden sein. Dafür stehen zahlreiche bekannte Oxidationsmittel zur Verfügung. Ein effektives und in den Reoxidationsversuchen oft verwendetes Oxidationsmittel, Glutathion in seiner reduzierten (GSH) und oxidierten (GSSG) Form (Creighton & Goldenberg, 1984), wurde hier mit einem bevorzugten Verhältnis von reduziertem (GSH) zu oxidiertem (GSSG) Glutathion von 10:1 verwendet.
Die Reoxidation erfolgte durch die Mischung des Reoxidationspuffers von 0,1M Tris/Ac, 10mM EDTA, pH8, der Reoxidationslösung von 10mM GSH und 1mM GSSG im Reoxidationspuffer und dem vollständig reduzierten Parvulustat aus dem ersten Eluat. 0,15 ml Reoxidationslösung wird zuerst mit 1,16 ml Reoxidationspuffer in einem 4ml-Röhrchen gemischt. Die Reoxidation wird dann durch Zugeben von 0,19 ml des ersten Eluats und kurzes Vortex gestartet. Die Konzentration an Parvulustat in dieser Lösung beträgt 0,18 mg/ml. Dieser 1,5ml Reoxidationsansatz liefert $15x100\mu l$ Proben für die dynamische Modifikation einschließlich der dabei erfolgten abgefangenen Intermediate des Parvulustats.

Die Reoxidation wird erst dann in Gang gebracht, wenn alle Ansätze für die anschließenden Modifikationen zum Abfangen der Intermediate sorgfältig vorbereitet sind.

Die dynamische Modifizierung beginnt in der Reihenfolge mit den Reaktionen des in zeitlichen Abständen herausgenommenen Reoxidationsansatzes mit dem bekannten seitenkettsspezifischen Modifikationsreagenz Jodacetamid, das alle bei der Rückfaltung und der Reoxidation noch übrigen und zugänglichen Thiolgruppen durch Carboxamidomethylierung irreversibel blockiert und zu den neutralen Amidogruppen am Cysteinrest reagiert. Zuerst werden jeweils 20$\mu$l Modifikationsreagenz von 0,6M Jodacetamid, 0.25M pH7,5 in 14 1,5ml-Röhrchen pipettiert, dann wird die Reoxidation wie im oben beschriebenen 1,5ml Ansatz gestartet, anschließend wird jeweils 100$\mu$l Reoxidationsansatz nach den geplanten zeitlichen Abständen von 1, 3, 6, 9, 15, 30, 45, 60, 90, 120, 150, 180, 210 und 240 Minuten, insgesamt 14 Mal, entnommen und sofort ins Röhrchen mit 20$\mu$l Modifikationsreagenz überführt, gemischt, nach 5minütiger Inkubation bei Raumtemperatur mit flüssigem Stickstoff behandelt und in der Tiefkühltruhe aufbewahrt **(Figur-3-A)**. Die Konzentration an Parvulustat in diesen Proben beträgt jeweils ca.12$\mu$g/100$\mu$l. Der rückfaltende und dabei reoxidierende Parvulustat wird dadurch je nach Zugänglichkeit der betreffenden Thiolgruppen der Cysteine in unterschiedlichem Ausmaß zu den strukturell relativ stabilen Intermediaten jeweils mit eigenen individuellen Charakteristiken modifiziert und steht dann für die gelelektrophoretische Auftrennung und weitergehende Identifizierung bereit.

Beispiel 4

Auftrennung und Quantifizierung der Intermediate und zweidimensionale Darstellung ihrer hydrodynamischen Größen als Funktion ihrer Entstehungszeitpunkte

Die Auftrennung der modifizierten Intermediate und die gleichzeitige Darstellung ihrer hydrodynamischen Größen erfolgen mit der erfindungsgemäß verbesserten diskontinuierlichen Nativ-Polyacrylamid-Gelelektrophorese in einer Apparatur von Hoefer Scientific SE600 nach Davis und Ornstein. Bei nativer Gelelektrophorese erfolgt die Wanderung der Proteine in Abhängigkeit von ihren Nettoladungen und Konformationen. Daher eignet sich diese Art der Elektrophorese besonders für die Auftrennung und Darstellung der bei der Rückfaltung modifizierten Proteinintermediate, die über ein fast gleiches molekulares Gewicht verfügen, aber strukturell in unterschiedlichen hydrodynamischen Größen vorliegen. Das Gel sollte möglichst einen Tag vor der Verwendung zur vollständigen Polymerisierung hergestellt und im Kühlschrank aufbewahrt werden. Das große Gel (16cmx18cmx0,1cm) hat 16 Probentaschen. In jede Tasche können maximal 120$\mu$l Probe mit 12-15$\mu$g Protein aufgeladen werden.

In der ersten und letzten Tasche des Gels wurden aufgrund des oft vorkommenden Randeffekts keine Proben aufgetragen. In die zweite und dritte Tasche des Gels wurden jeweils eine 100$\mu$l Probe des nativen Parvulustats und eine 100$\mu$l Probe des vollständig reduzierten und mit Jodacetamid modifizierten Parvulustats nach der Mischung mit 20$\mu$l Probenpuffer aufgetragen. Die 11 von 14 während der Rückfaltung und Reoxdation durch Carboxamidomethylierung im oben angegebenen Zeitschema von 1, 3, 6, 9, 15, 30, 45, 60, 120, 180 und 240 Minuten modifizierten Parvulustat-Proben, werden aus der Tiefkühltruhe herausgenommen und anschließend im Speed-Vac bis auf 100$\mu$l aufkonzentriert. Dann werden jeweils 20$\mu$l Probenpuffer in alle 11 Proben überführt. Nach der Mischung wurde jeweils 100$\mu$l von diesen Proben in den 11 Taschen auf das Gel aufgetragen. Das Gel (20%A, 3,25%T) lief 2 Stunden mit 120V für Sammelgel und anschließend 4 Stunden mit 180V für Trenngel in Kopplung mit einem Kühlthermostat sowie dem diskontinuierlichen Austausch des Laufpuffers im Kühlraum bei 4°C **(Figur-3-B)**.

Nach dem Gellauf und anschließender Coomassie-Färbung und Entfärbung wurden die bei der Rückfaltung in den angegebenen zeitlichen Abständen durch Modifikation abgefangenen Intermediate nach ihrem Rückfaltungszeitablauf und ihrer gleichzeitig gestalteten hydrodynamischen Größe in Form von seriellen Gelbanden dem 4 phasigen Multifaltungwege-Modells entsprechend zweidimensional auftrennend dargestellt **(Figur-3-B)**.

Auf diesem Gel-Bild **(Figur-3-B)** ist festzustellen, dass die gesamten bei der Rückfaltung entstandenen intramolekularen Intermediate mit unterschiedlichen hydrodynamischen Größen in 10 Banden erscheinen, dass sich die Arten, Anzahl und Quantität der Intermediate im Zeitablauf der Rückfaltung verändern, dass die in der ersten Phase der Faltung instinktiv superschnellen herausgebildeten Seedstrukturen der Faltungswege im ersten Gelband darstellt, der in zweiter Phase erfolgte Aufbau der Faltungswege oder -kanäle innerhalb von 15 bis 30 Minuten erreicht ist und sich dabei ein Rückfaltungsfingerabdruckprofil gebildet hat, dass der in der dritten Phase entlang dieser Wege oder Kanäle erfolgte Durchlauf der Faltung mehr als 2 Stunden dauert und, dass die weitergehende intramolekularen Umlagerung zur endgültigen Vervollständigung der nativen Struktur ca.4 Stunden braucht.

Es ist noch zu sehen, dass das vollständig denaturierte, reduzierte und gleich nach dem Start der Reoxidation mit Jodacetamid modifizierte Parvulustat, der aufgrund der erhöhten Sperrigkeit der 4 vergrößerten Cysteinreste über die höchste hydrodynamische Molekulargröße verfügt und dadurch am langsamsten wandert im Gel, wobei der native und renaturierte Parvulustat wegen seiner kompakten gleichen Strukturen gleich schnell im Gel wandert. Desweiteren ist zu sehen, dass die Gelbanden mit gleichen Höhen aus unterschiedlichen Zeitpunkten der Rückfaltung gleiche Intermediate aufweisen und dass die hydrodynamische Größe der Intermediate in Gelbande-10 kleiner ist, als die des nativen Parvulustats in Gelbande-9. Das Gel-Bild wurde gescannt und steht für die weitere Identifizierung der Intermediate in den Gelbanden bereit.

Beispiel 5

Fragmentierung der Intermediate durch In-Gel-Verdau

Die Fragmentierung der Intermediate aus Gelbanden erfolgt mit Trypsin In-Gel-Verdau. Trypsin (23,23 kDa) ist eine der am häufigsten verwendeten Serinproteasen in der Proteinanalytik, vor allem zur Herstellung von Peptidmustern. Es katalysiert spezifisch die Spaltung vom C-Terminus der Peptidbindung von Arginin und Lysin. Parvulustat hat zwei Arginine aber kein Lysin. Das erste Arginin steht in der Mitte des Inhibitoraktivzentrums Trp16-Arg17-Thr18 und das andere Arg44 steht gerade neben der Disulfidbrücke Cys43-Cys70. Die modifizierten Intermediate wurden vom Trypsin in 3 Fragmente gespalten, wobei noch 2 größere Fragmente durch zusätzliche Bindung der Disulfidbrücken zwischen den Fragmenten entstanden. Daher wurde jedes Intermediat mit 5 Fragmenten in exakten Massen bezeichnet **(Figur-4-A)**.

Der. Trypsin-Verdau erfolgt mit dem erfindungsgemäß verbesserten In-Gel-Verdau von Sigma (Trypsin, Proteomics Grade, Product Code T6567). Die 10 Gelbanden am Rückfaltungszeitpunkt von 60 Minuten wurden jeweils sorgfältig heraus geschnitten und auf einmal dem Trypsin-Verdau mit zusätzlicher Mikrowellen- und Ultraschallbehandlung unterzogen. Zum Schluss wurden alle Verdaulösungen jeweils mit der Pipette in Eppendorf-Röhrchen überführt und im Speed Vac bis 20μl als MALDI-MS-Probe aufkonzentriert. Diese 20μl Proben enthielten je nach Gelbande 0,3-1,5μg Fragmente des Parvulustats, die den Bedarf für MALDI-MS-Messungen völlig gedeckt haben.

Ausgehend davon, dass Parvulustat über acht theoretisch mögliche, durch native und nicht native Disulfidbildung erfolgte Intermediate bei der Rückfaltung verfügt **(Figur-2-B)** und alle diese Intermediate jeweils in 5 Fragmente mit exakten Massen darzustellen sind, wurde das Fragmente-Erkennungsmuster für alle Intermediate in Form einer Tabelle **(Figur-4-B)** mit den jeweiligen Intermediaten und ihren 5 Fragmenten zum Vergleich mit massenspektrometrischen Messdaten und mit dem Hilfsprogramm hergestellt.

Beispiel 6

Massenspektrometrische Detektion der fragmentierten Intermediate

Die Massen-Detektion der proteolytischen Fragmente aller Intermediate aus den Gelbanden erfolgt mit Hilfe von MALDI-MS-Messungen **(Figur-5)**. Die Intermediate von Gelbande-1 bis -7 haben relativ große hydrodynamische Größen und sind alle daher leicht proteolytisch zu spalten. Die Bindungen zwischen den gespalten Fragmenten sind aufgrund der fehlenden Unterstützung der stabilen Sekundärstrukturen relativ schwach und sind deshalb bei der MALDI-Messung leicht zu lösen. Dies führt dazu, dass diese Intermediate alle in 3 getrennten Fragmenten im MALDI-Spektrum vorliegen. Das Fehlen des zweiten Fragments eines Intermediats in Gelbande-8 deutet auf die Existenz der intramolekularen Umlagerung der Disulfidbrücke hin, nämlich, dass die nicht native Disulfidbrücke Cys25-Cys43 zu Cys25-Cys70 weiter zur nativen Disulfidbrücke Cys43-Cys70 umlagert. Das Fehlen des zweiten Fragments des Intermediats in Gelbande-10 zeigt die intramolekulare Umlagerung der nicht nativen Disulfidbrücke Cys25-Cys43 zur nativen Disulfidbrücke Cys9-Cys25. Das auf mehr als drei Fragmente hindeutetende Gemisch der Intermediate in den Gelbanden-8 und -9 wurde durch Elektroeluierung der Gelbanden und anschließender chromatographischer Auftrennung mit den Mikrosäulen bestätigt.

Die gemessenen Massen aller Fragmente, die durch Trypsin-Verdau entstanden sind, wurden entsprechend ihrer untersuchten 10 Gelbanden in einer Tabelle erfasst, in der die allmählich abnehmende hydrodynamische Größe der entsprechenden Gelbande -1 bis -10 von oben nach unten in der ersten Spalte und ihre Fragmente in den Zeilen von links nach rechts in der Reihenfolge als Fragmente-Massenmuster eingetragen wurden **(Figur-6)**. Die den Modifikationen zugrunde liegenden Intermediate mit eigenen Fragmente-Massenmuster wurden dann durch Vergleich mit den theoretischen Fragmente-Erkennungsmuster gekennzeichnet, differenziert und mit einer bestimmten Bezeichnung in die anschließenden Zellen, die für die erkannten Intermediate vorgesehen sind eingezeichnet.

Beispiel 7

Feststellung der Faltungswegezugehörigkeit der Intermediate

Die Wegezugehörigkeit ist die entscheidende Charakteristik eines Intermediates, womit sich das erfindungsgemäße Verfahren als wichtige Merkmal von allen bekannten konventionellen Verfahren unterscheidet. Sie kann nicht allein vereinzelt definiert, sondern von seiner eingeordneten Gruppenzugehörigkeit zu einem bestimmten Faltungsweg identifiziert werden. Die Feststellung der Wegezugehörigkeit der Intermediate beginnt mit der Gruppenzuordnung der identifizierten Intermediate.

In der oben beschriebenen Tabelle wurden insgesamt 12 stabile Intermediate aus 10 Gelbanden nach ihren individuellen Fragmente-Massenmuster differenziert. Es sind die 4 Konformationen der reduzierten Intermediate ohne Disulfidbrückenbildung jeweils aus Gelbande-2, -4, -6 und 7, gekennzeichnet mit C.C.C.C-1, -2, -3 und -4, die 5 Konformationen der nicht nativen Intermediate mit der nicht nativen Disulfidbrückenbildung Cys25-Cys43 jeweils aus Gelbande-3, -5, -8, -9 und -10 , gekennzeichnet mit C25-C43-1, -2, -3 -4 und 5, ein 1 kurzzeitig erscheinendes nicht natives Intermediat aus Gelbande-8 mit einer nicht nativen Disulfidbrückenbildung Cys25-Cys70, gekennzeichnet mit C25-C70, und 2 native Intermediate Cys9-Cys25 und Cys43-Cys70 jeweils aus der Gelbande-8 und-10, gekennzeichnet mit C9-C25 und C43-C70. Der vollständig entfaltete und modifizierte Parvulustat in Gelbande-1 wurde nicht als Intermediat betrachtet.

Alle bezeichneten Intermediate lassen sich jeweils nach der Gemeinsamkeit ihrer Bezeichnung und ihrem strukturellen Zusammenhang in 4 Gruppen einordnen (Figur-6). Die 4 reduzierten Intermediate ohne Disulfidbrückenbildung mit Bezeichnung C.C.C.C-1, -2, -3 und -4 gehören zur ersten Gruppe. Die 3 nicht nativen Intermediate mit Bezeichnung C25-C43-1, -2, und -3 gehört zur zweiten Gruppe. Dazu gehört auch das andere nicht native Intermediat C25-C70, das ein Produkt der intramolekularen Umlagerung von Intermediat C25-C43-4 ist. Die intermediat C25-C43-4 und 5 sowie das umgelagerte native Intermediat C9-C25 bilden sich die dritte Gruppe. Das signifikanteste native Intermediat C43-C70 vertritt die vierte Gruppe. Bei allen 12 Intermediaten lassen sich daher Rückschlüsse von ihrer eigenständigen Gruppenzugehörigkeit in Anbetracht ihrer abnehmenden hydrodynamischen Größen auf die Faltungswegezugehörigkeit schließen, also von den 4 Gruppen auf die 4 Faltungswege. Dadurch wurde die Wegezugehörigkeit aller 12 Intermediate jedes für sich differenzierend festgestellt.

Beispiel 8

Identifizierung und Klassifizierung der Intermediate

Die Identifizierung der 12 Intermediate erfolgte durch Feststellung ihrer hydrodynamischen Größen, des Entstehungszeitpunkts bei der Rückfaltung und der ermittelten Faltungswegezugehörigkeit. Weitergehend wurden diese 12 Intermediate, in 4 Gruppen und Faltungswegen eingeordneten Intermediate, je nach ihren gespielten Rollen für die Weg-Abzweigung, -Erweiterung, -Kreuzung, - Überquerung und das -Zusammentreffen klassifiziert.

Die relativen hydrodynamischen Größen der 12 Intermediate sind durch die unterschiedlichen Höhen der 10 Gelbanden entsprechend festgelegt. Ihre Entstehungszeit bis 60 Minuten nach dem Start der Rückfaltung ist ausschlaggebend. Ihre Faltungswegezugehörigkeit wird im letzten Abschnitt festgestellt. Dadurch sind alle 12 Intermediate ohne Quantifizierung ihrer Menge in der Tabelle (Figur-6) nach ihren 3 Charakteristiken definiert.

Vom veranschaulichten Gel-Bild ist zu ermitteln **(Figur-7)**, dass das zur vierten Gruppe gehörende Intermediat bezeichnet mit C43-C70 3 Minuten nach Start der Rückfaltung entstand und deshalb zum schnellsten Faltungsweg gehört, dass die Intermediate der ersten Gruppe bezeichnet mit C.C.C.C-1, -2, -3 und -4 6 Minuten nach dem Start der Rückfaltung entstanden und daher zum schnellen Faltungsweg gehören, dass die Intermediate der zweiten Gruppe bezeichnet mit C25-C43-1, -2, -3 -4 15 Minuten nach dem Start der Rückfaltung entstanden und zum langsamen Faltungsweg gehören, dass das Intermediat der dritten Gruppe bezeichnet mit C9-C25 30 Minuten nach dem Start der Rückfaltung entsteht und bis zu 3 Stunden dauernd erschien und deswegen zum langsamsten Faltungsweg gehört und dass alle Zustandsformen der Faltungswege wie das Zusammentreffen, die Abzweigung, die Erweiterung, die Kreuzung, oder die Überquerung im Molten Globule Zustand in Gelbande-8 stattfinden.

Es wurde weiterhin festgestellt, dass der schnellste Faltungsweg nur beim Intermediat mit nativer Disulfidbrücke Cys43-Cys70 vorkommt und keine intramolekulare Disulfidumlagerung daraus entsteht, dass die sich in schneller Faltung gruppierenden Intermediate bezeichnet mit C.C.C.C-1, -2, -3 und -4 die nativen Konformationen des reduzierten Parvulustats sind und über keine Disulfidbrücke verfügen, dass der langsame Faltungsweg hingegen über die Konformationen des Intermediats mit nicht nativen Disulfidbrücken Cys25-Cys43 verläuft und von der intramolekularen Disulfidumlagerung von Cys25-Cys43 zu Cys25-Cys70 begleitet wird, und dass die intramolekulare Disulfidumlagerung im Molten Globule Zustand mit einer hydrodynamischen Größe analog zur Gelbande-8 stattfindet.

Beispiel 9

Charakterisierung und visuelle Darstellung des Faltungsvorgangs von Parvulustat in einem 2- und 3-dimensionalen Koordinatensystem

Die Charakterisierung des Faltungsvorgangs des Parvulustats erfolgt durch parallele Darstellung aller sich um die 4 Faltungswege gruppierenden 12 Intermediate zunächst in einem 2-dimensionalen Koordinatensystem, wobei die Gelbanden entsprechend der hydrodynamischen Größen der 12 Intermediate jeweils gegen die Zeitpunkte ihrer ersten Entstehung bis zu 60 Minuten dargestellt wurde (Figur-8). Die hierbei in 3 Arten eingeteilten Intermediate wurden je nach ihren hydrodynamischen Größen und Wegezugehörigkeiten jeweils in seriellen kleinen symbolischen Graphiken mit den allmählich sich verkleinernden Größen in unterschiedlichen Graustufen in diesem Koordinatensystem präsentiert, wobei die logischen Faltungswege mit den Linien in verschiedenen Graustufen und Stärken zur Veranschaulichung dargestellt wurden. In diesem Koordinatensystem wurde die y-Ordinatenachse mit Energiezustände/Gelbande der Intermediate gegen die Zeit des Faltungsablauf auf der x-Ordinatenachse bezeichnet, denn die Energiezustände der Intermediate sind proportional zu ihrer hydrodynamischen Größen, die den unterschiedlichen Banden auf dem Gel entsprechen.

In dieser zweidimensionalen Darstellung ist anschaulich zu erkennen, dass die in 3 Arten eingeteilten 12 Intermediate, nämlich die 4 Konformationen des reduzierten Parvulustats ohne Disulfidbrücken, 2 Intermediate mit nativen Disulfidbrücken und 2 Intermediate mit nicht nativen Disulfidbrücken in 6 Konformationen, bei der Rückfaltung des Parvulustats entstanden, dass sich diese Intermediate in den 4 Faltungswegen als parallele Ereignisse in unterschiedlichen Geschwindigkeiten zum nativen Parvulustat falteten, dass der dem Intermediat mit der zuerst gebildeten nativen Disulfidbrücke (Cys43-Cys70) zugrunde liegende Faltungsweg-1 den Faltungsvorgang dominierte, dass die durch nicht native Disulfidbildung Cys25-Cys43 verursachte Missfaltung die intramolekularen Disulfidumlagerungen Cys25-Cys43 zu Cys25-Cys70, weiter zu Cys43-Cys70 und Cys25-Cys43 zu Cys9-Cys25 bewirkte, stundenlang dauerte und daher den Faltungsvorgang verlangsamte, dass bei allen hierbei stattgefundenen intramolekularen Disulfidumlagerungen ein kompaktes Molekularvolumen auf gleichen Strukturebenen von Molten Globule Zustand in Bande-8 erfolgte, dass Faltungsweg-2 und -3 bei dem Intermediat mit nativem Cys43-Cys70 zusammentrafen, den Faltungsweg-1 überquerte und zu renaturiertem Pavulustat erweiterte, dass der Faltungsweg-4 aus -weg-3 abzweigte und zu einer Disulfidumlagerung führt, dass das Intermediat mit nativer Disulfidbrücke Cys9-Cys25 das letzte vorkommende Intermediat der Rückfaltung war und dass dieses letzte durch intramolekulare Disulfidumlagerung entstehende Intermediat über eine hydrodynamische Größe kleiner als nativ, also einen Energiezustand niedriger als der native Parvulustat verfügt. Der Faltungsvorgang des Parvulustats wurde dadurch vollständig charakterisiert.

Der charakterisierte Faltungsvorgang wurde auch 3-dimensional in einem Multikoordinatensystem dargestellt **(Figur-9),** das jeweils mit dem Energiezustand entsprechend der Gelbande und der hydrodynamischen Größe als y-Ordinate, dem Entstehungszeitpunkt als x-Ordinate und dem Faltungsweg oder -kanal entsprechend ihrer Faltungswegezugehörigkeit als z-Ordinate definiert wurde, wobei die Faltungswege des Parvulustats und ihre vereinfachten Abläufe bis zur 60.sten Minute der Rückfaltung visualisiert wurde.

Beispiel 10

Erzeugnis des Verfahrens

Das Erzeugnis des erfindungsgemäßen Verfahrens bezüglich Parvulustat umfasst u.a. die 12 dynamisch modifizierten, aufgetrennten und nach ihren 4 Charakteristiken identifizierten Intermediate und dadurch gewonnenen Erkenntnisse, dass das Cystein-25 des Parvulustats an der Bildung der nicht nativen Disulfidbrücke Cys25-Cys43 beteiligt und für die Missfaltung der Intermediate bei 2 langsamen Faltungswegen verantwortlich ist, dass diese Missfaltungen die Bildung des aus der β-Faltblattstruktur resultierten Pharmakophors in erster Loopstruktur des Parvulustats verhindert, den gesamten Faltungsvorgang sehr verlangsamt und wegen der abnormen Wirkung und des dadurch initiierten intrazellulären Abbaus dieser falsch gefalteten Intermediate zu einem großen Verlust bei der *in vivo* Biosynthese des nativen Parvulustats führt.

Beispiel 11

Verwendung des Erzeugnisses des Verfahrens

Die vorstehend als Erzeugnis des Verfahrens beschriebenen Erkenntnisse über die Ursache und Folge der Missfaltung des Pavulustats werden hierbei zur Verbesserung seiner pharmakokinetischen Eigenschaften und Erhöhung seiner biotechnologischen Produktivität für die gezielten Modifizierungen, Redesigns und Fusionen des Parvulustats verwendet, wobei zum Beispiel die von der nicht nativen Disulfidbrücke Cys25-Cys43 verursachte Missfaltung und die dadurch bedingte abnorme Wirkung durch gezielten gentechnischen Austausch der Aminosäure Cysteins-25 des Parvulustats gegen Alanin und Threonin eliminiert wird.

**Abbildungen**

Die nachfolgenden Figuren erläutern die Erfindung.

**Figur-1** zeigt die NMR-Struktur des Parvulustats, seine hydrophile und polarisierte Molekularoberfläche von vorderer und hinterer Seite und seine aus 2 Disulfidbrücken resultierenden 2 Loopstrukturen.

**Figur-2** zeigt die schematische Aufgabenstellung der Charakterisierung von Parvulustat in einem 2-dimensionalen Koordinatensystem, die 8 theoretisch möglichen durch native und nicht native Disulfidbildung erfolgten Intermediate bei der Rückfaltung und das für die Modifikation ausgewählte seitenkettenspezifische Reagenz von Jodacetatmid und das Probe-Gewinnen des best optimal entfalteten Parvulustats mit maximalen hydrodynamischer Größen.

**Figur-3** zeigt die Ausführung der dynamischen Modifizierung des rückfaltenden Parvulustats in zeitlichen Abständen und die 2-dimensionale Darstellung der durch Nativ-Polyacrylamid-Gelelektrophorese aufgetrennten Intermediate mit unterschiedlichen hydrodynamischen Größen und entsprechenden unterschiedlichen Gelbanden als y-Ordinate und der Faltungszeit in unterschiedlichen Zeitabständen als x-Ordinate. Die erfindungsgemäß definierten 4 Phasen der Faltung und das Rückfaltungsfingerabdruckprofil werden dabei präsentiert.

**Figur-4** zeigt die Trypsin-Spaltungsmuster der 8 theoretisch möglichen durch native und nicht native Disulfidbildung erfolgten Intermediate bei den Rückfaltung und ihre Fragmente-Massenerkennungsmuster in Tabellenform mit Ansagen der molekularen Massen jedes Fragments.

**Figur-5** zeigt die Ergebnisse der Massen-Detektion der proteolytischen Fragmente aller Intermediate aus 10 Gelbanden mit MALDI-MS-Messungen.

**Figur-6** gibt in einer Tabelle die 12 differenzierten, bezeichneten und in 4 Gruppen und 4 Faltungswegen zugeordneten Intermediate aus 10 Gelbanden durch Vergleich der detektierten Fragmentemassen mit theoretischem Fragmente-Massenerkennungsmuster.

**Figur-7** zeigt die dem veranschaulichten Gel-Bild entsprechend 2-dimensional dargestellte Charakterisierung des Faltungsvorgans von Parvulustat.

**Figur-8** zeigt die Charakterisierung und Visualisierung des Faltungsvorgangs von Parvulustat bis zur 60sten Minute in einem 2-dimensionalen Koordinatensystem.

**Figur-9** zeigt die Visualisierung der Faltungswege und ihrer vereinfachten Abläufe von Parvulustat bis zur 60sten Minute in einem 3-dimensionalen Koordinatensystem.

**Figur-10** zeigt die Konzeption und den Entwurf der Automaten für die automatisierte Ausführung des erfindungsgemäßen Verfahrens

**Figur-11** zeigt das in einem multidimensionalen Energielandschaftkoordinatesystem übertragbare Multifaltungswege-Modell. Das Modell wird mit einer graphischformigen Beschreibung über die auf Experimente beruhte und mit 4 Faltungsphasen und 5 Funktionzonen zusammengefassten neuen Erkenntnissen des Verlaufs der Proteinfaltung dargestellt.

**Patentansprüche**

**1.** Verfahren zum Charakterisieren und multidimensionalen Darstellen des Faltungsvorgangs von Proteinen, **dadurch gekennzeichnet, dass** alle bei der Rückfaltung in zeitlichen Abständen modifizierten und strukturell abgefangenen Intermediate eines Proteins mindestens nach ihren 4 individuellen Charakteristiken, nämlich der hydrodynamischen Größe, des Entstehungszeitpunktes, der Faltungswegezugehörigkeit und der zu quantifizierenden Menge identifiziert und digitalisiert werden, dass ein Faltungsvorgang aus 4 Phasen besteht, dass das Verfahren für die Charakterisierung des Faltungsvorgangs für alle Proteine bereitsteht und das Verfahren die nachfolgenden Schritte umfasst:

1) Analyse des zu charakterisierenden Proteins auf Basis seiner primären, sekundären und gegebenenfalls dreidimensionalen Struktur, der biologischen und physicochemischen Eigenschaften und der Zielsetzung, wobei ein theoretisches Fragment-Massenerkennungsmuster erstellt und die entsprechende Vorgehensweise und Ausführungsform festgelegt werden,
2) Auftrennung des optimal entfalteten Anteils des Proteins mit maximierten hydrodynamischen Größen, wobei das Protein einer Denaturierung, gegebenenfalls einer Reduzierung und einer Befreiung von Reduktionsmittel, der chromatographischen Abtrennung und spektrometrischen Differenzierung unterzogen wird,

3) Dynamische Modifizierung des rückfaltenden und gegebenenfalls zugleich reoxidierenden Proteins, wobei alle in zeitlichen Abständen aufeinander folgenden Intermediate portionsweise vom Ansatz herausgenommen und je nach der Zugänglichkeit ihrer betreffenden Aminosäure reste mit entsprechenden Seitenkettenreagenzien in unterschiedlichen Ausmaßen modifiziert und zu den vielfältigen und strukturell relativ stabilen Intermediaten jeweils mit eigenen individuellen Charakteristiken, nämlich der hydrodynamischen Größe, des Zeitpunktes der Entstehung, der Faltungswegezugehörigkeit und der zu quantifizierenden Menge zugeordnet werden,

4) Auftrennung und Quantifizierung der Intermediate und Darstellung ihrer hydrodynamischen Größen und gegebenenfalls ihrer Menge als Funktion ihrer Entstehungszeitpunkte, wobei die bei der Rückfaltung modifizierten Intermediate entweder elektrophoretisch oder chromatographisch in Kopplung der spektrometrischen Messungen vorzugsweise mit Dynamischer Lichtstreuung (DLS) nach ihrem Rückfaltungszeitablauf und ihren hydrodynamischen Größen im Ablauf des Auftrennens, quantifiziert und multidimensional dargestellt, digitalisiert und für den nächsten Schritt bereit gestellt werden, wodurch ein Rückfaltungsfingerabdruckprofil erstellt wird,

5) Fragmentienrng der Intermediate, wobei die in zeitlichen Abständen modifizierten, aufgetrennten und nach ihren hydrodynamischen Größen differenzierten Intermediate vorzugsweise mit Trypsin gegebenenfalls zusätzlich mit den Endoproteasen Lys-C, Glu-C und Asp-N sowie ausgewählten Exoproteasen proteolytisch und notfalls chemisch als Ergänzung zur Differenzierung der Fragmente gespalten werden,

6) Detektion der fragmentierten Intermediate, wobei die Molekulargewichte aller einzelnen Fragmente und der durch Disulfidbrücken und/oder Crosslinker gebundenen großen Fragmente massenspektrometrisch gemessen, in einer Datenbank erfasst und in einer Fragmente-Massenerkennungstabelle zur Identifizierung aller Intermediate eingeordnet werden. Hierbei werden MALDI-TOF-MS/MS (Tandem-Massenspektrometrie) und/oder MALDI-TOF-MS-PSD (Post-Source-Decay) für die exakte Differenzierung ähnlicher Fragmente eingesetzt,

7) Feststellung der Faltungswegezugehörigkeit der Intermediate, wobei die Art, die Anzahl und der Ort der erfolgten Modifikation als eigene individuelle Merkmale für jedes Intermediat durch Vergleich der massenspektrometrisch erfassten Anzahl und Masse der Fragmente mit dem theoretischen Fragment-Massenerkennungsmuster ermittelt werden und die Intermediate mit gemeinsamen Merkmalen in bestimmte Gruppen zugeordnet werden und diese gruppierten Intermediate weitergehend auf bestimmte Faltungswege schließen lassen, wobei diese Feststellung mit den auf der Proteinevolution und Proteinfaltungskinetik beruhenden Kriterien kompensiert und komplementiert wird,

8) Identifizierung und Klassifizierung der Intermediate, wobei jedes Intermediat mit seinen festgestellten individuellen Charakteristiken, nämlich der hydrodynamischen Größe, dem Zeitpunkt seiner Entstehung, der Faltungswegezugehörigkeit und der quantifizierten Menge identifiziert wird und alle identifizierten und in unterschiedlichen Gruppen einzuordnende Intermediate in verschiedene Faltungswege klassifiziert werden, wobei auf die in bestimmte Faltungswege klassifizierten Intermediate noch drei Kriterien zutreffen können, nämlich Verfügung über gleiche Merkmale der Modifikation, allmähliche Verkleinerung ihrer gruppenzugehörigen hydrodynamischen Größe und dem kontinuierlichen Zeitablauf jedes der um den Faltungsweg gruppierten Intermediate,

9) Charakterisierung des Faltungsvorgangs, wobei der Weg der dominanten nativen Faltung, der Weg der schnellsten und langsamsten Faltung, der Weg zur Missfaltung, die Faltungskinetik in allen Wegeabläufen, die Reihenfolge der Disulfidbildung und/oder Crosslinkerbildung, die Entstehung der Knotenbildung und ihre Wirkung auf die Proteinfaltung, die intramolekularen Umlagerungen inklusive Disulfidumlagerung, die Faltungswege-Abzweigung, -Erweiterung, -Kreuzung, -Überquerung und - Zusammentreffen mit dabei betroffenen Intermediaten usw. durch die parallele Darstellung aller in unterschiedlichen Faltungswegen zugeordneten Intermediate jeweils mit ihren identifizierten Charakteristiken in einem multidimensionalen Koordinatensystem direkt ermittelt werden und der Faltungsvorgang des Proteins dadurch charakterisiert wird,

10) Multidimensionale Visualisierung des Faltungsvorgangs des Proteins, wobei der charakterisierte Faltungsvorgang durch die mit 4 Charakteristiken definierten Ordinaten mithilfe der Computergraphik in einem multidimensionalen Koordinatensystem in ihrer Vielfalt visualisiert und zur Animation gebracht wird und durch die Kombination und die Transformierung dieser 4 Charakteristiken ihre vielfältige Visualisierung weitergehend erweitert wird.

**2.** Verfahren nach Anspruch 1, **dadurch gekennzeichnet, dass** der Schritt 3) durchgeführt wird in mindestens einer der folgenden Ausführungsformen,

- die dynamische Modifizierung der disulfidhaltigen Proteine,
- die dynamische Modifizierung der disulfidfreien Proteine,

- die dynamische Modifizierung der Multidomänenproteine,
- die simuliert dynamische *in vitro* post- und cotranslationale Modifikation,
- die dynamische Modifizierung bei simulierter *in vitro* Proteinfaltung,
- die dynamische Modifizierung bei *in vitro* Proteinbiosynthese,

und mit mindestens einer der folgenden Vorgehensweisen,

- die Singlemodfizierung, wobei die Reste einzelner Aminosäurearten mit einem einzigen Seitenkettenreagenz bei optimierten Reaktionsbedingungen markiert werden,
- die Multimodifizierung, wobei die Reste von mehr als eine Art der Aminosäure mit einer einzigen Art oder mehr als einer Art von Seitenkettenreagenz durch Veränderung der Reaktionsbedingungen markiert werden oder die Markierung in einem einzelnen oder in mehreren Ansätzen zuerst separat durchgeführt dann zusammen gemischt werden,
- die interne Crosslinker-Modifizierung, wobei die interne Doppel-Modifizierung zwischen zwei Aminosäuren innerhalb eines Proteins mit den bifunktionellen Reagenzien bei regulierten Reaktionsbedingungen in variierten Ausführungsformen durchgeführt werden,

und mit mindestens einer der folgenden Reaktionskinetiken,

- Zeitskala zwischen Nano-, Mikro- bis Millisekunden für die sehr schnelle Proteinfaltung mit einer Zeitskala von Milli- bis Sekunden zur Bildung von Wasserstoff-Brücken, der Ausbildung der sekundären Strukturelemente und des hydrophoben Kollapses der Polypeptidkette,
- Zeitskala zwischen Mikrosekunden bis einer Minute für die schnelle Proteinfaltung mit einer Zeitskala von Mikrosekunden bis Minuten, die schnelle Faltungsphase und die differenzierende Entstehung der wegezugehörigen Intermediate betreffend,
- Zeitskala zwischen Millisekunden bis Minuten für die langsame Proteinfaltung mit einer Zeitskala von Minuten bis Stunden oder Tage, die Entwicklung der Intermediate, die Entstehung der Strukturen des "Molten Globules" und die weitere Faltung bis in den nativen Zustand betreffend,

und mit mindestens einer der folgenden Chemikalien,

- Denaturierungsmittel der Proteine,
- Reduzierungsmittel für die disulfidhaltigen Proteine,
- Reoxidationsmittel inklusive ihrer variierenden Komponenten und Zusammensetzungen, spezifisch für die Modifizierung der disulfidhaltigen Proteine,
- seitenkettenspezifische Reagenzien ohne und/oder mit Isotopenmarkierung,
- seitenkettenspezifische Reagenzien mit den fluoreszenz- oder/und spinmarkierten (spin label) Reportgruppen z.B. Reagenzien für DIGE (difference gel electrophoresis),
- seitenkettenspezifische nulllänge-, homo- und heterobifunktionelle Reagenzien zur internen Crosslinker-Markierung einschließlich der Reagenzien zur Photomarkierung,
- Biotinylierungsreagenzien,
- Reagenzien und/oder Enzyme für co- und posttranslationale Modifizierungen,
- Zellextrakten,
- Foldasen oder/und Chaperonen,
- Kandidaten-Inhibitoren der Foldasen oder/und Chaperonen,
- chemischen und biologischen Kandidaten-Wirkstoffen für Proteinfaltung und -abbau ,
- chemischen und biologischen Kandidaten-Wirkstoffen für in vitro cotranslationale Modifizierung,
- chemischen und biologischen Kandidaten-Wirkstoffen für in vitro posttranslationale Modifizierung,
- chemischen und biologischen Kandidaten-Wirkstoffen für in vitro Biosynthese,
- Hilfs- und Stabilisierungssubstanzen der Rückfaltung des Proteins.

3. Verfahren nach Anspruch 1, **dadurch gekennzeichnet, dass** der Schritt 4) entweder manuell oder nach der Konzeption und dem Entwurf der Erfindung automatisch und miniaturisiert durchgeführt wird und in mindestens einer der folgenden Durchführungsformen ausgeführt wird,

- die der Elektrophorese zugrunde liegende Durchführung bevorzugt mit der in folgender Weise modifizierten, auf die globalen und hydrophilen Proteine ausgerichteten Polyacrylamidgelelektrophorese mit mindestens einer der folgenden Verbesserungen,

- der Anschluss eines Zuführsystems der Pufferlösung für die während der Elektrophorese erfüllbare Regulierung der Pufferstärke, -zusammensetzung und des -pH-Werts, entweder durch Einbetten eines durchlässigen Behälters, der mit der gewünschten Pufferlösung gefüllt ist und sich im Pufferreservoir der Kathode befindet oder durch einen Puffer-Verteiler, der sich im Pufferreservoir der Kathode befindet und durch einen dünnen Schlauch mit dem äußeren Gefäß verbunden ist, um die gewünschte Pufferlösung nach gewünschtem Tempo kontinuierlich oder diskontinuierlich und dosierend zuzuführen,

- die dynamische Steuerung und Optimierung der Trennauflösung durch die während der Elektrophorese auf die Einstellungen der Pufferstärke, -zusammensetzung und des -pH-Werts zurückgeführte Erhöhung der dynamischen Differenzen der Ladungs- und Polaritätsverteilung der Intermediate eines Proteins und ihrer differenzierten Wechselwirkungen mit zusätzlichem Ionenstrom,

- die Anwendung der gepulst betriebenen Elektrophorese durch kurze Erhöhung der Spannung, kurze Veränderung der Polarität der Pufferbestandteile und/oder ihrer Konzentration und kurzes Umpolen kombiniert mit den stark hydrophoben Gegenionen zur Fokussierung der einzelnen Banden und Vergrößerung der Entfernung der Intermediate-Gelbanden,

- der Einsatz variabler Gelzusammensetzungen und -formen,

- das Unterdrücken der von Wärmeeffekten verursachten Diffusion der Intermediate zwischen den Gelbanden durch den Einbau einer aus den Peltier-Kühlplatten und einer zirkulierenden Flüssigkühlung bestehenden Vorrichtung zum gleichzeitigen Wärmeabtransport aus dem Gel und der Wärmeableitung ins eishaltige Abkühlreservoir,

- die Flüssigchromatographie, Elektrochromatographie und Feldflussfraktionierung in spektrometrischer Kopplung vorzugsweise der DLS und der Statischen Lichtstreuung (SLS) für die Auftrennung der Intermediate aller Proteine und Differenzierung ihrer hydrodynamischen Größen, vorwiegend für die Auftrennung und Größen-Differenzierung der Intermediate der nicht für die Elektrophorese-Methoden geeigneten stark sauren, stark basischen, hydrophoben, Membranproteine und großen Proteine, jeweils in Mikro-, Analytik- und Semipräparativ-Skala und mit folgenden Vorgehensweisen,

- die Monosäulenflüssigchromatographie durch bevorzugten Einsatz der jeweils nach Wunsch oder Bedarf mit unterschiedlichen Trennträgern eingefüllten einzelnen Mikrogelfiltrationssaulen oder Mikrofeldflussfraktionierungskanälen in Kopplung mit spektrometrischer Überprüfung zur direkten Ermittlung der Reihenfolge der hydrodynamischen Größen der dabei jeweils in einzelnem Eluat aufgetrennten Intermediate,

- die Multisäulenflüssigchromatographie in Kopplung mit spektrometrischen Messungen, die je nach Wunsch oder Bedarf in Serie und/oder parallel angeschlossenen Mikrosäulenkombinationen aus Gelfiltrations-, Hydroxyapatit-, Hydrophober, Ionenaustausch-, Reversedphase- und Affinitätschromatographie, einschließlich des Mikrofeldflussfraktionierungskanals für die spezifische Trennung und Größen-Differenzierung der Intermediate, die vor allem mit sehr ähnlichen hydrodynamischen Größen, aber jeweils entweder den gleichen oder unterschiedlichen Faltungswegen angehörig sind,

- die gekoppelte spektrometrische Differenzierung der hydrodynamischen Größen der aufgetrennten und jeweils in einzelnen Gefäßen oder in Mikrotiterplatten aufgefangenen Intermediate und ihre Quantifizierung, bevorzugt mit DLS (Dynamische Lichtstreuung) und SLS (Statische Lichtstreuung) sowie ergänzend mit Fluoreszenz, UV, CD, NMR, Fourier-Transformation und ESR,

- die spezifische Differenzierung der in einer einzelnen Probe enthaltenen Intermediate, die entweder in unterschiedlichen hydrodynamischen Größen verteilt vorliegen oder mit sehr ähnlichen hydrodynamischen Größen vorliegen und womöglich zu unterschiedlichen Faltungswegen gehören, mittels in folgender Weise angepassten DLS- und SLS-Geräten, die mindestens eine der folgenden zusätzlichen Funktionen beinhalten,

- Erhöhung der Differenzierungsauflösung durch verlängerte Messungszeit,

- Tm (Schmelzpunkt)-Differenzierung durch die allmählichen, vom Programm gesteuerten Temperaturerhöhungen,

- Konzentrationsänderung der Proben in einzelnen Mikrogefäßen oder in Mikrotiterplatten durch Verdünnung oder durch Aufkonzentrierung, die durch die eingebaute Vakuumverdunstung oder Belüftung erfüllt wird,

- Änderung des pH-Profils und Puffersystems durch Mikroautotitration oder manuelle Pipettierung gewünschter Pufferzusammensetzung,

- Bestimmung und Bewertung intrinsischer Viskosität und des Zeta-Potentials der Proben zur weitergehenden strukturellen Differenzierung der Intermediate,

- Veränderung der biorheologischen Eigenschaften der Proben durch Energiezufuhr und -abfuhr in Form von Bestrahlung, Erwärmung und Abkühlung, Ultraschall, Mikrowelle, elektrischem Feld und Magnetfeld,

- Bewertung und Anordnung der damit differenzierten Intermediate durch speziell dafür entwickelte Software,

- die der Kapillarelektrophorese zugrunde liegende Durchführung in Kopplung der Spektrometrie vorzugsweise mit DLS und SLS für die online automatische und miniaturisierte Auftrennung der Intermediate und Differenzierung ihrer hydrodynamischen Größen.

4. Verfahren nach Anspruch 1 bis 3, **dadurch gekennzeichnet, dass** der Faltungsvorgang durch Festlegung der 3-dimensionalen Strukturen aller oder der signifikanten Intermediate mit einer zusätzlichen 5ten Charakteristik, nämlich der Struktur des Intermediats charakterisiert wird und dabei in einem multidimensionalen Koordinatensystem mit dieser integrierten 5ten Ordinate dargestellt und in ihrer Vielfalt visualisiert wird.

5. Verwendung des Verfahrens nach den Ansprüchen 1 bis 4, **gekennzeichnet durch** die Untersuchung, Überprüfung und Bewertung der Aktivitäts- und Funktionalitätsveränderungen eines in unterschiedlichen Molekularumgebungen rückfaltenden Proteins gegenüber seines Substrates zur Verbesserung oder Optimierung der biotechnologischen Herstellung eines therapeutischen Proteins.

6. Verwendung des Verfahrens nach den Ansprüchen 1 bis 4, **gekennzeichnet durch** den Einsatz des Verfahrens im Protein-Engineering, wobei das den Erkenntnissen der erfindungsgemäß charakterisierten Faltungsvorgänge zugrunde liegende Protein-Engineering und die darauf zurückgeführten Veränderungen der Funktionalität und Aktivität eines Proteins überprüft, untersucht und bewertet werden.

7. Verwendung des Verfahrens nach den Ansprüchen 1 bis 4, **gekennzeichnet durch** die dynamische Charakterisierung und Quantifizierung der Vorgänge der simulierten *in vitro* Biosynthese und womöglich vorkommenden co- und posttranslationalen Modifizierung zur Bedingungsoptimierung der biotechnologischen Herstellung der *in vitro* posttranslational modifizierten Proteintherapeutika und Scanning der chemischen und biologischen Beihilfe- oder Hemmstoffe der co- und/oder posttranslationalen Modifizierungen, wobei die Charakterisierung der Vorgänge der simuliert dynamisch *in vitro* co- und posttranslationalen Modifikationen definiert durchgeführt und den entsprechenden Vergleichen und Auswertungen unterzogen wird.

8. Verwendung des Verfahrens nach den Ansprüchen 1 bis 4, **gekennzeichnet durch** die dynamische Charakterisierung des Rückfaltungsvorgangs der Proteine während ihrer *in vitro* simulierten bekannten *in vivo* posttranslationalen Modifizierungen zur Verfahrensentwicklung ihrer biotechnologischen Herstellung, wobei die Charakterisierung dieser Modifizierungen den definierten Schritten folgt und die Technologie der Proteinimmobilisierung und Proteinchipherstellung eingesetzt wird.

9. Verwendung des Verfahrens nach den Ansprüchen 1 bis 4, **gekennzeichnet durch** die Suche nach den biologischen oder chemischen Substanzen zur Beeinflussung der Proteinfaltung, wobei die ausgewählten biologischen oder/und chemischen Kandidaten-Hemmstoffe oder/und - Hilfsstoffe bei der Charakterisierung der Rückfaltuhg des zu untersuchenden Proteins einbezogen werden.

10. Verwendung des Verfahrens nach den Ansprüchen 1 bis 4, **gekennzeichnet durch** die Untersuchung der Wirkung der Foldasen oder Chaperone auf die Proteinfaltung, wobei die Wirkung **durch** den Vergleich der charakterisierten Faltungsvorgänge mit den und ohne die zu untersuchenden Foldasen oder Chaperone definiert wird.

11. Verwendung des Verfahrens nach den Ansprüchen 1 bis 4, **gekennzeichnet durch** die Suche nach biologischen und chemischen Hemmstoffen von Foldasen, Chaperonen und Wirkstoffen des Proteinabbaus, wobei das zu untersuchende Protein in den parallelen Ansätzen jeweils vorgegebener Foldasen, Chaperone und Wirkstoffe des Proteinabbaus zuerst ohne und dann mit den jeweiligen Kandidaten-Inhibitoren den Charakterisierungen, den Vergleichen und den Auswertungen dieser Faltungsvorgänge unterzogen wird.

12. Verwendung des Verfahrens nach den Ansprüchen 1 bis 4, **gekennzeichnet durch** die Untersuchung kontrollierter Selbstassemblierung und Polymerisation der Polypeptide oder Proteine bei ihrer Rückfaltung zur Entwicklung und Herstellung von Nano-Proteinmaterialien, wobei die anfänglichen Vorgänge der Selbstassemblierung und Polymerisation unter biologischen oder/und chemischen Einflussfaktoren in variierenden physiologischen und biochemischen Bedingungen charakterisiert und ausgewertet werden.

**13.** Verwendung des Verfahrens nach den Ansprüchen 1 bis 4, **gekennzeichnet durch** die Suche nach pharmakologischen Chaperonen gegen **durch** Proteine verursachte Erkrankungen (Proteopathien), wobei die Wirkung bestimmter biologischer oder/und chemischer Faltungsstabilisierungssubstanzen, die spezifisch an ungefaltete Proteine binden, die die Faltung verbessern und die Proteinstruktur stabilisieren, oder die hydrophoben Domänen fehlgefalteter Proteine maskieren und die Löslichkeit erhöhen und damit die Aggregation von ungefalteten Proteinen verhindern, auf Rücktaltung der proteopathischen Proteine **durch** die Charakterisierung festgestellt wird.

**14.** Verwendung des Verfahrens nach den Ansprüchen 1 bis 4, **gekennzeichnet durch** die Charakterisierung des Vorgangs zum einen der Umfaltung des PrP$^c$ ( Prion Protein cellular) zu PrP$^{Sc}$ (Prion Protein Scrapie; pathogene Form des Prion-Proteins) einschließlich erfolgter Aggregation und zum anderen der Umkehrung des PrP$^{Sc}$ zu PrP$^c$, einschließlich des Abbaus der Aggregation zur Klärung der Pathogenese, zur Suche nach Therapieoptionen und Möglichkeiten zur Prävention, wobei die Faltungsvorgänge eines Prion-Proteins in parallelen Ansätzen mit biologischen oder/und chemischen Faltungseinfluss-Substanzen unter Regulierung der destabilisierenden Bedingungen charakterisiert und mit dem zuvor charakterisierten Faltungsvorgang, ohne Faltungseinfluss-Substanzen verglichen werden.

**15.** Verwendung des Verfahrens nach den Ansprüchen 1 bis 4, **gekennzeichnet durch** die dynamische Charakterisierung des Faltungsvorgangs eines zu untersuchenden Proteins für die Untersuchung der auf Isomerisierung, Desamidierung und Racemisierung zurückgeführten Proteinalterung und des von Radikaleinwirkung, oxidativem Stress und Umwelteinflüssen verursachten Proteinverschleißes, wobei das zu untersuchende Protein bei seiner Rücktaltung entweder einer katalytisch beschleunigten Isomerisierung, Deamidierung und Racemisierung durch Zufuhr photochemischer und thermischer Energie oder einer von Radikaleinwirkung, oxidativem Stress und Umwelteinflüssen initiierten Modifikation unterzogen und sein **dadurch** veränderter Faltungsvorgang weitergehend charakterisiert und mit dem zuvor ohne Einflussfaktoren charakterisierten Vorgang verglichen wird.

**16.** Verwendung des Verfahrens nach den Ansprüchen 1 bis 4, **gekennzeichnet durch** die Klassifizierung (Taxonomie) der Proteine und Erforschung der Proteinevolution auf neuer Ebene der Proteinfaltung, wobei der charakterisierte Vorgang der Faltung als individueller Fingerabdruck jedes Proteins die funktionellen und evolutionären Zusammenhänge der Proteine liefert und als zusätzliches Kriterium in die bisher nur auf Struktur, Topologie, Homologie und evolutionäre Verwandtschaft basierenden Klassifizierung der Proteine integriert wird.

**17.** Verwendung des Verfahrens nach den Ansprüchen 1 bis 4, **gekennzeichnet durch** die dynamische Charakterisierung des Faltungsvorgangs einer Komplexbildung der Nukleo-, Glyko- und Lipoproteine zur Untersuchung des Entstehungsvorgangs und der damit begleiteten Aktivitäts- und Funktionalitätsveränderungen und der Suche nach den auf die Komplexbildung wirkenden chemischen und biologischen Wirkstoffen, wobei die Faltungsvorgänge des betreffenden Proteins und dessen Komplex zuerst einzeln charakterisiert werden und das Protein und sein Komplexanteil dann während ihrer Rückfaltung mit zeitlichen Abständen in nacheinander folgenden Ansätzen zur Komplexbildung zusammen gebracht und den Charakterisierungen, Vergleichen und Auswertungen ohne und mit Zufuhr dieses Protein-Komplexes unter variierenden chemischen und biologischen Einflussfaktoren wiederholt unterzogen werden.

**18.** Verwendung des Verfahrens nach den Ansprüchen 1 bis 4, **gekennzeichnet durch** die Diagnostik und Prognose der von Proteinmissfaltung verursachten Erkrankungen, wobei die Veränderungen des Faltungsvorgangs der krankheitsbezogenen Proteine charakterisiert und als Kriterien für Diagnostik und Prognosen bestimmter Erkrankungen definiert werden.

**19.** Verwendung des Verfahrens nach den Ansprüchen 1 bis 4, **gekennzeichnet durch** die dynamische Charakterisierung des anfänglichen Vorgangs der Aggregation der selben Proteine oder verschiedener Proteine während ihrer Rückfaltung zwischen den faltenden Proteinen oder zwischen den faltenden und nativen Proteinen zur Aufklärung des Mechanismus der Proteinaggregation und zur Suche nach ihren chemischen und biologischen Hemmstoffen, wobei die Faltungsvorgänge des zu untersuchenden Proteins zuerst ohne und dann unter dem Einfluss von anderen Proteinen und/oder chemischen und biologischen Hemmstoffen in unterschiedlichen molekularen Umgebungsbedingungen jeweils charakterisiert, verglichen und ausgewertet werden.

**20.** Verwendung des Verfahrens nach den Ansprüchen 1 bis 4, **gekennzeichnet durch** die dynamische Charakterisierung des Faltungsvorgangs in der Interaktion zwischen den rückfaltenden Proteinen oder zwischen den rückfaltenden und nativen Proteinen für die Untersuchung des Konformationsverhaltens und der Katalyseeigenschaften eines spezifischen Proteins auf der Ebene seiner Rückfaltung zur Suche nach therapeutischen Targetproteinen, zur Ermöglichung des rationalen Designs von Wirkstoffen und zur Optimierung biotechnologischer Prozesse, wobei die konzipierten Faltungsvorgänge des untersuchten Proteins zuerst ohne und dann unter dem Einfluss von anderen Proteinen in optimierter Molekularumgebung jeweils charakterisiert, verglichen und ausgewertet werden.

21. Verwendung des Verfahrens nach den Ansprüchen 1 bis 4, **gekennzeichnet durch** die dynamische Charakterisierung des Vorgangs der Mtigen-Antikörper-Reaktion und des Abbauprozesses des Antigen-Antikörper-Komplexes für die Optimierung und Rationalisierung des Antikörper-Engineerings, wobei die neu konstruierten Antikörper und Antigene den Charakterisierungen, Vergleichen und Auswertungen zunächst einzeln, dann zusammen unter optimierten physiologischen und biochemischen Molekularumgebungen ohne und/oder mit den chemischen und biologischen Einflussfaktoren für die Untersuchung der Selektivität, Spezifität, Affinität, Faltungseffizienz, thermodynamischen Stabilität, pharmakologischen Kinetik und biotechnologischen Produktivität des redesignten Antikörpers und der Antigenität des Antigens unterzogen werden.

22. Verwendung des Verfahrens nach den Ansprüchen 1 bis 4, **gekennzeichnet durch** die *in vitro* Charakterisierung des Faltungsvorgangs eines biosynthetisierenden und womöglich cotranslational modifizierenden Proteins zur Aufklärung der initiierten Faltung des Proteins, während seiner nascenten Biosynthese und Suche nach Substanzen mit Einfluss auf die initiierte Faltung des Proteins, wobei die in den zellfreien Ansätzen in unterschiedlichen Längen synthetisierten Polypeptid-Fragmente eines Proteins, die unter optimierten biologischen und physikochemischen Einflussfaktoren **durch** regulierte Zufuhr der notwendigen Aminosäuren mit oder ohne Isotopenmarkierung gewonnen und als Mini-Intermediate bezeichnet werden, zuerst zusammen gesammelt und dann gezielt chromatographisch aufgetrennt und anschließend den Charakterisierungen und dem Vergleich ihrer Faltungsvorgänge unterzogen werden.

23. Verwendung von Mitteln zur Durchführung des Verfahrens nach dem Anspruch 1, **gekennzeichnet durch** Kits, Geräte und Software, sowie konzipierte und entworfene Automaten, die jeweils für die manuelle oder automatisierte Durchführung des Verfahrens gebraucht werden.

## Claims

1. Method for characterizing and multidimensional presentation of the folding process of proteins, **characterized in that** all modified and structurally isolated intermediates of a protein in certain time intervals during the refolding process at least according to 4 of their own special characteristics, namely the hydrodynamic size, the time of formation, the folding pathway identity and amount of the intermediate are identified and digitized, that a folding process consists of 4 phases and that a method is available for the characterization of the folding process of all proteins which comprises the following steps:

    1) analysis of the protein to be characterized on the basis of its primary, secondary, and, where appropriate, three-dimensional structure, of the biological and physicochemical properties and the objective, whereby a theoretical mass fragments detection pattern is created and the appropriate procedure and embodiment are defined,
    2) separation of the optimally unfolded portion of the protein with the maximized hydrodynamic parameters, whereby the protein is denatured, if appropriate, reduced, separated from the reduction agent, separated by chromatography and spectrometrically differentiated,
    3) dynamic modification of the refolding and, if appropriate. also reoxidizing protein, whereby small portions with all intermediates are removed from the reaction chamber within defined periods of time, and according to the accessibility of their amino acids, are modified with appropriate side chain reagents in different extents and the varied and structurally relatively stable intermediates are classified each on its own special characteristics, namely by the hydrodynamic size, the time of formation, the folding pathways identity and quantified amount.
    4) separation and quantification of the intermediates and presentation of their hydrodynamic sizes and, if necessary, their quantities as a function of their time of appearance, whereby the modified intermediates during the refolding either by electrophoresis or by chromatography preferably in combination with the spectrometric measurements preferably by dynamic light scattering after the period of time of their refolding and their hydrodynamic parameters during the separation process are quantified, multidimensionally presented, digitized and made available for the next step (fig. 3), whereby a refolding fingerprint profile is created,
    5) fragmentation of the intermediates, whereby the intermediates, modified in time intervals, separated and differentiated according their hydrodynamic sizes, are preferably cleaved with trypsin and, if necessary, additionally proteolytic with endoproteases Lys-C, Glu-C and Asp-N and Asp-N as well as selected exoproteases and if necessary additionally chemically to differentiate the fragments,

6) detection of the fragmented intermediates, whereby molecular weights of the individual fragments and also the big fragments, which are connected by disulfide bridges or crosslinkers were determined by mass spectroscopy and registered in a data base and are arranged in a mass fragments detection table in order to identify all intermediates recorded in a database and are placed in a mass fragments detection table to identify. Whereby MALDI-TOF-MS/MS (tandem mass spectrometry), and / or MALDI-TOF-MS-PSD (post-source decay) are used for the precise differentiation of similar fragments,

7) determination of intermediates folding pathway identity, whereby the type, the number and the location of the modification performed as own individual characteristics for each intermediate are determined by comparison of mass spectrometrically detected number and mass of the fragments with the theoretical fragments mass detection patterns and the intermediates with common characteristics can be assigned to special groups and from the features of the grouped intermediates can be concluded that specific folding pathways occur, whereby this finding is compensated and complemented by the criteria based on the protein evolution and protein folding kinetics

8) identification and classification of the intermediates, whereby each intermediate is identified with its determined and individual characteristics, namely the hydrodynamic size, the time of its appearance, the folding pathway identity as well as the quantified amount and all intermediates are identified and arranged in different groups and classified into different folding pathways, whereby three criteria can be applied to the classified intermediates in definite folding pathways, namely the same features of the modification, gradual decreasing of their group specific hydrodynamic size and the continuous passage of time of each intermediate with the folding pathway.

9) characterization of the folding process, whereby the path of the dominant native folding, the path of the fastest and slowest folding, the path of misfolding, the folding kinetics in all paths, the order of disulfide formation and / or cross-linking formation, the formation of nodules and their effect on protein folding, the intramolecular rearrangements including disulfide arrangements, the branches of folding-pathways, extension, intersection, crossing and coincidence with the concerned intermediates, etc. by parallel presentation of all intermediates assigned to different folding pathways intermediate each with their identified characteristics in a multi-dimensional coordinate system are determined directly, and therefore the folding process of the protein is characterized.

10) multi-dimensional visualization of the folding process of the protein, whereby the characterized folding process is visualized by the ordinates, which are defined by the four characteristics, using the computer graphics in a multi-dimensional coordinate system in all its vriety and is brought to animation and is extended by the combination and transformation of these four characteristics of their varied visualization.

2. A method according to claim 1, characterized that step 3) is carried out in at least one of the following embodiments,

- the dynamic modification of the disulfide-containing proteins,
- the dynamic modification of the disulfide-free proteins,
- the dynamic modification of the multi-domain proteins,
- the simulated dynamic in vitro post-and co-translational modification,
- the dynamic modification under simulated in vitro protein folding,
- the dynamic modification in vitro protein biosynthesis,
- and at least one of the following procedures,
- single modification, wherein the radicals of individual amino acids are marked with a single side chain agent at optimized reaction conditions,
- multi-modification, wherein the radicals of more than one amino acid with a single type of or more than one type of side chain agents are marked by changing the reaction conditions, or the marking is performed in a single or in several batches by at first separately then by mixing together,
- internal cross-linker modification, wherein the internal double modification between two amino acids within one protein with bifunctional reagents are carried out at controlled reaction conditions in varied embodiments and at least with one of the following reaction kinetics,
- time scale from nano-, micro- up to milliseconds for the very fast protein folding, with a time scale from milliseconds up to seconds for the formation of hydrogen bonds, the formation of secondary structure elements and the hydrophobic collapse of the polypeptide chain,
- time scale from microseconds up to a minute for fast protein folding phase with a time scale from microseconds up to minutes, concerning the fast folding phase and differentiating development of intermediates associated with the pathways,
- time scale of milliseconds to minutes for the slow protein folding with a time scale from minutes up to hours or days, concerning the development of the intermediates, the formation of the structures of the "Molten Globules" and ongoing folding into the native state,

and at least with one of the following chemicals,

- denaturing agents for the proteins,
- reducing agents for the disulfide containing proteins,
- reoxidants including their varying components and compositions specific for the modification of the disulfide containing proteins,
- side chain-specific reagents without and / or with isotopic labeling,
- side chain-specific reagents with the fluorescence-and / or spin-labeled report groups e.g. agents for DIGE (Difference gel electrophoresis),
- side chain-specific zero-length, homo-and hetero-bifunctional reagents for internal cross-linker-marking including reagents for photolabeling,
- biotinylation reagents,
- reagents and / or enzymes for co- and posttranslational modifications,
- cell extractions,
- foldases and / or chaperones,
- candidate inhibitors of foldases and / or chaperones,
- chemical and biological candidate agents for protein folding and degradation,
- chemical and biological candidate drugs for cotranslational modification in vitro,
- chemical and biological candidate drugs for posttranslational modification in vitro,
- chemical and biological candidate agents for biosynthesis in vitro,
- supporting and stabilizing substances for the refolding of the protein.

3. A method according to claim 1, **characterized in that** the step 4 is carried out either manually or automatically according to the design and the draft of the invention and at least in one of the following implementations,

- the performance based on the electrophoresis, preferable on the modified polyacrylamide gel optimized for the global and hydrophilic proteins, with at least one of the following improvements,

- the connection of a feed system for the buffer solution to control and adjust the buffer strength and the pH value during the electrophoresis, either by embedding a permeable container, which is filled with the desired buffer solution and which is located in the buffer reservoir of the cathode or by a buffer distributor, which is located in the buffer reservoir of the cathode and is connected by a thin tube with the outer vessel, in order to supply the desired buffer solution in the desired speed continuously or discontinuously,
- the dynamic control and optimization of the separation resolution by adjustment of the buffer strength, buffer composition and the pH value during the electrophoresis increase the dynamic differences of the charge and polarity distribution of the intermediates of a protein and its differential interactions with additional ion current,
- the application of pulsed electrophoresis by short increase in voltage, short changing of the polarity of the buffer components and / or of their concentration and short polarity reversal combined with the highly hydrophobic counter ions for focusing the single bands and increasing the distances between the gel bands of the intermediates,
- the use of variable gel compositions and shapes,
- suppressing the diffusion of the intermediates between the gel bands caused by thermal effects by installing a device consisting of the Peltier cooling plates and a circulating liquid cooling for simultaneous heat removal from the gel and the dissipation of heat in the ice containing cooling reservoir,

- spectrometric coupling of liquid chromatography, electro chromatography and field-flow fractioning preferably with DLS and static light scattering (SLS) for the separation of the intermediates of all proteins and differentiation of their hydrodynamic sizes, mainly for the separation and sizes differentiation of the intermediates, which are not suitable for the electrophoresis methods like strongly acidic, strongly basic, hydrophobic proteins, membrane proteins or large proteins, each on the micro, analytical- and semi-preparative scale, and with the following procedures,
- the monocolumn liquid chromatography filled with the respectively desired or needed different separation media in various microgel filtration columns or micro field flow fractionation channels coupled with direct spectrometric control for direct determination of the sequence of the hydrodynamic sizes of the intermediates separated in the single eluate,
- the multicolumn liquid chromatography coupled with spectrometric measurements, which are connected, as desired or required, in series and / or parallel with combinations of micro columns of gel filtration-, hydroxyapatite-, hydrophobic-, ion exchange-, reversed phase- and affinity-chromatography, including the multicolumn liquid chromatography for specific separation and size differentiation of the intermediates, which show mainly very similar hydrodynamic sizes, but belong either to the same or to different folding pathways

- the coupled spectrometric differentiation of the hydrodynamic sizes of the intermediates separated and collected each in separate vessels or in microtiter plates and their quantification, preferably with DLS (dynamic light scattering) and SLS (static light scattering), complemented with fluorescence, UV, CD, NMR, Fourier Transformation Infrared and ESR,

- the specific differentiation of intermediates in a single sample that are present either with different hydrodynamic sizes or are present with very similar hydrodynamic sizes and possibly belong to different folding pathways, by adjusted DLS and SLS devices that at least include one of the following additional features,

- Increasing the resolution differentiation by extended measurement time,
- Tm (melting point) differentiation by gradual increases in temperature controlled by program,
- change in the concentration of the samples in individual micro-vessels or in microtiter plates by dilution or by concentration, which is performed by the installed vacuum evaporation or ventilation,
- change in the pH profiles and buffer systems by microautotitration or manual pipetting the desired buffer composition,
- determination and evaluation of the intrinsic viscosity and the zeta potential of the samples for further structural differentiation of the intermediates,
- change in the biorheological properties of the samples by energy input and dissipation in the form of radiation, heating and cooling, ultrasound, microwaves, electric field and magnetic field,
- review and arrangement of the so differentiated intermediates by a specially developed software,

- coupling capillary electrophoresis with spectrometry preferably with DLS and SLS for online automatic and miniaturized separation of intermediates and differentiation of their hydrodynamic sizes.

4. A method according to claims 1 to 3, **characterized in that** the folding process is specified by fixing the three-dimensional structures of all or of the significant intermediates with an additional 5th characteristic. The structure of the intermediate is depicted in a multi-dimensional coordinate system with the integrated 5th ordinate and is therefore visualized in its diversity.

5. Application of the method according to claims 1 to 4, **characterized in that** the analysis, review and evaluation of the changes in activity and functionality in a refolding protein in different molecular environments to its substrate to improvement or optimization of the biotechnological production of a therapeutic protein.

6. Application of the method according to claims 1 to 4, **characterized by** the use of the method in protein engineering, wherein the findings of the characterized folding processes based on the present invention and the underlying protein engineering and the changes in the functionality and activity of a protein due to it, are checked, examined and evaluated.

7. Application of the method according to claims 1 to 4, **characterized by** the dynamic characterization and quantification of the procedures of the simulated in vitro biosynthesis and possibly occurring co-and posttranslational modification to optimize the conditions of the biotechnological production of the in vitro posttranslationally modified protein therapeutics and scanning of the chemical and biological accelerators or inhibitors of the co-and / or post-translational modifications used, wherein the characterization of the processes of the simulated dynamic in vitro co- and post-translational modifications are made in a defined way and subjected to the corresponding comparisons and evaluations.

8. Application of the method according to claims 1 to 4, **characterized by** the dynamic characterization of the refolding process of the proteins during their simulated in vitro, known in vivo, posttranslational modifications for process development of their biotechnological production and the characterization of these modifications follows defined steps of the present invention and the technology of protein immobilization and protein chip fabrication is used.

9. Application of the method according to claims 1 to 4, **characterized by** the search for the biological or chemical substances to influence protein folding, wherein the selected biological and / or chemical candidate inhibitors and / or excipients to be included in the characterization of the refolding of the specific protein.

10. Application of the method according to claims 1 to 4, **characterized by** the study of the effect of the chaperones or foldases on the protein folding, wherein the effect is defined by comparing the characterized folding processes with and without the foldases and chaperons under investigation.

11. Application of the method according to claims 1 to 4, **characterized by** the search for biological and chemical inhibitors for foldases, chaperones and agents for protein degradation, wherein the protein to be examined in parallel tests with selected foldases, chaperones and agents for protein degradation, first with and then without the respective candidate inhibitor, is subjected to the characterizations, comparisons and evaluations of these folding processes.

12. Application of the method according to claims 1 to 4, **characterized by** examination of controlled self-assembly and polymerization of the polypeptides or proteins in its refolding process in order to develop and produce nano-protein materials, wherein the initial steps of the self-assembly and polymerization under biological and / or chemical factors are characterized and evaluated under varying physiological and biochemical conditions.

13. Application of the method according to claims 1 to 4, **characterized by** the search for pharmacological chaperones against deseases caused by proteins (proteopathies), whereby the effect of certain biological and / or chemical folding stabilizing substances, which specifically bind to unfolded proteins, that improve the folding and stabilize the protein structure or mask the hydrophobic domain of misfolded proteins and increase the solubility and thus prevent the aggregation of unfolded proteins, the refolding of proteopathic proteins is determined by the characterization.

14. Application of the method according to claims 1 to 4, **characterized by** the characterization of the process on the one hand of the refolding of the PrP$^c$ (Cellular Prion Protein) to PrP$^{Sc}$ (Scrapie Prion Protein, pathogenic form of the prion protein) including complete aggregation and on the other hand the reversal of the PrP$^{Sc}$ to PrP$^c$ including the degradation of the aggregation to clarify the pathogenesis, to search for therapy options and possibilities for prevention, whereby the folding processes of a prion protein are **characterized in** parallel tests with biological and / or chemical substances influencing the folding process substances under regulation of the destabilizing conditions and are compared with the previously characterized folding processes without substances influencing the folding process.

15. Application of the method according to claims 1 to 4, **characterized by** the dynamic characterization of the folding process of a targeted protein for the study of the protein aging traced back to isomerization, deamidation and racemization and of the protein aging caused by free radical exposure, oxidative stress and environmental factors, wherein the targeted protein at its refolding process is exposed either to a catalytically accelerated isomerization, deamidation and racemization applying photochemical and thermal energy or a modification initiated by free radical impact, oxidative stress and environmental factors and its resulting changed folding process is further characterized and compared with the previously characterized folding process without these impacts.

16. Application of the method according to claims 1 to 4, **characterized in that** the classification (taxonomy) of the proteins and exploration of the protein evolution on a new level of the protein folding, whereby the characterized process of folding as the individual fingerprint of each protein provides the functional and evolutionary relationship of the proteins and is integrated as additional criterion into the up to now only based on the structure, topology, homology and evolutionary relationship classification of proteins.

17. Application of the method according to claims 1 to 4, **characterized by** the dynamic characterization of the folding process of the complex formation of the nucleo-, glyco- and lipoproteins to examine the formation process and the related activity and functionality changes and search for chemical and biological agents influencing the folding processes, whereby the folding processes of the respective protein and its complex are at first characterized individually and then the protein and its complex part are brought together in time intervals during its refolding process in successive tests and are examined repeatedly by characterizations, comparisons and evaluations with and without addition of this protein complex under varying chemical and biological conditions.

18. Application of the method according to claims 1 to 4, **characterized in that** the diagnosis and prognosis of the diseases caused by protein misfolding, the changes in the folding process of the disease-related proteins can be characterized and defined as a criterion for the diagnosis and prediction of certain diseases.

19. Application of the method according to claims 1 to 4, **characterized by** the dynamic characterization of the initial process of the aggregation of the same proteins or different proteins during their refolding process between the folding proteins, or between the folding and native proteins to investigate the mechanism of protein aggregation, and to search for their chemical and biological inhibitors, whereby the folding operations of the protein to be examined are characterized, compared and evaluated each first without and then under the influence of other proteins and / or chemical and biological inhibitors in different molecular environments

20. Application of the method according to claims 1 to 4, **characterized by** the dynamic characterization of the folding process in the interactions between the refolding protein and between the refolding and native proteins for the investigation of the conformational behavior and the catalytic properties of a specific protein on the level of its refolding to search for therapeutic target proteins for enabling the rational design of drugs and for the optimization of biotechnological processes, whereby the designed folding processes of the examined protein are each characterized, compared and evaluated first without and then under the influence of other proteins in an optimized molecular environment.

21. Application of the method according to claims 1 to 4, **characterized by** the dynamic characterization of the process of the antigen-antibody reaction and the degradation process of the antigen-antibody complex for optimization and rationalization of the antibody engineering, wherein the newly engineered antibodies and antigens are characterized, compared and evaluated, at first isolated, then together under optimized physiological and biochemical molecular environments without and / or with the influence of chemical and biological factors to study of the selectivity, specificity, affinity, folding efficiency, thermodynamic stability, pharmacological kinetics and bio-productivity of the redesigned antibody and the antigenicity of the antigen.

22. Application of the method according to claims 1 to 4, **characterized by** the in vitro characterization of the folding process of a biosynthetising and possibly co-translationally modifying protein to investigate the initiated folding of the protein, while its nascent biosynthesis and search for substances influencing the initiated folding of the protein, wherein the synthesized polypeptide fragments of different length of a protein in the cell-free batches, which are obtained under optimized biological and physicochemical factors by the controlled supply with the essential amino acids, with or without isotope labeling and referred to as mini-intermediates are at first collected and then specifically separated by chromatography and then characterized and their folding processes compared.

23. Application of the method according to claim 1 **characterized by** kits, equipment and software, as well as newly designed automatic machines, which are necessary for manual or automatic performance of the procedure.

**Revendications**

1. Procédé pour caractériser et représenter le processus de repliement des protéines d'une manière multidimensionnelle, **caractérisé par le fait que** durant le processus de repliement tous les intermédiaires protéiques qui ont été auparavant modifiés et structurellement recueillis à des intervalles de temps donnés sont identifiés et digitalisés au moins selon leurs quatre caractéristiques individuelles à savoir la taille hydrodynamique, le moment de formation, le chemin de repliement suivi et la quantité à déterminer et **par le fait que** ce procédé de repliement est constitué de quatre phases et que le procédé pour la caractérisation du processus de repliement pour toutes les protéines est disponible et comprend les étapes suivantes:

1) Analyse de la protéine à caractériser sur la base de sa structure primaire, secondaire et éventuellement tridimensionnelle, ses propriétés biologiques et physicochimiques ainsi que l'objectif à atteindre tout en définissant un fragment théorique servant comme spécimen et en établissant un plan d'action approprié et un mode de réalisation convenable,
2) Séparation de la portion de la protéine dépliée d'une manière optimale avec des tailles hydrodynamiques maximisées tout en soumettant la protéine à une dénaturation, et, le cas échéant, à une réduction avec libération de l'agent réducteur, à une séparation chromatographique et à une différenciation spectrométrique,
3) Modification dynamique de la protéine qui se replie et éventuellement se réoxyde tout en prélevant périodiquement et par portions les intermédiaires souhaités selon l'accessibilité de leurs résidus d'acides aminés correspondants avec des réactifs des chaines latérales appropriés, en les modifiant en différentes dimensions et les classant aux divers et structurellement relativement stables intermédiaires avec leurs propres caractéristiques individuelles respectives à savoir la taille hydrodynamique, le moment de formation, la voie de repliement traversée et la quantité à mesurer,
4) Séparation et quantification des produits intermédiaires et représentation de leurs tailles hydrodynamiques et éventuellement leur quantité en fonction de leur moment de formation, au cours duquel les intermédiaires modifiés pendant le repliement sont séparés soit par électrophorèse ou chromatographie en couplage avec les mesures de spectrométrie de préférence par commande dynamique de la lumière selon leur temps de repliement et leurs tailles hydrodynamiques ensuite quantifiés, représentés d'une façon multidimensionnelle, digitalisés et enfin préparés pour l'étape suivante tout en établissant un profil d'empreinte du repliement,

5) Fragmentation des espèces intermédiaires, pendant lequel les intermédiaires modifiés périodiquement, séparés et différentiés selon leurs tailles thermodynamiques en ajoutant de préférence la trypsine et en additionnant éventuellement les endoprotéases Lys-C, Glu-C et Asp-N ainsi que des exoprotéases choisies et en complément ce clivage protéolytique en cas de besoin par un clivage chimique pour la différenciation des fragments,

6) Détection des intermédiaires fragmentés, pendant lequel les poids moléculaires de chaque fragment et ceux des grands fragments liés par des ponts disulfure et / ou par des agents de réticulation sont mesurés par spectrométrie de masse, enregistrés dans une banque de données et classés dans un tableau servant à reconnaître et à identifier tous intermédiaires. MALDI-TOF-MS/MS (spectrométrie de masse en tandem) et/ou MALDI-TOF-MS-PSD (Post-Source decay) seront employées à ce propos pour la différenciation précise des fragments similaires,

7) Détermination des voies de pliage des intermédiaires, pendant lequel le genre, le nombre et le lieu de la modification effectuée sont déterminés comme propres caractéristiques individuelles de chaque intermédiaire par comparaison du nombre et masse des fragments obtenus de la spectrométrie de masse avec le fragment modèle théorique servant à reconnaître la masse des fragments et les intermédiaires ayant des caractéristiques communes sont classés dans des groupes spécifiques et ces intermédiaires groupés permettent de conclure aux voies de pliement déterminées, pendant lequel cette constatation est compensée et complétée par les critères se basant sur l'évolution des protéines et leurs cinétiques de replieinent,

8) Identification et classification des intermédiaires, pendant lequel chaque intermédiaire est identifié selon ses propres caractéristiques particulières à savoir la taille hydrodynamique, le moment de sa formation, les voies de pliage et la quantité déterminée et tous les intermédiaires identifiés et associés aux différents groupes sont classés dans des chemins de repliement variés, pendant lequel trois autres critères peuvent s'appliquer sur les intermédiaires classés dans certains chemins de repliement à savoir la disposition sur des mêmes caractéristiques de modification, diminution progressive de leurs tailles hydrodynamiques associées au groupe et le parcours continu du temps de chaque intermédiaire groupé dans le chemin de repliement,

9) Caractérisation du processus de repliement pendant lequel le chemin de repliement natif dominant, la vitesse du chemin de repliement le plus rapide et le plus lent, le chemin du repliement incorrect, la cinétique du repliement dans tous les parcours de ce chemin, l'ordre de formation des ponts disulfures et/ou formation d'agents de réticulation, la formation de nodules et leur effet sur le repliement des protéines, les réarrangements intramoléculaires y compris le réarrangement des liaisons disulfures, le détournement, élargissement, croisement, traversée et rencontre avec les intermédiaires concernés etc. sont établis directement dans un système de coordonnées multidimensionnel et ceci par la représentation parallèle respective de tous les intermédiaires associés à différents chemins de repliement,

10) visualisation multidimensionnelle du processus de repliement de la protéine en visualisant et animant les ordonnées définis par quatre caractéristiques dans un système de coordonnées multidimensionnel et en visualisant cette diversité à l'aide de l'infographie et en élargissant cette visualisation en combinant et transformant ces quatre caractéristiques.

2. Procédé selon la revendication 1, **caractérisé en ce que** l'étape 3) est réalisée dans au moins l'un des modes de réalisation suivants,

- la modification dynamique des protéines contenant des ponts disulfure,
- la modification dynamique des protéines ne contenant pas des ponts disulfure,
- la modification dynamique des protéines multidomaines,
- la modification dynamique post- et cotraductionnelle simulée in vitro,
- la modification dynamique du repliement des protéines simulée in vitro,
- la modification dynamique de la biosynthèse des protéines in vitro,

et avec au moins une des procédures suivantes,

- la modification simple, où les radicaux de chaque type d'acides aminés sont marqués avec un seul réactif de chaines latérales dans des conditions optimales de réaction,
- la modification multiple, dans laquelle les radicaux de plus d'un type d'acides aminés sont marqués avec un seul type (ou plus) de réactif de chaines latéraux et ceci par changement des conditions de réaction ou ce marquage peut aussi être réalisé en une seule ou en plusieurs approches qui peuvent être d'abord effectuées séparément et ensuite mélangées ensemble,
- la modification interne de réticulation, pendant lequel la double modification interne entre deux acides aminés au sein d'une protéine avec les réactifs bifonctionnels est effectuée à des conditions réactionnelles régulées et dans des modes de réalisation variés,

et avec au moins une des cinétiques de réaction suivantes,

- échelle du temps entre nano-, micro-, jusqu'aux millisecondes pour le repliement des protéines le plus rapide et une échelle du temps de milli-, jusqu'aux secondes pour la formation des ponts hydrogène, la formation d'éléments de structure secondaires et le collapsus hydrophobe de la chaîne polypeptidique,
- échelle de temps de microsecondes à une minute pour le repliement rapide des protéines y compris la phase rapide de repliement et la création différentielle des intermédiaires associées à ce chemin,
- échelle de temps de quelques millisecondes à quelques minutes ou même jours pour le repliement lent des protéines, y compris le développement des intermédiaires, la formation des structures des globules fondus "Molten Globules" et un autre repliement jusqu'à l'état natif,

et avec au moins un des produits chimiques suivants,

- agents dénaturants des protéines,
- agents réducteurs pour les protéines disulfures,
- agents réoxydants y compris leurs variables composants et compositions qui sont spécifiques pour la modification des protéines contenant des ponts disulfures,
- réactifs spécifiques aux chaînes latérales sans et/ou avec marquage par des isotopes,
- réactifs spécifiques aux chaînes latérales et qui sont marqués par fluorescence ou/et par des spins, réactifs pour électrophorèse sur gel DIGE (différence gel electrophoresis) par exemple,
- homo-et hétéro-bifonctionnels réactifs ou à longueur nulle spécifiques pour les chaines latérales et utilisés pour le marquage interne des agents de réticulation y compris les réactifs pour le photomarquage,
- réactifs de Biotinylation,
- réactifs et/ou des enzymes pour les modifications co- et posttraductionnelles,
- les extraits de cellules,
- les foldases et/ou les chaperons,
- les inhibiteurs des foldases et/ou des chaperons,
- les substances actives de nature biologique et chimique candidates pour le repliement des protéines et de leur dégradation,
- les substances actives de nature biologique et chimique candidates pour la modification cotraductionnelle *in vitro,*
- les substances actives de nature biologique et chimique candidates pour la modification posttraductionnelle *in vitro,*
- les substances actives de nature biologique et chimique candidates pour la biosynthèse *in vitro,*
- les substances permettent à soutenir et à stabiliser le repliement des protéines.

3. Procédé selon la revendication 1, **caractérisé en ce que** l'étape 4 est réalisée soit manuellement ou automatiquement d'après la conception et le plan de l'invention, miniaturisée et effectuée dans au moins l'une des modes de réalisation suivants,

- la réalisation se basant sur l'électrophorèse de préférence celle sur gel de polyacrylamide orientée sur les protéines globales et hydrophiles et ayant subi au moins l'une des améliorations suivantes,

- la connexion d'un système d'approvisionnement de la solution tampon pour l'ajustage pendant l'électrophorèse de la force ionique du tampon et sa composition ainsi que la valeur du pH et ceci soit en incrustant un récipient perméable qui est rempli avec la solution tampon souhaitée et se trouvant dans le réservoir de la solution tampon de la cathode ou en utilisant un distributeur de la solution tampon, situé dans le réservoir de la solution tampon de la cathode et relié à la cuve extérieure avec un tuyau mince pour alimenter la solution tampon souhaitée selon le rythme voulu et ceci à des dosages continus ou discontinus,
- le contrôle dynamique et l'optimisation de la résolution de séparation par augmentation des différences dynamiques de la répartition de la charge et de la polarité des intermédiaires de la protéine due à l'ajustement ou réglage de la force ionique du tampon et sa composition ainsi que la valeur du pH pendant l'électrophorèse et leurs interactions différenciées avec un courant ionique supplémentaire,
- l'application de l'électrophorèse en mode pulsé par augmentation brève de la tension, un changement court de la polarité des composants du tampon et/ou de leur concentration ainsi qu'une courte inversion de la polarité combinée à des contre-ions hautement hydrophobes pour focalisation de chaque bande et l'agrandissement de l'enlèvement des bandes de gel contenant les intermédiaires de la protéine
- l'utilisation des compositions et des formes de gel variables,
- réduction de la diffusion des espèces intermédiaires causées par des effets thermiques entre les bandes du gel par l'installation d'un dispositif constitué des plaques de refroidissement de Peltier et un liquide de refroidissement circulant pour un transport simultané de la chaleur du gel et de la dissipation de chaleur dans un réservoir de refroidissement contenant de la glace,

- chromatographie en phase liquide, électrochromatographie et fractionnement par flux de champ en couplant spectroscopiquement, de préférence la dispersion dynamique et statique de la lumière pour la séparation des espèces intermédiaires de toutes les protéines et la différenciation de leurs tailles hydrodynamiques, essentiellement pour la séparation et la différenciation de la taille des espèces intermédiaires des protéines qui ne sont pas convenables aux méthodes d'électrophorèse à cause de leur forte acidité, forte basicité et hydrophobicité, des protéines membranaires et des grandes protéines, chacune à micro-échelle ainsi qu'à échelle analytique et semi-préparatif tout en procédant comme suit,

- chromatographie liquide à seule colonne en utilisant de préférence selon les souhaits et les besoins des micro-colonnes individuelles de filtration sur gel chargées avec des agents de séparation différents ou des micro-canaux de fractionnement de flux de champ en couplage avec une vérification spectrométrique pour la détermination directe de l'ordre des tailles hydrodynamiques des intermédiaires séparés respectivement dans chaque éluât,
- chromatographie liquide à multi-colonnes en couplage avec des vérifications spectrométriques et qui s'effectue selon les souhaits ou les besoins par des micro-colonnes connectées en série et/ou en parallèle **caractérisées par** des combinaisons de différentes chromatographies tel que celle de filtration sur gel, hydroxyapatite, hydrophobes, á échange d'ions, à phase inversée et d'affinité y compris aussi celle des micro-canaux de fractionnement de flux de champ pour la séparation spécifiques et la différenciation de la taille des intermédiaires qui sont surtout de tailles hydrodynamiques très similaires mais appartenant respectivement à des chemins de repliement qui sont soit identiques ou différents,
- la différenciation spectrométrique couplée des tailles hydrodynamiques des intermédiaires séparés et récoltés respectivement dans des récipients individuels ou dans des microplaques et leur quantification préférablement par commande dynamique et statique de la lumière en complément par fluorescence, UV, dichroïsme circulaire, RMN, transformation de Fourier et résonance de spin électronique,
- la différenciation spécifique des intermédiaires contenus dans un essai individuel qui sont soit répartis en différentes tailles hydrodynamiques ou présentant des tailles hydrodynamiques très similaires et appartenant si ça se trouve aux différents chemins de repliement au moyen des appareils de commande dynamique et statique de la lumière ajustés de la façon suivante et qui comprennent au moins une des fonctions supplémentaires suivantes,

- augmentation de la résolution de la différentiation en prolongeant le temps de mesure,
- différentiation de la température de fusion Tm par l'augmentation graduelle commandée par programme de la température,
- altération de la concentration des échantillons dans les micro-récipients individuels ou dans des microplaques par dilution ou par concentration, ceci est réalisé par évaporation sous vide ou par aération,
- modification du profil de pH et du système de tampon par micro-autotitration ou par pipetage manuel de la composition de tampon souhaité,
- détermination et évaluation de la viscosité intrinsèque et du potentiel zêta des échantillons pour une différenciation structurelle supplémentaire des intermédiaires,
- changement des propriétés biorhéologiques des échantillons par un apport d'énergie et enlèvement d'énergie en utilisant le rayonnement, le chauffage et le refroidissement, les ultrasons, les micro-ondes, le champ électrique et le champ magnétique,
- évaluation et arrangement des intermédiaires différentiés précédemment par un logiciel spécialement développé pour ce but,

- la réalisation sur laquelle se base l'électrophorèse capillaire en couplage avec la spectrométrie, de préférence avec une commande dynamique et statique de la lumière pour une séparation automatique et miniaturisée des intermédiaires et la différentiation de leurs tailles hydrodynamiques.

**4.** Procédé selon l'une des revendications 1 à 3, **caractérisé en ce que** le processus de repliement est **caractérisé par** la détermination des structures en trois dimensions de la totalité des intermédiaires ou des intermédiaires significatifs ayant une cinquième caractéristique supplémentaire et ensuite représentés dans un système de coordonnées multidimensionnel y compris la cinquième ordonnée intégrée visualisant ainsi la diversité structurelle des intermédiaires.

**5.** Application du procédé selon les revendications 1 à 4, **caractérisé par** l'investigation, la vérification et l'évaluation des changements d'activité et de fonctionnalité d'une protéine repliée dans des milieux moléculaires différents envers son substrat pour l'amélioration ou optimisation de la fabrication biotechnologique d'une protéine thérapeutique.

**6.** Application du procédé selon les revendications 1 à 4, **caractérisé par** l'utilisation du procédé dans l'ingénierie des protéines, dans lequel sont caractérisés les processus de repliement conformément à l'invention tout en vérifiant, examinant et évaluant les changements de fonctionnalité et d'activité de la protéine qui en résultent.

**7.** Application du procédé selon les revendications 1 à 4, **caractérisé par** la caractérisation dynamique et la quantification des processus de biosynthèse simulée *in vitro* et la modification co- et post-traductionnelle éventuelles pour optimiser les conditions de la production biotechnologiques des protéines thérapeutiques subissant une modification post-traductionnelle *in vitro* et scanner les substances chimiques et biologiques permettant de soutenir ou d'inhiber les modifications co et/ou post-traductionnelles , pendant lequel la caractérisation des processus de modifications co- et/ou postraductionnelles simulées dynamiquement *in vitro* est effectuée d'une façon définie et soumise à des comparaisons et évaluations correspondantes.

**8.** Application du procédé selon les revendications 1 à 4, **caractérisé par** la caractérisation dynamique du processus de repliement de protéines pendant leurs modifications post-traductionnelles connues in vivo et simulées *in vitro* pour le développement du procédé de leur production biotechnologique, pendant lequel la caractérisation de ces modifications suit des étapes définies et que la technique de la mobilisation des protéines et la production de la puce protéique sont utilisées.

**9.** Application du procédé selon les revendications 1 à 4, **caractérisé par** la recherche de substances biologiques ou chimiques pour influencer le repliement des protéines, pendant lequel les substances biologiques et/ou chimiques choisies ayant un rôle de soutien et/ou d'inhibition sont inclues dans la caractérisation du repliement de la protéine à étudier.

**10.** Application du procédé selon les revendications 1 à 4, **caractérisé par** l'étude de l'effet des foldases ou chaperons sur le repliement des protéines, pendant lequel cet effet est défini par une comparaison des processus de repliement caractérisés avec ou sans les foldases ou chaperons à étudier.

**11.** Application du procédé selon les revendications 1 à 4, **caractérisé par** la recherche d'inhibiteurs biologiques et chimiques des foldases, chaperons et d'agents de dégradation des protéines, pendant lequel la protéine à étudier est traitée en parallèle respectivement avec les foldases, chaperons et les agents de dégradation des protéines et ceci tout d'abord en absence et ensuite en présence des inhibiteurs correspondants et ensuite soumise à des caractérisations, comparaisons et évaluations des processus de repliement.

**12.** Application du procédé selon les revendications 1 à 4, **caractérisé par** l'examen d'un auto-assemblage contrôlé et la polymérisation des polypeptides ou de protéines dans leur repliement pour le développement et la production des matériaux nano-protéiques, pendant lequel les processus initiaux d'auto-assemblage et de polymérisation sont caractérisés et évalués sous l'effet des facteurs biologiques et/ou chimiques agissant dans des conditions physiologiques et biologiques variables.

**13.** Application du procédé selon les revendications 1 à 4, **caractérisé par** la recherche de chaperons pharmacologiques contre les maladies causées par des protéines (protéopathies), pendant lequel en se basant sur la caractérisation du repliement des protéines protéopathiques on détermine l'effet de certaines substances biologiques et/ou chimiques qui stabilisent le repliement en se liant spécifiquement aux protéines dépliées, améliorent le repliement, stabilisent la structure de la protéine ou masquent les domaines hydrophobes des protéines repliées d'une manière incorrecte et augmentent la solubilité empêchant ainsi l'agrégation des protéines non repliées

**14.** Application du procédé selon les revendications 1 à 4, **caractérisé par** la caractérisation des processus d'une part de déformation de la PrP$^c$ (Protéine prion cellulaire) à la PrP$^{Sc}$ (Protéine prion de scrapie, forme pathogène de la protéine prion), y compris l'achèvement de l'agrégation et d'autre part du renversement de la PrP$^{Sc}$ à la PrP$^{Sc}$ y compris la dégradation de l'agrégation pour clarifier la pathogenèse, rechercher les options de thérapie et les possibilités de prévention dans lequel sont caractérisés les processus de repliement de la protéine prion dans des essais parallèles avec des substances biologiques et/ou chimiques influençant le repliement sous régulation des conditions déstabilisantes et comparé avec processus de repliement caractérisé auparavant en absence des substances influençant le repliement.

15. Application du procédé selon les revendications 1 à 4, **caractérisé par** la caractérisation dynamique du processus de repliement d'une protéine cible pour l'étude du vieillissement de la protéine causé par isomérisation, désamidation et racémisation et de sa détérioration causée par l'action des radicaux libres, du stress oxydatif et des influences de l'environnement, pendant lequel cette caractérisation s'effectue en soumettant la protéine cible en voie de repliement soit par accélération catalytique d'isomérisation, désamidation et racémisation en fournissant de l'énergie photochimique et thermique ou par une modification initiée par l'action des radicaux libres, du stress oxydatif et des influences de l'environnement et le changement de processus de repliement qui en résulte sera ensuite caractérisé et comparé au processus de repliement effectué précédemment en absence de facteurs d'influences.

16. Application du procédé selon les revendications 1 à 4, **caractérisé par** la classification (taxonomie) des protéines, et la recherche de l'évolution des protéines sur un nouveau niveau de repliement pendant lequel le processus de repliement caractérisé comme empreinte individuelle de chaque protéine fournit des relations fonctionnelles et évolutionnaires des protéines et il est intégré en tant que critère supplémentaire dans la classification des protéines qui est basée jusqu'à présent seulement sur la structure, topologie, homologie et parenté évolutionnaire.

17. Application du procédé selon les revendications 1 à 4, **caractérisé par** la caractérisation dynamique des processus de repliement d'une formation du complexe de nucléoprotéines, glycoprotéines et lipoprotéines pour étudier le processus de formation et les changements d'activité et fonctionnalité qui l'accompagnent et aussi la recherche des agents chimiques et biologiques qui agissent sur la formation du complexe, pendant lequel les processus de repliement de la protéine concernée et de son complexe sont d'abord caractérisés séparément et ensuite mis ensemble pendant leur repliement pour former le complexe et ceci dans des essais successifs avec des intervalles de temps donnés et enfin caractérisés, comparés et évalués de façon répétée en absence et en présence de ce complexe protéique sous l'influence des agents chimiques et biologiques variables.

18. Application du procédé selon les revendications 1 à 4, **caractérisé par** le diagnostic et le pronostic des maladies provoquées par le repliement incorrect des protéines pendant lequel les changements du processus de repliement des protéines liées à la maladie sont caractérisés et définis en tant que critères pour le diagnostic et le pronostic de certaines maladies.

19. Application du procédé selon les revendications 1 à 4, **caractérisé par** la caractérisation dynamique du processus initial d'agrégation de la même protéine ou de protéines différentes au cours de leur repliement entre les protéines qui se plient ou entre les protéines qui se plient et les protéines natives et ceci pour l'éclaircissement du mécanisme d'agrégation des protéines et la recherche de leurs agents inhibiteurs d'origine chimiques ou biologiques, pendant lequel les processus de repliement de la protéine à étudier sont caractérisés, comparés et évalués respectivement dans des conditions de réactions moléculaires différentes d'abord sans influence et ensuite sous l'influence d'autres protéines et/ou d'agents inhibiteurs d'origine chimique et biologiques.

20. Application du procédé selon les revendications 1 à 4, **caractérisé par** la caractérisation dynamique du processus de repliement dans l'interaction entre les protéines qui se replient ou bien entre les protéines qui se replient et les protéines natives permettant ainsi d'étudier le comportement conformationnel et les propriétés catalytiques d'une protéine spécifique au niveau de son repliement afin de chercher les protéines thérapeutiques cibles de façon à permettre le design rationnel des substances actives et l'optimisation des procédés biotechnologiques tout en caractérisant, comparant et évaluant respectivement les processus conçus de repliement de la protéine à étudier d'abord sans puis sous l'influence d'autres protéines et ceci dans un environnement moléculaire optimisé.

21. Application du procédé selon les revendications 1 à 4, **caractérisé par** la caractérisation dynamique du processus de la réaction antigène-anticorps et du processus de décomposition du complexe antigène-anticorps pour l'optimisation et la rationalisation de l'ingénierie des anticorps, pendant lequel les anticorps et les antigènes nouvellement construits sont soumis à des caractérisations, comparaisons et évaluations d'abord séparément puis réunis dans des environnements moléculaires physiologiques et biochimiques optimisés sans et/ou avec les facteurs influençant d'origine chimique et biologique pour étudier la sélectivité, la spécificité, l'affinité, l'efficacité de repliement, la stabilité thermodynamique, la cinétique pharmacologique et la productivité biotechnologique de l'anticorps redessiné et de l'antigénicité de l'antigène.

22. Application du procédé selon les revendications 1 à 4, **caractérisé par** la caractérisation in vitro du processus de repliement d'une protéine biosynthétisée et éventuellement subie une modification co-traductionnelle pour l'éclaircissement du repliement initié de la protéine durant sa biosynthèse naissante et la recherche de substances qui affectent le pliage initié de la protéine, dans lequel les fragments polypeptidiques d'une protéine synthétisés en différentes longueurs dans des essais exempts de cellules sont obtenus par une alimentation régulée par des acides aminés nécessaires avec ou sans marquage isotopique et sous l'influence des facteurs biologiques et physicochimiques optimisés, désignés comme mini-intermédiaires, d'abord collectés ensemble, puis séparés sélectivement par chromatographie et enfin soumis aux caractérisations et comparaison de leurs processus de repliement.

23. Application du moyen pour l'accomplissement du procédé selon la revendication 1, **caractérisé par** les kits, appareils et logiciel, ainsi que les automates conçus utilisés respectivement pour la réalisation manuelle ou automatique du procédé.

# Figur-1

## Figur-1-A

## Figur-1-B

## Figur-1-C

15 Aminosäuren

26 Aminosäuren

# Figur-2

**Energiezustand/hydrodynamische Größe**

**Figur-2-A**

entfalteter Parvulustat

welche Intermediate?
an welchem Faltungswegen?

Entfaltung / Rückfaltung

nativer Parvulustat

renaturierter Parvulustat

**Faltungszeit**

**Figur-2-B**

Die 2 nativen Intermediaten

Nativer Parvulustat

Die 4 nichtnativen Intermediaten

Entfaltung
- Denatuierung
- Reduktion

Rückfaltung
- Renatuierung
- Deoxidation
- Intermediaten
  Abfangen

Nativer u. vollmodifizierter

◄ -CH₂CONH₂

— Disulfidbrücke des Parvulustats

**Figur-2-C**

- PL-Konzentration mg/ml
- Zahl der freien Thiolreste / PL
- reduziertes PL in Prozent

| Probe | 1 | 2 | 3 | 4 | 5 |
|---|---|---|---|---|---|
| $A_{412nm}$ | 0,78 | 0,88 | 1,07 | 0,25 | 0,175 |
| $A_{280nm}$ | 0,35 | 0,44 | 0,52 | 0,38 | 0,26 |
| ■ PL-Konzentration | 1,2 mg/ml | 1,5mg/ml | 1,8mg/ml | 1,3mg/ml | 0,9mg/ml |
| ▣ Zahl der freien Thiolreste | 3,97 | 3,55 | 3,66 | 1,17 | 1,19 |
| ■ Reduziertes PL in Prozent | 99% | 89% | 92% | 29% | 30% |

# Figur-3

Figur-3-A

### Modifikation und Abfangen der Intermediate des Parvulustats ( kinetisch in Minutenskala)

#### Ansatz der Modifikation und Abfanglösung (frische Herstellung vor Gebrauch)

| 0,6M Jodacetmid (µl) | 110,5mg Jodacetamid im 1,5ml-Röhrchen mit 0,25M Tris/HCl pH7,5 auf 1ml | | | | | | | | | | | | | |
|---|---|---|---|---|---|---|---|---|---|---|---|---|---|---|
| Probennummer | 1 | 2 | 3 | 4 | 5 | 6 | 7 | 8 | 9 | 10 | 11 | 12 | 13 | 14 |
| Abfangstart (min) | 1 | 3 | 6 | 9 | 15 | 30 | 45 | 60 | 90 | 120 | 150 | 180 | 210 | 240 |
| 0,6M Jodacetmid (µl) | 20 | 20 | 20 | 20 | 20 | 20 | 20 | 20 | 20 | 20 | 20 | 20 | 20 | 20 |
| Abfangvolumen (µl) | 100 | 100 | 100 | 100 | 100 | 100 | 100 | 100 | 100 | 100 | 100 | 100 | 100 | 100 |
| Abfangzeit (min) | 5 | 5 | 5 | 5 | 5 | 5 | 5 | 5 | 5 | 5 | 5 | 5 | 5 | 5 |
| Abfangende (min) | 6 | 8 | 11 | 14 | 20 | 35 | 50 | 65 | 95 | 125 | 155 | 185 | 215 | 245 |

Gelbande/Energiezustand
Hydrodynamische Größe

Figur-3-B

Faltungsablauf in Minute 0    1    3    6    9    15    30    45    60    120    180    240    Zeit

Faltungsablauf in 4 Phasen  |* |————— Phase-2 ————►|————— Phase-3 ————►|— Phase-4 —►|
Aufbau der 4 Faltungswege    Faltung entlang 4 Wegen  Struktur-Recovery

Faltungsablauf in Typen    |————Rückfaltungsfingerabdruckprofil ————►|

*: sympolisierte Phase-1        N: nativer Parvulustat; R: reduzierter Parvulustat

# Figur-4

## Figur-4-A

**2 native Intermediate**

Trypsin     Trypsin

4967,2     6507,1

1929,9   3037,3   3469,8

C9-C27   ATGSPVAEC VEYFQSWB WTDVHNGCADAVSVTVEYTHGQWAPC VIEPGGWATFAGYGTDGNYVTGLHTCDPATPSGV

5025,5    6447,9

1988,2   3037,3   3410,6

C43-C70   ATGSPVAEC VEYFQSWB WTDVHNGCADAVSVTVEYTHGQWAPC VIEPGGWATFAGYGTDGNYVTGLHTCDPATPSGV

**4 nicht-native Intermediate**

4967,2    6507,1

1929,9   3037,3   3469,8

C9-C43   ATGSPVAEC VEYFQSWB WTDVHNGCADAVSVTVEYTHGQWAPC VIEPGGWATFAGYGTDGNYVTGLHTCDPATPSGV

5025,5    6447,9

1988,2   3037,3   3410,6

C25-C70   ATGSPVAEC VEYFQSWB WTDVHNGCADAVSVTVEYTHGQWAPC VIEPGGWATFAGYGTDGNYVTGLHTCDPATPSGV

4967,5    6449,1

1988   2979,3   3469,8

C25-C43   ATGSPVAEC VEYFQSWB WTDVHNGCADAVSVTVEYTHGQWAPC VIEPGGWATFAGYGTDGNYVTGLHTCDPATPSGV

5025,2    6505,9

1929,9   3095,3   3410,6

C9-C70   ATGSPVAEC VEYFQSWB WTDVHNGCADAVSVTVEYTHGQWAPC VIEPGGWATFAGYGTDGNYVTGLHTCDPATPSGV

**der durch Carboxamidomethylierung vollmodifizierte und der renaturierte Parvulustat**

5083,5    6565,1

1988,2   3095,3   3469,8

C. C.C.C   ATGSPVAEC VEYFQSWB WTDVHNGCADAVSVTVEYTHGQWAPC VIEPGGWATFAGYGTDGNYVTGLHTCDPATPSGV

4909,2    6389,9

1929,9   2979,3   3410,6

C-C_C-C   ATGSPVAEC VEYFQSWB WTDVHNGCADAVSVTVEYTHGQWAPC VIEPGGWATFAGYGTDGNYVTGLHTCDPATPSGV

## Figur-4-B

### Fragmente-Massenerkennungsmuster in *Da*

**Trypsin-Spaltung der Intermediate und Einordnung der Fragmentsmassen mit -CH$_2$CONH$_2$**

| Bezeichnung | Fragment-1 | Fragment-2 | Fragment-3 | Fragmente-4 | Fragmente-5 |
|---|---|---|---|---|---|
| **2 native Intermediate** | | | | | |
| C9-C25 | 1929,9 | 3037,3 | 3469,8 | 4967,2 | 6507,1 |
| C43-C70 | 1988,2 | 3037,3 | 3410,6 | 5025,5 | 6447,9 |
| **4 nicht-native Intermediate** | | | | | |
| C9-C43 | 1929,9 | 3037,3 | 3469,8 | 4967,5 | 6507,1 |
| C25-C70 | 1988,2 | 3037,3 | 3410,6 | 5025,5 | 6447,9 |
| C25-C43 | 1988,2 | 2979,3 | 3469,8 | 4967,5 | 6449,1 |
| C9-C70 | 1929,9 | 3095,3 | 3410,6 | 5025,2 | 6505,9 |
| **vollmodifizierter PL** | | | | | |
| C.C.C.C. | 1988,2 | 3095,3 | 3469,8 | 5083,5 | 6565,1 |
| renaturierter Parvulustat C-C_C-C (8282,03 *Da*) | 1929,9 | 2979,3 | 3410,6 | 4909,2 | 6389,9 |

**Figur-5**

## Figur-6

**Tabelle für Differenzierung, Bezeichnung, Gruppen- u. Faltungswegezuordnung der Intermediate**

Intermediate-Identifizierung nach Trypsin-Verdau und MALDI-MS der 10 Gelbanden

| Nr.* | Trypsin-Verdau-Fragmente Toleranz bis zu 36 (Da) | | | Differenzierung | Intermediate-Bezeichnung | Nr. | Gruppe | Wege |
|---|---|---|---|---|---|---|---|---|
| 1 | 1986,2 | 3097,4 | 3467,9, | vollmodifizierter Parvulustat | C.C.C.C | - | - | |
| 2 | 1986,6 | 3091,8 | 3465,2 | vollmodifizierter Parvulustat | C.C.C.C -1 | 1 | 1 | 2 |
| 3 | 1986,5 | 2997,9 | 3488,5 | nicht-natives Intermediat | C25-C43 | 2 | 2 | 3 |
| 4 | 2000,6 | 3118,8 | 3485,7 | vollmodifizierter Parvulustat | C.C.C.C -2 | 3 | 1 | 2 |
| 5 | 1995,6 | 2998,8 | 3478,3 | nicht natives Intermediat | C25-C43 | 4 | 2 | 3 |
| 6 | 1999,1 | 3115,4 | 3482,8 | vollmodifizierter Parvulustat | C.C.C.C -3 | 5 | 1 | 2 |
| 7 | 1993,3 | 3097,4 | 3476,5 | vollmodifizierter Parvulustat | C.C.C.C -4 | 6 | 1 | 2 |
| 8 | 1991,6 | | 3474,8 4968,2 | Nicht natives Intermediat | C25-C43 | 7 | 2 | 3 |
| | 1991,6 | 3035,5 | 5030,0 6445,6 | Nicht natives Intermediat | C25-C70 | 8 | 2 | 3 |
| | | | 5030,0 6445,6 | natives Intermediat | C43-C70 (C9-CH$_2$CONH$_2$) | 9 | 4,1 | 1,2 |
| 9 | 1996,0 | 2988,7 | 3478,3 | Nicht nativer Parvulustat | C25-C43 | 10 | 3 | 4 |
| | | | 8300,2 | renaturierter Parvulustat | PL | | | |
| | | | 8345,2 | renat. PL mit C9-Amidierung | PL(C9-CH$_2$CONH$_2$) | | | |
| 10 | 1996,0 | | 3476,1 4982,4 | Nicht natives Intermediat | C25-C43 (C9-CH$_2$CONH$_2$ ) | 11 | 3 | 4 |
| | | | 3476,1 4982,4 | native Intermediate | C9-C25 | 12 | 3 | 4 |

Nr.*: Nummer der Gelbande; PL: Parvulustat

## Figur-7

Gelbande/Energiezustand/Hydrodynamische Größe

2D-Veranschaulichung des Faltungsvorgangs von Parvulustat im Gel-Bild von Figur3-B

Figur-8

Visuelle Darstellung des Faltungsvorgangs des Parvulustats in einem 2D-Koordinatensystem

**Figur-9**

## Faltungswege des Parvulustats in 3D-Darstellung
## (bis zu 60 Minuten)

**Konzeption und Entwurf für die automatisierte Ausführung des erfindungsgemäßen Verfahrens**

Figur-10

## Figur-11

**Multifaltungswege-Modell**

hohe     niedrig     hohe

Konformationsgrad

groß

Phase-I

Phase-II

Phase-III

Phase-IV

klein

Energiezustand
hydrodynamische Größe
heterogenität

Tempolimitzone
Intermediatezone

A
B
C
D
E

Funktionszone: A, B, C, D und E

0     Ende     0

Rückfaltungszeit

3 von Primärstruktur definiert dominante strukturelle Seedpopulationen mit ihrer potenziellen Subpopulationen

Primärfaltungsweg     Sekundärfaltungsweg     Missfaltungsweg

3 primäre Zugange jeweils zu ihren Haupt- und 2 nachrangigen Faltungswegen inklusive ihrer potenziellen Intermediaten

3 sekundäre Zugange jeweils zu ihren Haupt- und 2 nachrangigen Faltungswegen inklusive ihrer potenziellen Intermediaten

3 zu Missfaltung gehörte Zugange zu ihren Haupt- und 2 nachrangigen Faltungswegen inklusive ihrer potenziellen Intermediaten

Abzweigunge zwischen den 3 dominanten Faltungswegen

primäre und umgelagerte native Intermediate

sekundäre native Intermediate     missfaltete Intermediate

renatuiertes Protein     vervollständigtes natives Protein

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **BILSEL et al.** *Current Opinion in Structural Biology,* vol. 16 (1), 86-93
- **CHEN Y et al.** *Archives of Biochemistry and Biophysics,* vol. 469 (1), 4-19
- **ALEXEI V. BUEVICH et al.** *Journal of the American Chemical Society,* vol. 124 (24), 7156-7162
- **BHARATH S. MAMAHTAMBIKA et al.** *Annual Review of Cell and Developmental Biology,* vol. 24 (1), 211-235
- **PAMELA K. JENSEN et al.** *Analytical Chemistry,* vol. 70 (10), 2044-2049
- **JIANG WU et al.** *Protein Science,* vol. 7 (4), 1017-1028
- **J THROCK WATSON.** *Journal of Molecular Graphics and Modelling,* vol. 19 (1), 119-128
- **STEPHAN REHM et al.** *Chembiochem,* vol. 10 (1), 119-127
- Disulfide Folding Pathway of BPTI. *Science,* 03. April 1992, vol. 256